# EUROPEAN PATENT APPLICATION

(11) **EP 1 586 581 A2**
(43) Date of publication of application: **19.10.2005**
(21) Application number: 05007788.2
(22) Date of filing: 08.04.2005
(51) Int. Cl.: C07K 7/06, C07K 7/23, A61K 38/09, C07K 16/26, C12N 15/00

(54) **Non-Mammalian GnRH analogs and uses thereof in regulation of fertility and pregnancy**

(30) Priority: 08.04.2004 US 820477
(71) Applicant: Siler-Khodr, Theresa M., San Antonio, TX 78257 (US)
(72) Inventor: Siler-Khodr, Theresa M., San Antonio, TX 78257 (US)
(74) Representative: Walker, Ross Thomson

(57) **Abstract**

Chicken II and salmon GnRH or its analog decapeptides resistant to degradation by peptidase incorporating D-arginine, D-leucine, D-tBu-Serine, D-Trp or other active D amino acids at position 6 and ethylamide, aza-Gly-amide or other Gly amide at position 10. The non-mammalian GnRH or its analogs demonstrate preferential binding to male and female reproductive system GnRH receptors as well as tumor cell GnRH receptors in these systems. Biopotency is greater within the reproductive system and at tumor cells than at the pituitary. These non-mammalian GnRH or its analogs may be used in pharmaceutical preparations, and specifically in various treatment methods as a contraceptive or post-coital contraceptive agent. The non-mammalian GnRH or its analogs are also provided in pharmaceutical preparations that may be used clinically for maintaining pregnancy when used in very low doses and administered in pulsatile fashion, as well as in preparations for the treatment of male and female reproductive system disorders including cancers of these systems or other system with GnRH II receptors. The aza-Gly (10) amide non-mammalian analogs are yet other embodiments of the non-mammalian GnRH analogs provided as a part of the invention.

Sequence Listing

<110>Siler-Khodr, Theresa M.

<120>Non-Mammalian GnRH Analogs and Uses Thereof in Regulation of
Fertility and Pregnancy

<130>P7345.2(CIP)6

<140>US 10/820,477

<141>2004-04-08

<150>US 10/639,405

<151> 2003-08-12

<160>16

<210>1
<211>30
<212>DNA
<213>Gallus gallus (Chicken II GnRH)

<400>1
cagcactggt cccatggctg gtaccctgga     30

<210>2
<211>10
<212>PRT
<213>Unknown

<220>
<221>mat_peptide
<222>6
<223>Chicken II GnRH Analog. MOD_RES substitution of Gly residue
at 10 by aza-Gly-NH2, ethylamide or other Gly amide. Xaa represents
D-Arg, D-Leu, D-tBu-Ser, or D-Trp. MOD_RES Glu at position 1 is
pyroglutamic acid.

<400>2
Glu His Trp Ser His Xaa Trp Tyr Pro Gly
            5                    10

<210>3
<211>30
<212>DNA
<213>Salmo salar (Salmon GnRH)

<400>3

cagcactggt cttatggctg gctgcctgga     30

<210>4
<211>10
<212>PRT
<213>Unknown

<220>
<221>mat_peptide
<222>6
<223>Salmon GnRH Analog. MOD_RES substitution of Gly residue
at 10 with aza-Gly-NH2, ethylamide or other Gly amide. Xaa represents
D-Arg. MOD_RES Glu at position 1 is pyroglutamic acid.

<400>4
Glu His Trp Ser Tyr Xaa Trp Leu Pro Gly
            5                   10

<210>5
<211>10
<212>PRT
<213>Homo sapiens

<220>
<221>mat_peptide
<222>unknown
<223>Mammalian GnRH. MOD_RES Glu at position 1 is pyroglutamic acid.

<400>5
Glu His Trp Ser Tyr Gly Leu Arg Pro Gly
            5                   10

<210>6
<211>10
<212>PRT
<213>Gallus gallus

<220>
<221>mat_peptide
<222>Within brain MRNA 121-150, within brain gene 2174-2203
<223>Chicken II GnRH. MOD_RES Glu at position 1 is pyroglutamic acid.

<400>6
Glu His Trp Ser His Gly Trp Tyr Pro Gly
            5                   10

<210>7
<211>10
<212>PRT
<213>Salmo salar

<220>
<221>mat_peptide
<222>unknown
<223>Salmon GnRH. MOD_RES Glu at position 1 is pyroglutamic acid

<400>7
Glu His Trp Ser Tyr Gly Trp Leu Pro Gly
                5                   10
<210>8
<211>30
<212>RNA
<213>Gallus gallus (Chicken II GnRH)

<400>8
gucgugacca ggguaccgac caugggaccu 30

<210>9
<211>30
<212>RNA
<213>Salmo salar (Salmon GnRH)

<400>9
gucgugacca gaauaccgac cgacggaccu 30

<210>10
<211>9
<212>PRT
<213>Unknown

<220>
<221>mat_peptide
<222>6
<223>Buserelin. MOD_RES Glu at position 1 is pyroglutamic acid.
Xaa represents D-Ser (t-Bu). MOD_RES Pro residue at 9 bound to
ethylamide.

<400>10
Glu His Trp Ser Tyr Xaa Leu Arg Pro
                5

<210>11
<211>9
<212>PRT
<213>Unknown

<220>

<221>mat_peptide
<222>6
<223>Leuprolide. MOD_RES Glu at position 1 is pyroglutamic acid.
Xaa represents D-Leu. MOD_RES Pro residue at 9 bound to ethylamide.

<400>11
Glu His Trp Ser Tyr Xaa Leu Arg Pro
                5

<210>12
<211>10
<212>PRT
<213>Unknown

<220>
<221>mat_peptide
<222>1,2,3,5,6,8,10
<223>Antide. Xaa 1 is Ac-D-Nal, Xaa2 is D-Cpa, Xaa3 is D-Pal, Xaa5 is NicLys,
Xaa6 is D-NicLys, Xaa8 is ILys, Xaa10 is D-Ala.

<400>12
Xaa Xaa Xaa Ser Xaa Xaa Leu Xaa Pro Xaa
                5                   10

<210>13
<211>10
<212>PRT
<213>Gallus gallus

<220>
<221>mat_peptide
<222>unknown
<223>Chicken I GnRH. MOD_RES Glu at position 1 is pyroglutamic acid.

<400>13
Glu His Trp Ser Tyr Gly Leu Gln Pro Gly
                5                   10

<210>14
<211>10
<212>PRT
<213>Lampetra genus

<220>
<221>mat_peptide
<222>unknown
<223>Lamprey GnRH. MOD_RES Glu at position 1 is pyroglutamic acid.

<400>14
Glu His Tyr Ser Leu Glu Trp Lys Pro Gly
                5                   10

<210>15
<211>30
<212>DNA
<213>Clupea harengus (Herring GnRH)

<400>15
cagcactggt cttatggctg gctgcctgga 30

<210>16
<211>10
<212>PRT
<213>Unknown

<220>
<221>mat_peptide
<222>6
<223>Herring GnRH Analog. MOD_RES substitution of Gly
residue at 10 with aza-Gly-NH2, ethylamide or other Gly
amide. Xaa represents D-Arg. MOD_RES Glu at position 1 is
pyroglutamic acid.

<400>16
Glu His Trp Ser Tyr Xaa Leu Ser Pro Gly
                5                   10

## Description

### FIELD OF THE INVENTION

The present invention relates generally to the field of regulating reproductive hormone functions, fertility and pregnancy. More particularly, it concerns the use of unique non-mammalian peptide hormone analogs of GnRH designed to be useful in male and female fertility regulation, post-coital contraception and as a menses-inducing agent, in the management of ovarian cyst, polycystic ovarian disease, in vitro fertilization protocols, endometriosis, abnormal uterine bleeding, leiomyomas, abnormal pregnancies, ectopic pregnancies, molar pregnancies, and trophoblastic disease in the management of disorders of the male reproductive system and regulation of cell growth, particularly cancer cell growth of these systems.

### BACKGROUND OF THE INVENTION

Before the chemical characterization of the mammalian hypothalamic GnRH, it was realized that hypothalamic substances regulated production of pituitary LH and FSH. Current steroid contraceptive methods are centered on the existing knowledge of GnRH-gonadotropin-ovarian physiology. The delineation of mammalian GnRH (also known as GnRH I) made possible the ability to create methods to detect and quantify this molecule. The human placenta and the chorionic membranes have also been observed to contain a GnRH-like substance. The present investigator has localized, quantified and demonstrated the synthesis of a GnRH-like substance by the human placenta. *Burgus R., Guillemim R 1970 Hypothalamic releasing factors. Ann Rev Biochem 39: 499-526; Gibbons JM, Mitnick M, Chieffo V 1975 In vitro biosynthesis of TSH- and LH-releasing factors by the human* *placenta. Am J Obstet Gynecol 121:127-131; Siler-Khodr TM, Khodr GS 1978 Luteinizing hormone releasing factor content of the human placenta. Am J Obstet Gynecol 130: 216-219; Khodr GS, Siler-Khodr TM1978 Localization of luteinizing hormone releasing factor (LRF) in the human placenta. Fert Steril 29: 523-526; Siler-Khodr TM, Khodr GS 1979 Extrahypothalamic luteinizing hormone releasing factor (LRF): Release of immunoreactive LRF by the human placenta in vitro. Fert Steril 22: 294-296; Khodr GS, Siler-Khodr TM 1980 Placental LRF and its synthesis. Science 207:315-317.*

The concentration of immunoreactive GnRH-like material in the placenta and maternal blood has been found to vary with gestational age, following a pattern similar to that of hCG. It was also demonstrated that exogenous synthetic mammalian GnRH can stimulate hCG production from human placental explants in vitro, and that the GnRH stimulation of hCG release was a receptor mediated event, since it was specific and could be inhibited by a GnRH antagonist, [N-Ac-Pro,D-p-Cl-Phe,D-Nal(2)]-GnRH. In addition to the inhibition of hCG, progesterone production was dramatically suppressed. A gestational age-related action of the GnRH antagonist on the release of hCG and steroids was observed. The present investigator also observed that hCG response was related to the gestational age of the placenta. Further studies demonstrated a potent action of mammalian GnRH on placental prostanoids, again resulting in their inhibition when endogenous chorionic GnRH was the highest. The GnRH antagonist also inhibited basal prostaglandin production with greater potency than equimolar concentrations of GnRH, and this action was partially reversed by mammalian GnRH. Thus, a chorionic GnRH was identified by the present investigator to regulate hCG in a paracrine fashion within the human placenta. These data demonstrated that this paracrine axis is of physiologic significance in cell to cell communication, and not of inconsequential, ectopic, tumor production. *Siler-Khodr TM, Khodr GS, Valenzuela G 1984* *Immunoreactive gonadotropin-releasing hormone level in maternal circulation throughout pregnancy. Am JObstet Gynecol 150:376-379; Sorem KA, Smirkel CB, Spencer DK, Yoder BA, Grayson MA, Siler-Khodr TM 1996 Circulating maternal CRH and GnRH in normal and abnormal pregnancies. Am J Obstet Gynecol 175:912-916; Khodr GS, Siler-Khodr TM 1979 The effect of luteinizing hormone releasing factor (LRF) on hCG secretion. Fert Steril 30:301-304; Siler-Khodr TM, Khodr GS 1981 Dose response analysis of GnRH stimulation of hCG releases from human term placenta. Biol Reprod* 25: 353-358; *Siler-Khodr TM, Khodr GS 1979 Extrahypothalamic luteinizing hormone releasing factor (LRF): Release of immunoreactive LRF by the human placenta in vitro. Fert Steril 22:294-296; Siler-Khodr TM, Khodr GS, Vickery BH, Nestor JJ, Jr. 1983 Inhibition of hCG, alpha hCG and progesterone release from human placental tissue in vitro by a GnRHantagonist. Life Sci 32:2741-2745; Siler-Khodr TM, Khodr GS, Valenzuela G, Rhode J 1986 Gonadotropin-releasing hormone effects on placental hormones during gestation: 1 Alpha-human chorionic gonadotropin, human chorionic gonadotropin and human chorionic somatomammotropin. Biol Reprod 34:245-254; Siler-Khodr TM, Khodr GS, Rhode J, Vickery BH, Nestor JJ, Jr. 1987 Gestational age related inhibition of placental hCG, hCG and steroid hormone release in vitro by a GnRH antagonist. Placenta 8:1-14; Siler-Khodr TM, Khodr GS, Valenzuela G, Harper J, Rhode J 1986 GnRH effects on placental hormones during gestation. 111 Prostaglandin E, prostaglandin F, and 13, 14-dihydro-15-keto-prostaglandin F. Biol Reprod 35:312-319; Kang IS, Koong MY, Forman JS, Siler-Khodr TM 1991 Dose-related action of GnRH on basal prostanoid production from the human term placenta. Am J Obstet Gynecol 165:1771-1777; Siler-Khodr TM, Khodr GS, Harper MJ, Rhode J, Vickery BH, Nestor JJ, Jr. 1986 Differential inhibition of human placental prostaglandin release in vitro by a GnRH antagonist. Prostaglandins 31:1003-1010; Siler-Khodr T.M. and G.S. Khodr. 1981. The* *production and activity of placental releasing hormones. In Fetal Endocrinology. J Resko and W. Montagna, editors. Academic Press Inc. New York. 183-210; Siler-Khodr, T.M and G.S. Khodr. 1982 GnRH in the placenta. In role of Peptides and Proteins in Control of Reproduction; D.S. Khindsa and S.M. McCann, editors. Elsevier North Holland, New York 347-363; Siler-Khodr TM 1983 Hypothalamic-like releasing hormones of the placenta. Clin Perinatol 10: 533-566; Siler-Khodr TM 1983 Hypothalamic-like peptides of the placenta. Semin Reprod Endocrinol 1:321-333.*

Studies of other investigators have reported on the actions of mammalian GnRH on placental function. The chorionic GnRH axis has also been identified as having a feedback interaction for activin, inhibin, follistatin, neurotransmitter, prostaglandin and steroids. These and other studies established the presence of this paracrine axis, including a negative feedback loop for progesterone and estrogen, similar to that of the hypothalamic-pituitary-gonadal axis. This placental axis, multiple paracrine axes for GnRH and other hypothalamic-like releasing and inhibiting activities have now been defined in the placenta, eye, pancreas, ovary, brain, bone, etc., and are now recognized as essential to normal physiologic functions. *Shi LY*, *Zhang ZW*, *Li WX 1994 Regulation of human chorionic gonadotropin secretion and messenger ribonucleic acid levels by follistatin in the NUCC-3 choriocarcinoma cell line. Endocrinology 134:2431-2437; Steele GL, Currie WD, Yuen BH, Jia XC*, *Perlas E, Luang PC* 1993 *Acute stimulation of human chorionic gonadotropin secretion by recombinant human activin-A in first trimester human trophoblast. Endocrinology 133: 297-303; Li W*, *Olofsson JI*, *Jeung EB, Krisinger J*, *Yuen BH*, *Leung PC 1994 Gonadotropin-releasing hormone (GnRH) and cyclic AMP positively regulate inhibit subunit messenger RNA levels in human placental cells. Life Sci 55:1717-1724; Petraglia F, Vaughan J*, *Vale W 1991 Inhibin and activin modulate the release of gonadotropin-releasing hormone, human chorionic* *gonadotropin, and progesterone from cultured human placental cells. Proc Natl Acad Sci U S A 86:5114-5117; Petraglia F, Sawchenko P, Lim ATW, Rivier J, Vale W 1987 Localization, secretion, and action of inhibin in human placenta. Science 237:187-189; Shi CZ, Zhuang LZ 1993 Norepinephrine regulates human chorionic gonadotropin production by first trimester trophoblast tissue in vitro. Placenta 14:683-693; Cemetikic B, Maulik D, Ahmed MS 1992 Opioids regulation of hCG release from trophoblast tissue is mediated by LHRH. Placenta Ahstract: 9(Abstr.); Petraglia F, Vaughan J, Vale W 1990 Steroid hormones modulate the release of immunoreactive gonadotropin-releasing hormone from cultured human placental cells. J Chn Endocrinol Metab 70:1173-1178; Haning RV, Jr., Choi L, Kiggens AJ, Kuzma DL, Summerville JW 1982 Effects of dibutyryl adenosine 3'*, *5'-monophosphate, luteinizing hormone-releasing hormone, and aromatase inhibitor on simultaneous outputs of progesterone 17b-estradiol, and human chorionic gonadotropin by term placental explants. J Clin Endocrinol Metab 55:213-218; Petraglia F, Lim AT, Vale W* 1987 *Adenosine 3', 5-monophosphate, prostaglandin, and epinephrine stimulate the secretion of immunoreactive gonadotropin-releasing hormone from cultured human placental cells. J Clin Endocrinol Metab 65:1020-1025; Harting R V, Jr. Choi L, Kiggens AJ, Kuzma DL 1982 Effects of prostaglandin, dibutyryl camp LHRH, estrogen, progesterone, and potassium on output of prostaglandin F2a, 13, 14-dihydro-15-keto-prostaglandin F2a, hCG, estradiol, and progesterone by placental minces. Prostaglandins 24: 495-506; Barnea EP, Feldman D, Kaplan M 1991 The effect of progesterone upon first trimester trophoblastic cell differentiation and human chorionic gonadotropin secretion. Hum Reprod 6:905-909; Barnea ER, Kaplan M 1989 Spontaneous, gonadotropin-releasing hormone-induced, and progesterone-inhibited pulsatile secretion of human chorionic gonadotropin in the first trimester placenta in vitro. J Clin Endocrinol Metab 69:215-217; Branchaud C, Goodyear C,* *Lipowski L 1983 Progesterone and estrogen production by placental monolayer cultures: Effect of dehydroepiandrosterone and luteinizing hormone-releasing hormone. J Chn Endocrinol Metab 56:761-766; Ahmed NA, Murphy BE 1988 The effects of various hormones on human chorionic gonadotropin production in early and late placental explant cultures. Am J Obstet Gynecol 159:1220-1227; Iwashita M, Watanabe M, Adachi T, Ohira A, Shinozaki Y, Takeda Y, Sakamoto S 1989 Effect of gonadal steroids on gonadotropin-releasing hormones stimulated human chorionic gonadotropin release by trophoblast cells. Placenta 10:103-112; Haning RV, Jr., Choi L, Kiggnes AJ, Kuzma DL, Summerville JW 1982 Effects of dibutyryl cAMP, LHRH, and aromatase inhibitor on simultaneous outputs of prostaglandin F2a, and 13, 14-dihydro-15-keto-prostaglandin F2a by term placental explants. Prostaglandins 23:29-40; Wilson E, Jawad M 1980 Luteinizing hormone-releasing hormone suppression of human placental progesterone production. Fert Steril 33: 91-93; Siler-Khodr, T.M. 1992. The Placenta: Part IV-Function of the Human Placenta. In Neonatal and Fetal Medicine. R.A. Polin and W.W. Fox, editors. W.B. Saunders Co. Philadelphia, PA. 74-86; Youngblood WW, Humni J, Kizer JS 1979 TRH-like immunoreactivity in rat pancreas and eye, bovine and sheep ideals, and human placenta: Non-identity with synthetic Pyroglu-His-Pro-NH2 (TRH). Brain Res 163: 101-110; Dubois MP 1975 Immunoreactive somatostatin is present in discrete cells of the endocrine pancreas. Proc Natl Acad Sci U S A 72:1340-1343; Adashi. E.Y. 1996. The Ovarian Follicular Apparatus. In Lippincott-Raven Publishers. E. Y. Adashi. J.A. Rock, and Z. Rosenwaks, editors. Lippincott-Raven Publishers, Philadelphia. 17-40.*

The isolation and characterization of a mammalian GnRH gene in the placenta, which is transcribed to a mRNA identical to that in the hypothalamus with the exception of the inclusion of the first intron and a very long first exon. The message has been localized to the syncytio- and cytotrophoblast, as well as the stroma of the placenta, and is present in higher concentrations during the first half of pregnancy. Multiple transcription sites have been identified for the mammalian GnRH gene in reproductive tissues, including the placenta. Steroid regulatory sites on the promoter have also been identified. The functionality of this promoter is supported by showing that GnRH mRNA can be regulated by steroids. *Radovick S, Wondisford FE, Nakayama Y, Yamada M, Cutler GB, Jr., Weintraub BD 1990 Isolation and characterization of the human gonadotropin-releasing hormone gene in the hypothalamus and placenta. Mol Endocrinol 4:476-480; Adelman JP, Mason AJ, Hayflick JS, Seeburg PH 1986 Isolation of the gene and hypothalamic cDNA for the common precursor of gonadotropin-releasing hormone and prolactin release-inhibiting factor in human and rat. Proc Natl Acad Sci USA 83:179-183; Seeberg PH, Adelman JP 1984 Characterization of cDNA for precursor of human luteinizing hormone releasing hormone. Nature 311:666-668; Duello TM, Tsai SJ, Van Ess PJ 1993 In situ demonstration and characterization of pro gonadotropin-releasing hormone messenger ribonucleic acid in first trimester human placentas. Endocrinology 133: 2617-2623; Kelly AC, Rodgers A, Dong KW, Barrezueta NX, Blum M, Roberts JL 1991 Gonadotropin-releasing hormone and chorionic gonadotropin gene expression in human placental development DNA Cell Biol 10:411-421; Dong KW, Yu KL, Roberts JL 1993 Identification of a major up-stream transcription start site for the human pro gonadotropin-releasing hormone gene used in reproductive tissues and cell lines. Mol Endocrinol 7:1654-166; Dong KW, Duval P, Zeng Z, Gordon K, Williams RF, Hodgen GD, Jones G, Kerdelhue B, Roberts JL 1996 Multiple transcription start sites for the GnRH gene in rhesus and cynomolgus monkeys: a non-human primate model for studying GnRH gene regulation. Mol Cell Endocrinol 117:121-130; Dong KW, Yu KL, Chen ZG, Chen YD, Roberts JL 1997 Characterization of multiple promoters directing tissue-specific expression* *of the human gonadotropin-releasing hormone gene. Endocrinology 138:2754-2762; Chandran UR*, *Attardi B, Friedman R*, *Dong KW*, *Roberts JL, DeFranco DB 1994 Glucocorticoid receptor-mediated repression ofgonadotropin-releasing hormone promoter activity in GTI hypothalamic cell lines. Endocrinology 134:1467-1474; Dong KW, Chen ZG*, *Cheng KW*, *Yu KL 1996 Evidence for estrogen receptor-mediated regulation of human gonadotropin-releasing hormone promoter activity in human placental cells. Mol Cell Endocrinol 117:241-246; Joss JM*, *King JA, Millar RP 1994 Identification of the molecular forms of and steroid hormone response to gonadotropin-releasing hormone in the Australian lungfish Neoceratodus forsteri. Gen Comp Endocrinol 96:392-400*; *Montero M*, *Le Belle N, King JA, Millar RP, Dufour S 1995 Differential regulation of the two forms of gonadotropin-releasing hormone (mGnRH and chicken GnRH-II) by sex steroids in the European female silver eel (Anguilla anguilla)*. *Neuroendocrinology 61:525-535; Ikeda M*, *Taga M*, *Sakakibara H*, *Minaguchi H*, *Ginsburg E, Vonderhaar BK 1996 Gene expression of gonadotropin-releasing hormone in early pregnant rat and steroid hormone exposed mouse uteri. JEndocrinol Invest 19:708-713*; *Gothilf Y*, *Meiri II*, *Elizur A, Zohar Y 1997 Preovulatory changes in the levels of three gonadotropin-releasing hormone-encoding messenger ribonucleic acids (mRNAs), gonadotropin. B-subunit mRNAs plasma gonadotropin, and steroids in the female gilthead seabream, Sparus aurata*, *Biol Reprod 57:1145-1154.*

The GnRH receptor in the placenta has not been characterized as fully as the GnRH receptor in the pituitary. It is known that a placental mammalian GnRH receptor exists, having a Ka of only 10⁻⁶M. In addition, superagonist or antagonist for the pituitary mammalian GnRH receptor shows very different affinity for these analogs than does the placental receptor in primates. GnRH receptor activity, as well as the mRNA for the mammalian GnRH receptor, varies throughout gestation in the human placenta.- The receptor is greatest in early gestation and appears to be down regulated by 12-20 weeks. While the receptor is again detectable in term placentas, the mRNA for mammalian GnRH (using a mammalian GnRH decapeptide probe and in situ hybridization methodology) was undetectable at this state of gestation. This pattern of receptor activity is consistent with the concentration of GnRH-like material in placental tissue and maternal blood throughout gestation, and supports the hypothesis that certain concentrations of chorionic GnRH may down-regulate its chorionic receptors, as can mammalian GnRH, and its analogs at the pituitary level. Studies by the present investigator and those of Barnea et al, have demonstrated competitive inhibition by certain mammalian GnRH antagonist in placental tissue. Other studies of Szilagyi et al. and Currie et al. indicate that mammalian GnRH agonist can down-regulate the placental GnRH receptor. In addition, the demonstration that the placental GnRH receptor can be up regulated in cell cultures by estradiol supports the hypothesis that this receptor is functional in the regulation of placental hormonogenesis. *Sealfon SC, Weinstein H*, *Millar RP 1997 Molecular mechanism of ligand interaction with the gonadotropin-releasing hormone receptor. Endocr Rev 18:180-205; Karten MJ*, *Rivier JE 1986 Gonadotropin-releasing hormone analog design. Structure-function studies toward the development of agonists and antagonists: Rationale and perspective*. *Endocr Rev 7:44-66*. *Escher E, Mackiewicz Z*, *Lagace G, Lehoux J*, *Gallo-Payet N, Bellabarba D, Belisle S 1988 Human placental LHRH receptor: Agonist and antagonist labeling produces differences in the size of the non-denatured, solubilize receptor. JRecept Res 8:391-405; Bramley TA, McPhie CA, Menzies GS 1992 Human placental gonadotropin-releasing hormone (GnRH) binding sites: Characterization, properties and ligand specificity. Placenta 12:555-581*; *Bramley TA*, *McPhie CA*, *Menzies GS 1994 Human placental gonadotropin-releasing* *hormone (GnRH) binding sites: 111. Changes in GnRH binding levels with stages of gestation. Placenta 15:733-745; Lin LS, Roberts VJ, Yen SS 1997 Expression of human gonadotropin-releasing hormone receptor gene in the placenta and its functional relationship to human chorionic gonadotropin secretion. J Clin Endocrinol Metab 80:580-585; Siler-Khodr TM, Khodr GS, Valenzuela G 1984 Immunoreactive gonadotropin-releasing hormone level in maternal circulation throughout pregnancy. Am J Obstet Gynecol 150: 376-379; Siler-Khodr TM, Khodr GS 1978 Luteinizing hormone releasing factor content of the human placenta. Am J Obstet Gynecol 130: 216-219; Siler-Khodr TM, Khodr GS, Vickery BH, Nestor JJ, Jr. 1983 Inhibition of hCG, alpha hCG and progesterone release from human placental tissue in vitro by a GnRH antagonist. Life Sci 32:2741-2745; Siler-Khodr TM, Khodr GS, Harper MJ, Rhode J, Vickery BH, Nestor JJ, Jr. 1986 Differential inhibition of human placental prostaglandin release in vitro by a GnRH antagonist. Prostaglandins 31:1003-1010; Barnea ER, Kaplan M, Naor Z 1991 Comparative stimulatory effect of gonadotropin releasing hormone (GnRH) and GnRH agonist upon pulsatile human chorionic gonadotropin secretion in superfused placental explants: reversible inhibition by a GnRH antagonist. Hum Reprod 6:1063-1069; Szilagyi A, Benz R, Rossmanith WG 1992 The human first-term placenta in vitro: regulation of hCG secretion by GnRH and its antagonist. Gynecol Endocrinol 6:293-300; Currie WD, Setoyarna T, Lee PS, Bairnbridge KG, Church J, Yuen BH, Leung PC 1993 Cytosolic free Ca2+ in human syncytiotrophoblast cells increased by gonadotropin-releasing hormone. Endocrinology 133:2220-2226; Bliatacharya S, Chaudhary J, Das C 1992 Responsiveness to gonadotropin releasing hormone of human term trophoblast cells in vitro: induction by estradiol. Biochem Int 28: 363-371.*

Another factor that regulates a hormone=s activity is its metabolism. The dominant enzyme that degrades GnRH in chorionic tissues and blood during pregnancy differs during pregnancy from the enzyme that degrades GnRH in the pituitary or the blood of non-pregnant individuals. In placental tissue, the primary enzymatic activity for the degradation of GnRH is chorionic peptidase-1 (C-ase-1), a post-proline peptidase. C-ase-1 is a glycoprotein with a molecular weight of 60,000. It acts as a post-proline peptidase, and is inhibited by bacitracin, para-amino-benzamidine, acetopyruvate and certain cations. GnRH is actively degraded by C-ase-1 at neutral pH, having a Km of 10⁻⁸M. Using immunofluorescent methodology, C-ase-1 has been localized by the present inventor in the cytoplasm of the syncytiotrophoblast and syncytial buds. It is secreted into maternal blood, where GnRH is not stable without specific inhibitors of this post-proline peptidase C-ase-1 is present in very high concentrations, and accounts for virtually all GnRH degrading activity in the placenta under physiological conditions. *Siler-Khodr TM, Kang IS, Jones MA, Harper MJK, Khodr GS, Rhode J 1989 Characterization and purification of a placental protein that inactivates GnRH, TRH and Angiotensin 11. Placenta 10: 283-296; Kang IS, Siler-Khodr TM 1992 Chorionic peptidase inactivates GnRH as a post-proline peptidase. Placenta 13:81-87; Kang IS, Gallwitz J, Guzman V, Siler-Khodr TM 1990 Definition of the enzyme kinetics and optimal activity of chorionic peptidase-1. The 23*^{*rd*} *Annual Meeting of the Society for the Study of Reproduction (Vancouver) (Abstract #311):144(Abstr.); Benuck M, Marka N 1976 Differences in the degradation of hypothalamic releasing factors by rat and human serum. Life Sci 19:1271-1276.*

These *in vitro* studies support the hypothesis of the specific, receptor-mediated and enzyme-regulated action of mammalian GnRH on placental hormonogenesis, and demonstrate the paracrine effects and feedback interactions for numerous intrauterine hormones interacting with chorionic GnRH.

Petraglia et al have described the pulsatile release of a GnRH-like substance, which has a specific pulse frequency, amplitude and duration, with increased amplitude during early gestation. Further studies on the action of mammalian GnRH and its analogs *in vivo* have also demonstrated these paracrine interactions for chorionic GnRH-like activity and numerous other chorionic hormones, and have established the physiologic role of GnRH in the maintenance of normal pregnancy. The secretion of a GnRH-like substance by the peri-implantation rhesus monkey embryo, which precedes the secretion of chorionic gonadotropin has been demonstrated. *Duello TM*, *Tsai SJ*, *Van Ess PJ 1993 In situ demonstration and characterization ofpro gonadotropin-releasing hormone messenger ribonucleic acid in first trimester human placentas. Endocrinology 133:2617-262-3; Lin LS, Roberts VJ*, *Yen SS 1997 Expression of human gonadotropin-releasing hormone receptor gene in the placenta and its functional relationship to human chorionic gonadotropin secretion. J Clin Endocrinol Metab 80:580-585; Dong KW*, *Chen ZG, Cheng KW*, *Yu KL 1996 Evidence for estrogen receptor-mediated regulation of human gonadotropin-releasing hormone promoter activity in human placental cells. Mol Cell Endocrinol 117:241-246; Petraglia F, Genazzani AD, Aguzzoli L, Gallinelli A*, *de Vita D, Caruso A, Genazzani AR 1994 Pulsatile fluctuations of plasma-gonadotropin-releasing hormone and corticotropin-releasing factor levels in healthy pregnant women. Acta Obstet Gynecol Scand 73:284-289; Siler-Khodr, T.M. 1993. Luteinizing Hormone Releasing Hormone (LHRH) and the Placenta and Fetal Membranes. In Molecular Aspects of Placental and Fetal Membrane Autocoids. G.E. Rice and S.P. Brennecke*, *editors. CRC Press, Inc. Ann Arbor. 339-350; Petraglia F, Calza L, Garuti GC, Giardino L, De Ramundo BM*, *Angioni S 1990 New aspects of placental endocrinology. J Endocrinol Invest 65:262-267; Seshagiri PB, Terasawa E, Hearn JP 1994 The secretion of gonadotropin-releasing hormone by peri-implantation embryos of the rhesus monkey: comparison with the secretion of chorionic gonadotropin. Hum Reprod 9:1300-1307.*

Other investigators have shown that administration of high doses of mammalian GnRH, its agonistic analogs or antibodies, to pregnant baboons and monkeys effects a sharp decrease of CG and progesterone production, which in most cases leads to termination of pregnancy. We have administered mammalian GnRH antagonists to pregnant baboons of more than 35 days post implantation and in some animals we observed pregnancy loss or a negative outcome of the pregnancies. In a saline controlled study we administered mammalian GnRH agonist or an antagonist to pregnant baboons just following implantation and have found pregnancy loss using high doses in many of these animals. Interruption of pregnancy was most consistently observed when these mammalian GnRH analogs were administered around the time of or shortly following implantation. *Gupta SK, Singh M 1985 Characteristics and bioefficacy of monoclonal antigonadotropin releasing hormone antibody. Am J. Repro Immunol Microbiol 7:104-108; Das C, Gupta SK, Talwar GP 1985 Pregnancy interfering action of LHRH and anti-LHRH. J. Steroid Biochem 23:803-806; Hodges JK, Hearn JP 1977 Effects of immunization against luteinizing hormone releasing hormone on reproduction of the marmoset monkey Callithrix jacchus. Nature 265: 746-748; Vickery BH, McRae GI Stevens VC 1981 Suppression of luteal and placental function in pregnant baboons with agonist analogs of luteinizing hormone-releasing hormones. Fert Steril 36:664-668; Das C, Talwar GP 1983 Pregnancy-terminating action of a luteinizing hormone-releasing hormone agonist D-Ser(But)6desGlyl0ProEA in baboons. Fert Steril 39:218-223; Rao A, Moudgal N 1984 Effect of LHRH injection on serum chorionic: gonadotropin levels in the pregnant bonnet monkey (Macaca radiata). Obstet Gynecol 12:1105-1106; Rao AJ, Chakraborti R, Kotagi SG, Ravindranath N, Moudgal NR 1985 Effect of LHRH agonists and antagonists in male and female bonnet monkeys (Macaca Radiata). J. Steroid Biochem 23:807-809; T. M Siler-Khodr, T. J. Kuehl, and B. H. Vickery. Effects of a gonadotropin* *releasing hormone antagonist on hormonal levels in the pregnant baboon and on fetal outcome. Fertil.Steril. 41:448-454, 1984; T. M Siler-Khodr, T. Kuehl, and B. Vickery. Action of a GnRH antagonist on the pregnant baboon. The 32nd Annual Meeting of The Society for Gynecologic Investigation (Phoenix) (Abstract #249):142, 1985; I. S. Kang, T. J. Kuehl, and T. M Siler-Khodr. Effect of treatment with gonadotropin-releasing hormone analogues on pregnancy outcome in the baboon. Fertil.Steril. 52:846-853, 1989.*

In pregnant women, administration of low doses of mammalian GnRH does not significantly change circulating hCG. However, this finding was dose and gestational age related. A study of Devreker et al reports that the use of long-acting mammalian GnRH analogs in IVF impaired the implantation rate. While these analogs have proven to be generally nontoxic, long-term chronic use has been associated with a hypo-estrogenic state. Accidental administration of mammalian GnRH analogs during early pregnancy has been reported, with varied outcomes. Generally, pregnancy outcomes appeared unaffected, but increased cases of spontaneous abortion and pre-term labor have also been observed. The varied outcomes may reflect the different doses and protocols of administration of these mammalian GnRH analogs, as well as the different analogs employed. For analogs that can be rapidly metabolized by the chorionic tissues, little effect, if any, would be anticipated. In addition, the affinity for the placental receptor for many of these mammalian GnRH analogs is greatly reduced as compared to the pituitary receptor=s affinity and they are degraded by the placental enzymes. In those cases, little chorionic effect would be observed. *Tamada T, Akabori A, Konuma S, Araki S 1976 Lack of release of human chorionic gonadotropin by gonadotropin-releasing hormone. Endocrinol Jap 23:531-533; Perez-Lopez FR, Robert J, Teyeiro J 1984 Prl, TSH, FSH, B-hCG and oestriol responses to repetitive (triple) LRH*/*TRH administration in the third trimester of human pregnancy. Acta Endocrinol 106:400-404;* *Egyed J, Gati I 1985 Elevated serum hCG level after intravenous LH-RH administration in human pregnancies. Endocrinol Exp 19:11-15; Iwashita M, Kudo Y, Shinozaki Y, Takeda Y 1993 Gonadotropin-releasing hormone increases serum human chorionic gonadotropin in pregnant women. Endocrine Journal 40:539-544; Devreker F, Govaerts I, Bertrand E, Van den Bergh M, Gervy C, Englert Y 1996 The long-acting gonadotropin-releasing hormone analogues impaired the implantation rate. Fert Steril 65:122-126, Siler-Khodr, T.M. 1994 Potentials for embryo damage ofGnRH analogs. In Ovulation Induction: Basic Science and Clinical Advances. M Filicor and C. Flamigni, editors Elsevier Science B. V. Amsterdam. 279-306.*

The ovary is also known to produce a GnRH-like peptide. The presence of a GnRH receptor was first described in rat luteal cells in 1979. A GnRH receptor in human corpus luteum was later described by Bramley et al and the expression of an mRNA for mammalian GnRH in human ovarian tisues was later described by Dong et al. However, the affinity of the ovarian and placental receptor for mammalian GnRH or its analogs is greatly reduced as compared to the pituitary's mammalian GnRH receptor. Other investigators have described mammalian GnRH mRNA expression in the fallopian tube and the early embryo. In addition, the expression of mRNA for mammalian GnRH in the endometrium has been reported. *Aten RF, Williams AT, Behrman HR Ovarian gonadotropin-releasing hormone-like protein(s): demonstration and characterization. Endocrinology 1986; 118: 961-967*; *Aten RF, Polan ML*, *Bayless R, Behrman HR. A gonadotropin-releasing hormone (GnRH)-like protein in human ovaries: similarity to the GnRH-like ovarian protein of the rat. J. Clin. Endocrinol. Metab. 1987; 64: 1288-1293; Peng C, Fan NC, Ligier M*, *Vaananen J*, *Leung PCK*. *Expression and regulation of gonadotropin-releasing hormone (GnRH) and GnRH receptor messenger ribonucleic acids in human granulosa-luteal cells. Endocrinology 1994; 135:* *1740-1746; Clayton RN, Harwood JP, Catt KJ. Gonadotropin-releasing hormone analogue binds to luteal cellsand inhibits progesterone production. Nature 1979; 90: 282; Bramley TA, Menzies GS, Baird DT. Specific binding of gonadotropin-releasing hormoneand an agonist to human corpus luteum homogenates: Characterization, properties, and luteal phase levels. J. Clin. Endocrinol. Metab. 1985; 61: 834-841; Dong KW, Yu KL, Roberts JL. Identification of a major up-stream transcription start site for the human progonadotropin-releasing hormone gene used in reproductive tissues and cell lines. Mol. Endocrinol. 1993; 7: 1654-1666; Casan EM, Raga F, Polan ML. GnRH mRNA and protein expression in human pre implantation embryos. Mol. Hum. Reprod. 1999; 5: 234-239;*
*Casan EM, Raga F, Bonilla-Musoles F, Polan ML. Human oviductal gonadotropin-releasing hormone: possible implications in fertilization, early embryonic development, and implantation. J. Clin. Endocrinol. Metab. 2000; 85: 1377-1381; Raga F, Casan EM, Kruessel JS, Wen Y, Huang H- Y, Nezhat C, Polan ML. Quantitative gonadotropin-releasing hormone (GnRH) gene expression and immunohistochemical localization in human endometrium throughout the menstrual cycle. Biol. Reprod. 1998; 59: 661-669; Casan EM, Raga, Kruessel JS, et.al. Immunoreactive gonadotropin-releasing hormone expression in the cycling human endometrium of fertile patients. Fertil. Steril. 1998; 70: 102-106.*

Most studies describing the activity of mammalian GnRH and its analogs in extra-pituitary tissues have been plagued with the finding of low affinity binding sites and the high concentration of mammalian GnRH required to affect tissue function. Similar observations have been made for the placental GnRH receptor. Although there is substantial data to support the hypothesis that there are active GnRH axes in the ovary, endometrium and the placenta and other extrapituitary sites, the physiologic relevance of mammalian GnRH in these extra-pituitary tissues seemed questionable. *Bramley TA, McPhie CA, Menzies GS.* *Human placental gonadotrophin-releasing hormone (GnRH) binding sites: I. Characterization, properties and ligand specificity. Placenta 1992; 13: 555-581.*

It has previously been accepted that only non-mammalian vertebrates have multiple forms of GnRH in the same species. Therefore, the hypothesis of more than one form of GnRH in the human placenta was considered dubious. However, Dellovad, et al and King et al have described chicken II GnRH in shrew, mole and bat brain, thus demonstrating that two different isomers of GnRH existed in the mammal. Chicken II GnRH has now been characterized in the guinea pig and in the primate brain. Separate genes for chicken II GnRH and mammalian GnRH have also been described. Other isomers of GnRH, such as salmon GnRH and chicken II GnRH, have a much greater affinity for the placental receptor, yet bind with a lesser affinity to the human pituitary receptor. These data demonstrate the existence of a specific placental receptor for GnRH-like molecules, yet the true ligand for this receptor is not known. In amphibians, a chicken II GnRH receptor as well as a mammalian GnRH receptor have been shown. The specificity and evolutionary aspects of the GnRH receptor have been studied in many species. Mammalian GnRH has been reported to be active in many vertebrate classes. Other GnRHs, such as chicken II GnRH and salmon GnRH, have reduced affinity for the mammalian pituitary receptor. *Bramley TA, McPhie CA, Menzies GS 1992 Human placental gonadotropin-releasing hormone (GnRH) binding sites: Characterization, properties and ligand specificity. Placenta 12:555-581; Dellovade TL, King JA, Millar RP, Rissman EF 1993 Presence and differential distribution of distinct forms of immunoreactive gonadotropin-releasing hormone in the musk shrew brain. Neuroendocrinology 58:166-177; King JA, Steneveld A A, Curlewis JD, Rissman EF, Millar RP 1994 Identification of chicken GnRHII in brains of inetatherian and early-evolved eutherian species of mammals. Regul Pept 54:467-477; Jimenez-Linan M, Rubin BS, King JC* *1997 Examination of guinea pig luteinizing hormone-releasing hormone gene reveals a unique decapeptide and existence of two transcripts in the brain. Endocrinology 13 8:4123-4130; Lescheid D, Terasawa E, Abler LA, Urbanski HF, Warby CM, Millar RP, Sherwood NM 1997 A second form of gonadotropin-releasing hormone (GnRH) with characteristics of chicken GnRH-II is present in the primate brain. Endocrinology 138:1997; White SA, Bond CT, Francis RC, Kasten TL, Fernald RD, Adelman JP 1994 A second gene for gonadotropin-releasing hormone: cDNA and expression pattern in the brain. Proc Natl Acad Sci USA 91:1423-1427; Lin XW, Peter RE 1997 Cloning and expression pattern of a second [His5Trp7Tyr8] gonadotropin-releasing hormone (chicken GnRH-H-11) mRNA in goldfish; evidence for two distinct genes. Gen Comp Endocrinol 107: 262-272.*

There are also implications for regulation of cell growth and function, particularly cancer cell growth. Before the chemical characterization of the mammalian GnRH it was realized that a hypothalamic substance regulated the production of pituitary LH and FSH and that these gonadotropins regulated gonadal steroidogenesis. The delineation of mammalian GnRH enabled Applicants to synthesize this decapeptide and administer it systemically to human. It was then recognized that a long acting superagonist of this mammalian GnRH effected a flare release of pituitary gonadotropins followed by their inhibition. The inhibition was effected by a down-regulation of the pituitary GnRH receptor which corresponded downstream to an inhibition of the gonadal steroids, estrogen, progesterone and/or testosterone. This is a form of chemical castration. *Burgus R, Guillemin R 1970 Hypothalamic releasing factors. Ann Rev Biochem 39:499-52; Baba Y, Matsuo H, Schally AV 1971 Structure of the porcine LH- and FSH-releasing hormone. II. Confirmation of the proposed structure by conventional sequential analyses. Biochem Biophys Res Commun 44:459-463; Corbin A 1982 From contraception to cancer: A review of the therapeutic* *applications ofLHRH analogues as antitumor agents. Yale J Biol Med 55:27-47.*

Since certain types of tumors, such as certain breast and prostate cancers, are now known to be dependent on gonadal steroids, mammalian GnRH analogs have been used to suppress gonadal steroids via their chemical castration activity. Thus, we know that the use of mammalian GnRH analogs is feasible as a treatment of certain cancers. *Buzdar AU, Hortobagyi G 1998 Update on endocrine therapy for breast cancer. Clin Cancer Res 4:527-534; Bare, RL. and F.M. Torti. 1998. Endocrine therapy of prostate cancer. In Biological and hormonal therapies of cancer. K.A. Foon and H.B. Muss, editors. Kluwer Academic Publishers, Boston. 69-86; Corbin A 1982 From contraception to cancer: A review of the therapeutic applications of LHRH analogues as antitumor agents. Yale JBiol Med 55:27-47.*

It was only with the development of sensitive and specific radioimmunoassays for GnRH and GnRH-like molecules that a very surprising finding was reported. That finding was initially described by Applicants. Applicants reported that GnRH-like molecules exist and function not only in the hypothalamic-pituitary axis, which functions as an endocrine system to distribute hormone systemically, but GnRH-like molecules also exist in extra-hypothalamic tissues to provide a paracrine action i.e. localized signal secretion. It is now realized that paracrine action of GnRH-like substances have functions in the placenta, chorionic tissues, gonad, fallopian tube, uterus, breast, prostate, testis, sperm, etc., as well as in many other non-pituitay tissues and cancerous tumors. *Siler-Khodr TM, Khodr GS 1978 Luteinizing hormone releasing factor content of the human placenta. Am J Obstet Gynecol 130: 216-219; Khodr GS, Siler-Khodr TM 1978 Localization of luteinizing hormone releasing factor (LRF) in the human placenta. Fert Steril 29:523-526; Siler-Khodr TM, Khodr GS 1979 Extrahypothalamic luteinizing hormone releasing factor (LRF): Release of immunoreactive LRF by the human placenta in vitro. Fert Steril 22:294-29; Khodr GS, Siler-Khodr TM 1980 Placental LRF and its synthesis. Science* *207:315-317; Siler-Khodr, T.M. 1992. The Placenta: Part IV-Function of the Human Placenta. In Neonatal and Fetal Medicine. R.A. Polin and W.W. Fox, editors. W.B. Saunders Co. Philadelphia, PA. 74-86; Youngblood WW, Humm J*, *Kizer JS 1979 TRH-like immunoreactivity in rat pancreas and eye, bovine and sheep pineals, and human placenta: Non-identity with synthetic Pyroglu-His-Pro-NH2 (TRH)*. *Brain Res 163:101-110; Dubois MP 1975 Immunoreactive somatostatin is present in discrete cells of the endocrine pancreas. Proc Natl Acad Sci USA 72:1340-1343; Adashi, E.Y. 1996. The Ovarian Follicular Apparatus. In Lippincott-Raven Publishers. E.Y. Adashi*, *J.A. Rock*, *and Z. Rosenwaks*, *editors. Lippincott-Raven Publishers, Philadelphia. 17-40; Szende B, Srkalovic G, Groot K, Lapis K*, *Schally AV 1990 Growth inhibition of mouse MXT mammary tumor by the luteinizing hormone-releasing hormone antagonist SB-75. J Natl Cancer Inst 82:513-517; Srkalovic G, Wittliff JL, Schally AV 1990 Detection and partial characterization of receptors of [D-Trp(6)]-luteinizing hormone-releasing hormone and epidermal growth factor in human endometrial carcinoma. Cancer Res 50:1841-1846; Szende B, Srkalovic G, Groot K*, *Lapis K*, *Schally AV 1991 Regression of nitrosamine-induced pancreatic cancers in hamsters treated with luteinizing hormone-releasing hormone antagonists or agonists. Cancer Res 50:3716-372; Ohno T, Atsushi I*, *Furui T, Takahashi K*, *Tamaya T 1993 Presence ofgonadotropin-releasing hormone and its messenger ribonucleic acid in human ovarian epithelial carcinoma. Am JObstet Gynecol 169:605-610; Palyi I*, *Vincze B, Kalnay A, Turi G, Mezo I*, *Teplan I*, *Seprodi J*, *Pato J*, *Mora M 1996 Effect of gonadotropin-releasing hormone analogs and their conjugates on gonadotropin-releasing hormone receptor-positive human cancer cell lines. Cancer Detect Prev 20:146-152; Teissmann T*, *Klenner T*, *Deger W, Hilgard P, McGregor GP, Voigt K*, *Engel J 1996 Pharmacological studies with cetrorelix (SB-75), a potent antagonist of luteinising hormone-releasing hormone. Eur J Cancer 32A:1574-1579; Chatzaki E, Bax MR, Eidne KA,* *Anderson L, Grudzinskas JG, Gallagher CJ 1996 The expression of gonadodtropin-releasing hormone and its receptor in endometrial cancer, and its relevance as an autocrine growth factor. Cancer Res 56: 2059-2065; Jungwirht A, Galvan G, Pinski J, Halmos G, Szepeshazi K, Cai RZ, Groot K, Schally AV 1997 Luteinizing hormone-releasing hormone antagonist cetrorelix (SB-75) and bombesin antagonist RC-3940-II inhibit the growth of androgen-independent PC-3 prostate cancer in nude mice. Prostate 32:164-172; Jungwirth A, Pinski J, Galvan G, Halmos G, Szepeshazi K, Cai RZ, Groot K, Vadillo-Buenfil M, Schally A V 1997 Inhibition of growth of androgen-independent DU-145 prostate cancer in vivo by luteinising hormone-releasing hormone antagonist cetrorelix and bombesin antagonists RC-3940-II and RC-3950-II. Eur J Cancer 33:1141-1148; Bahk JY, Hyun JS, Lee H, Kim MO, Cho GJ, Lee BH, Choi WS 1998 Expression ofgonadotropin-releasing hormone (GnRH) and GnRHreceptor mRNA in prostate cancer cells and effect of GnRH on the proliferation of prostate cancer cells. Urol Res 26:259-264; Van Groeninghen JC, Kiesel L, Winkler D, Zwirner M 1998 Effect of luteinising-hormone-releasing hormone on nervous-system tumors. Lancet 352:372-373; Yin H, Cheng KW, Hwa H, Peng C, Auersperg N, Leung PCK 1998 Expression of the messenger RNA for gonadotropin-releasing hormone and its receptor in human cancer cell lines. Life Sci 62:2015-2023; Lamharzi N, Schally AV, Koppan M* 1998 *Luteinizing hormone-releasing hormone (LH-RH) antagonist Cetrorelix inhibits growth of DU-145 human androgen-independent prostate carcinoma in nude mice and suppresses the levels and mRNA expression of IGF-II in tumors. Regul Pept 77:185-192; Brewer CA, Shevlin D 1998 Encouraging response of an advanced steroid-cell tumor to GnRH agonist therapy. Obstet Gynecol 92: 661-663; Mesia AF, Williams FS, Yan Z, Mittal K 1998 Aborted leiomyosarcoma after treatment with leuprolide acetate. Obstet Gynecol 92:664-666; Motomura S 1998 Inductions of apoptosis in ovarian carcinoma cell line by gonadotropin-releasing hormone* *agonist. Kurume Med J 45:27-32; Imai A, Takagi A, Horibe S, Takagi H, Tamaya T 1998 Fas and Fas ligand system may mediate antiproliferative activity ofgonadotropin-releasing hormone receptor in endometrial cancer cells. Int J Oncol 13:97-100; Lamharzi N, Halmos G, Jungwirth A, Schally A V 1998 Decrease in the level and mRNA expression of LH-RH and EGF receptors after treatment with LH-RH anatagonist Cetrorelix in DU- 145 prostate tumor xenografts in nude mice. Int J Oncol 13:429-435.*

Even with this general knowledge, the effective use of mammalian GnRH analogs to act directly on particularly tumor tissue has not resulted. One of the goals of the present invention was to utilize novel forms of GnRH not previously envisioned for regulation of cell function and cancer therapy that bind to the tumor GnRH-like receptor with 50 to 1000 fold the activity of mammalian GnRH or its superagonist and have potent bioactivity in inhibiting tumor cell growth.

The initial studies on GnRH activity in tumor tissues and the human placenta utilized mammalian GnRH and its analogs, in accordance with the teaching that the human encodes for only one isoform of GnRH *Sherwood NM*, *Lovejoy DA, Coe IR 1993 Origin of mammalian gonadotropin-releasing hormones. Endocr Rev 14:241-254; King JA, Millar RP 1995 Evolutionary aspects ofgonadotropin-releasing hormone and its receptor. Cell Mol Neurobiol 15:5-23.*

Of particular interest to this invention are previous reports of the presence of GnRH-like substances and receptors in numerous cancer tissues and their cell lines. GnRH-like activity and its receptors have been identified in the breast, bronchial, ovarian, endometrial, prostate, gastrointestinal tumors. The function of a GnRH-like substance and its receptors in tumor tissues is supported by the demonstration that mammalian GnRH can stimulate hCG from human and animal tumors and can inhibit cell growth in vitro. These findings have led to numerous studies of the effects of mammalian GnRH analogs on the expression of GnRH receptors, cell signal transduction, apoptosis, and overall growth of tumor cell lines. The growth of tumors in vivo has also been studied with individual case reports of patients responsive to mammalian GnRH analogs. *Srkalovic G, Wittliff JL, Schally A V 1990 Detection and partial characterization of receptors of [D- Trp(6)]-luteinizing hormone-releasing hormone and epidermal growth factor in human endometrial carcinoma. Cancer Res 50:1841-1846; Ohno T, Atsushi I Furui T, Takahashi K, Tamaya T 1993 Presence of gonadotropin-releasing hormone and its messenger ribonucleic acid in human ovarian epithelial carcinoma. Am J Obstet Gynecol 169: 605-610; Chatzaki E, Bax MR, Eidne KA, Anderson L, Grudzinskas JG, Gallagher CJ 1996 The expression of gonadodtropin-releasing hormone and its receptor in endometrial cancer, and its relevance as an autocrine growth factor. Cancer Res 56: 2059-2065; Bahk JY, Hyun JS, Lee H, Kim MO, Cho GJ, Lee BH, Choi WS 1998 Expression ofgonadotropin-releasing hormone (GnRH) and GnRH receptor mRNA in prostate cancer cells and effect of GnRH on the proliferation of prostate cancer cells. Urol Res 26:259-264; Yin H, Cheng KW, Hwa H, Peng C, Auersperg N, Leung PCK 1998 Expression of the messenger RNA for gonadotropin-releasing hormone and its receptor in human cancer cell lines. Life Sci 62:2015-2023; Lamharzi N, SchallyAV, Koppan M 1998 Luteinizing hormone-releasing hormone (LH-RH) antagonist Cetrorelix inhibits growth of DU-145 human androgen-independent prostate carcinoma in nude mice and suppresses the levels and mRNA expression of IGF-II in tumors. Regul Pept 77:185-192; Imai A, Takagi A, Horibe S, Takagi H, Tamaya T 1998 Fas and Fas ligand system may mediate antiproliferative activity of gonadotropin-releasing hormone receptor in endometrial cancer cells. Int J Oncol 13: 97-100; Emons G, Muller V, Ortmann O, Schulz KD 1998 Effects of LHRH-analogues on mitogenic signal transduction in cancer cells. JSteriod Biochem Mol Biol 65:1-6; Pahwa GS, Kullander S, Vollmer G, Oberheuser F, Knuppen R, Emons G 1991 Specific low affinity binding sites for goandotropin releasing hormone in human endometrial carcinoma. Eur J Obstet* *Gynecol Reprod Biol 41:135-142Lamharzi N, Halmos G, Jungwirth A, Schally AV 1998 Decrease in the level and mRNA expression of LH-RH and EGF receptors after treatment with LH-RH anatagonist Cetrorelix in DU-145 prostate tumor xenografts in nude mice. Int J Oncol 13:429-435; Szende B, Srkalovic G, Groot K, Lapis K, Schally AV 1990 Growth inhibition of mouse MXT mammary tumor by the luteinizing hormone-releasing hormone antagonist SB-75. J Natl Cancer Inst 82:513-517; Szende B, Srkalovic G, Groot K, Lapis K, Schally AV 1991 Regression of nitrosamine-induced pancreatic cancers in hamsters treated with luteinizing hormone-releasing hormone antagonists or agonists. Cancer Res 50:3716-3721; Palyi I*, *Vincze B, Kalnay A*, *Turi G, Mezo I*, *Teplan I*, *Seprodi J, Pato J, Mora M 1996 Effect of gonadotropin-releasing hormone analogs and their conjugates on gonadotropin-releasing hormone receptor-positive human cancer cell lines. Cancer Detect Prev 20:146-152; Teissmann T, Klenner T, Deger W, Hilgard P, McGregor GP, Voigt K, Engel J 1996 Pharmacological studies with cetrorelix (SB-75), a potent antagonist of luteinising hormone-releasing hormone. Eur J Cancer 32A:1574-1579; Jungwirht A, Galvan G, Pinski J*, *Halmos G, Szepeshazi K*, *Cai RZ*, *Groot K, Schally AV 1997 Luteinizing hormone-releasing hormone antagonist cetrorelix (SB-75) and bombesin antagonist RC-3940-II inhibit the growth of androgen-independent PC-3 prostate cancer in nude mice. Prostate 32:164-172; JungwirthA, Pinski J*, *Galvan G, Halmos G, Szepeshazi K*, *Cai RZ*, *Groot K*, *Vadillo-Buenfil M*, *Schally AV 1997 Inhibition of growth of androgen-independent DU-145 prostate cancer in vivo by luteinising hormone-releasing hormone antagonist cetrorelix and bombesin antagonists RC-3940-II and RC-3950-II. Eur J Cancer 33:1141-1148; Motomura S 1998 Inductions of apoptosis in ovarian carcinoma cell line by gonadotropin-releasing hormone agonist. Kurume Med J 45:27-32; Nagy A, Schally AV, Armatis P, Szepeshazi K, Halmos G, Kovacs M*, *Zarandi M*, *Groot K, Miyazaki M*, *Jungwirth A, Horvath JE 1996 Cytotoxic analogs of luteinizing hormone-releasing hormone containing* *doxorubicin or 2-pyrrolinodoxorubicin, α derivative 500-1000 times more potent. Proc Natl Acad Sci USA 93:7269- 7273; Vincze B, Palyi I*, *Gaal D, Pato J, Mora M, Mezo I Teplan I Seprodi J 1996 In vivo studies of the new gonadotropin-releasing hormone antagonist-copolymer conjugates having antitumor activity. Cancer Detect Prev 20:153-159; Neri C, Berthois Y, Schatz B, Drieu K, Martin PM 1990 Compared effects of GnRHanalogs and 4-hydroxytamoxifen on growth and steroid receptors in antiestrogen sensitive and resistant MCF- 7 breast cancer cell sublines. Breast Cancer Res Treat 15:85-93; Crighton IL, Dowsett M, Lal A, Man A, Smith IE 1989 Use of luteinising hormone-releasing hormone agonist (Leuprorelin) in advanced post-menopausal breast cancer. Br J Cancer 60: 644-648; Teodorczyk-Injeyan J, Jewett MAS, Kellen JA, Malkin A 1981 Gonadoliberin (LHRH) mediated release of choriogonadotropin in experimental human and animal tumors in vitro. Endocr Res Commun 8:19-24; Van Groeninghen JC, Kiesel L, Winkler D, Zwirner M 1998 Effect of luteinising-hormone-releasing hormone on nervous-system tumors. Lancet 352:372-373; Brewer CA, Shevlin D 1998 Encouraging response of an advanced steroid-cell tumor to GnRH agonist therapy. Obstet Gynecol 92: 661-663; Mesia AF, Williams FS, Yan Z, Mittal K 1998 Aborted leiomyosarcoma after treatment with leuprolide acetate. Obstet GynecoI92:664-666; Bruckner HW, Motwani BT 1989 Treatment of advanced refractory ovarian carcinoma with a gonadotropin-releasing hormone analogue. Clin Med 161:1216-1218; Cassano A, Astone A, Garufi C, Noviello MR, Pietrantonio F, Barone C 1987 A response in advanced post-menopausal breast cancer during treatment with the luteinising hormone releasing hormone agonist-Zoladex. Exp Biol Med 48:123-124; Klijn JGM, DeJong FH 1982 Treatment with a luteinising-hormone releasing-hormone analogue (Busereline) in premenopausal patients with metastatic breast cancer. Lancet 1982:1213-1216.*

However, some very problematic findings from previous studies in the tumor tissue has led to skepticism about the true role of mammalian GnRH analogs in the tissue. The GnRH receptor affinity for GnRH in the tumors is on the order of 10⁻⁵ to 10⁻⁶ M. The biological significance of such a weak affinity in light of much lower levels of endogenous GnRH-like activity must be questioned. In addition, Applicants have observed in human pregnancy studies, both in vitro and in vivo, that mammalian GnRH appears to act as a partial agonist not a true agonist of tumor GnRH. When receptors are available, mammalian GnRH acts as an agonist of tumor GnRH, but when tumor receptors are low or occupied, mammalian GnRH competes with the more potent tumor GnRH resulting in a partial agonist action. Furthermore, Applicants and others have observed that certain antibodies for mammalian GnRH reacted with chorionic GnRH with a different affinity. These findings led Applicants to propose that neither the extra-hypothalamic GnRH nor its receptor are identical to mammalian GnRH and its mammalian GnRH pituitary receptor *Pahwa GS, Kullander S, Vollmer G, Oberheuser F, Knuppen R, Emons G 1991 Specific low affinity binding sites for goandotropin releasing hormone in human endometrial carcinoma. Eur J Obstet Gynecol Reprod Biol 41:135-142; Sealfon SC, Weinstein H, Millar RP 1997 Molecular mechanism of ligand interaction with the gonadotropin-releasing hormone receptor. Endocr Rev 18:180-205; Karten MJ, Rivier JE 1986 Gonadotropin-releasing hormone analog design. Structure-function studies toward the development of agonists and antagonists: Rationale and perspective. Endocr Rev 7:44-66; Siler-Khodr TM, Khodr GS, Valenzuela G, Rhode J 1986 Gonadotropin-releasing hormone effects on placental hormones during gestation: I Alpha-human chorionic gonadotropin, human chorionic gonadotropin and human chorionic somatomammotropin. Biol Reprod 34: 245-254; Kang IS, Koong MK, Forman JS, Siler-Khodr TM 1991 Dose-related action of gonadotropin-releasing hormone on basal prostanoid production from the human term placenta. Am J Obstet Gynecol 165:1771-1776; Gautron JP, Pattou E, Kordon C 1981 Occurrence of higher molecular forms of LHRH in fractionated* *extracts from rat hypothalamus, cortex and placenta. Mol Cell Endocrinol 24:1-15; Mathialagan N, Rao AJ 1986 Gonadotropin releasing hormone in first trimester human placenta: Isolation, partial characterisation and in vitro biosynthesis. J Biosci 10:429-441; Gautron JP, Pattou E, Bauer K, Rotten D, Kordon C 1989 LHRH-like immunoreactivity in the human placenta is not identical to LHRH. Placenta 10: 19-35,- Nowak RA, Wiseman BS, Bahr JM 1984 Identification of a gondotropin-releasing hormone-like factor in the rabbit fetal placenta. Biol Reprod 31:67-75(Abstr.); Siler-Khodr, T.M. 1987. LHRH in pregnancy. In LHRH and Its Analogs: Contraceptive and Therapeutic Applications, Part II. B.H. Vickery and J.J. Nestor, Jr. editors. MTP Press, Ltd. Lancaster. 161-178; Siler-Khodr, T.M. 1988. Hypothalamic-like activities of fetal membranes. In Proceedings of the 1st International Symposium on the Physiology of Human Fetal Membranes. J. Challis and B. Mitchell, editors. Perinatology Press, Ithaca, NY. 91-116.*

Applicants have defined yet another difference in extra-hypothalamic GnRH, i.e., its metabolism. The metabolism of a hormone is as important for maintaining biologically active concentrations of that hormone, as that which stimulates the hormone=s synthesis and release. For GnRH, in the non-pregnant human, both in the pituitary and in the circulation, the predominant enzymatic degradation is directed to the 5-6 peptide bond catalyzed by an endopeptidase. Thus, existing analogs of the mammalian GnRH each bear a D-amino acid substituted in the 6 position. However, Applicants have isolated and characterized the dominant enzyme that degrades GnRH in the placenta and it is a post-proline peptidase acting to cleave the proline-glycine peptide bond at the 9-10 position. Applicants have recently obtained similar data for the metabolism of GnRH in breast tumor cells. Thus, there appears to be cell specific metabolism of GnRH at the placenta and breast tumor cells which differs from that in blood and the pituitary. *Siler-Khodr TM*, *Kang IS, Jones MA*, *Harper MJK*, *Khodr GS, Rhode J 1989 Characterization and purification of a placental protein that inactivates GnRH*, *TRH and* *Angiotensin II. Placenta 10:283-296; Kang IS, Siler-Khodr TM 1992 Chorionic peptidase inactivates GnRH as a post-proline peptidase. Placenta 13: 81-87.*

Since it appeared as though there was a different form of GnRH at work at the placenta and breast tumor cells, various isoforms of GnRH were investigated. Different isoforms of GnRH have been identified in non-mammalian species, such as fish and aves. The unique sequence of these GnRH are known. Chicken I, chicken II, salmon, catfish, dogfish, lamprey and more recently herring GnRH have also been reported. In most cases, each decapeptide conserves the first four, the sixth and in every case, the last two amino acids in the GnRH molecule, but have varying amino acids in the fifth, seventh and/or eighth position. These modifications render the molecule unique, having only weak affinity for the mammalian pituitary receptor, although conversely mammalian GnRH is active in many lower vertebrate classes. *King JA, Millar RP 1995 Evolutionary aspects of gonadotropin-releasing hormone and its receptor. Cell Mol Neurobiol 15:5-23; Carolsfeld J, Powell JFF, Park M, Fischer WH, CraigAG, ChangJP, Rivier JE, Sherwood NM 2000 Primary structure and function of three gonadotropin-releasing hormones, including a novel form, from an ancient teleost, herring. Endocrinology 141:505-512. In lower vertebrates a number of GnRH isoforms can be expressed in the same species Sherwood NM, Lovejoy DA, Coe IR 1993 Origin of mammalian gonadotropin-releasing hormones. Endocr Rev 14: 241-254; Montero M, Le Belle N, King JA, Millar RP, Dufour S 1995 Differential regulation of the two forms of gonadotropin-releasing hormone (mGnRH and cGnRH-II) by sex steroids in the European female silver eel (Anguilla anguilla). Neuroendocrinology 61:525-535; Gothilf Y, Meiri 1, Elizur A, Zohar Y 1997 Preovulatory changes in the levels of three gonadotropin-releasing hormone-encoding messenger ribonucleic acids (mRNAs), gonadotropin B-subunit mRNAs plasma gonadotropin, and steroids in the female gilthead seabream, Sparus aurata. Biol Reprod 57:1145-1154; Powell JF, Reska-Skinner SM,* *Prakash MO, Fischer WH, Park M, Rivier JE, Craig AG, Mackie GO, Sherwood NM 1996 Two new forms of gonadotropin-releasing hormone in a protochordate and the evolutionary implications. Proc Natl Acad Sci USA 93:10461-10464; Powell JF, Zohar Y, ElizurA, ParkM, Fischer WH, Craig AG, Rivier JE, Lovejoy DA, Sherwood NM 1994 Three forms of gonadotropin-releasing hormone characterized from brains of one species. Proc Natl Acad Sci USA 91:12081-12085; Montero M, Vidal B, King JA, Tramu G, Vandesande F, Dufour S, Kah O 1994 Immunocytochemical localization of mammalian GnRH(gonadotropin-releasing hormone) and chicken GnRH-II in the brain of the European silver eel (Anguilla anguilla L.). J Chem Neuroanat 7:227-241; White SA, Kasten TL, Bond CT, Adelman JP, Fernald RD* 1995 *Three gonadotropin-releasing hormone genes in one organism suggest novel roles for an ancient peptide. Proc Natl Acad Sci USA 92:8363-8367; Powell JF, Fischer WH, Park M, Craig AG, Rivier JE, White SA, Francis RC, Fernald RD, Licht P, Warby C, et al 1995 Primary structure of solitary form of gonadotropin-releasing hormone (GnRH) in cichlid pituitary; three forms of GnRH in brain of cichlid and pumpkinseed fish. Regul Pept 57:43-53; Zohar Y, Elizur A, Sherwood NM, Powell JF, Rivier JE, Zmora N 1995 Gonadotropin-releasing activities of the three native forms of gonadotropin-releasing hormone present in the brain ofgilthead seabream, Sparus aurata. Gen Comp Endocrinol 97:289-299; LinXW, Peter RE 1996 Expression of salmon gonadotropin-releasing hormone (GnRH) and chicken GnRH-II precursor messenger ribonucleic acids in the brain and ovary of goldfish. Gen Comp Endocrinol 101:282-296; Di Fiore MM, King JA, D'Aniello B, Rastogi RK 1996 Immunoreactive mammalian and chicken-II GnRHs in Rana esculenta brain during development. Regul Pept 62:119-124; Powell JF, Krueckl SL, Collins PM, Sherwood NM 1996 Molecular forms of GnRH in three model fishes: rockfish, medaka and zebrafish. J Endocrinol 150:17-23; Iela L, Powell JFF, Sherwood NM, D'Aniello B, Rastogi RK, Bagnara JT 1996 Reproduction in the Mexican leaffrog, Pachymedusa dacnicolor.* *VI. Presence and distribution of multiple GnRH forms in the brain. Gen Comp Endocrinol 103: 235-243; Powell JF, Standen EM, Carolsfeld J, Borella MI, Gazola R, Fischer WH, ParkM, Craig AG, Warby CM, Rivier JE, Val-Sella MV, Sherwood NM 1997 Primary structure of three forms of gonadotropin-releasing hormone (GnRH) from the pacu brain. Regul Pept 68:189-195King JA, Millar RP 1995 Evolutionary aspects of gonadotropin-releasing hormone and its receptor. Cell Mol Neurobiol 15:5-23.*

As mentioned, in certain lower vertebrates a number of GnRH isoforms are expressed in the same species. In amphibians, a chicken II GnRH receptor as well as a mammalian GnRH receptor has been reported. However, it was not until 1994, when Dellovade et al. and King et al. described chicken II GnRH in musk shew, mole and bat brain, that the existence of multiple isoforms of GnRH in a mammal was realized. Even then, it was still thought that modern placental mammalian species did not encode or express other than mammalian GnRH. Recently however, chicken II GnRH has been characterized in the tree shew, guinea pig, and primate brain and their separate genes have been described. We have recently demonstrated the production of GnRH and the presence and activity of a specific GnRH II receptor in numerous non-hypothalamic tissues of primates and humans. Only this year has the code for the chicken II GnRH receptor been identified in human tissues, although its protein expression and function has been questioned. *White SA, Bond CT, Francis RC, Kasten TL, Fernald RD, Adelman JP 1994 A second gene for gonadotropin-releasing hormone: cDNA and expression pattern in the brain. Proc Natl Acad Sci USA 91:1423-1427; SLinXW, Peter RE 1997 Cloning and expression pattern of a second [His5Trp7Tyr8]gonadotropin-releasing hormone (chicken GnRH-H-II) mRNA in goldfish: evidence for two distinct genes. Gen Comp Endocrinol 107:262-272; Dellovade TL, King JA, Millar RP, Rissman EF 1993 Presence and differential distribution of distinct forms of immunoreactive gonadotropin-re leasing hormone in the musk shrew brain. Neuroendocrinology* *58:166-177 King JA, Steneveld AA, Curlewis JD, Rissman EF, Millar RP 1994 Identification of chicken GnRH II in brains of metatherian and early-evolved eutherian species of mammals. Regul Pept 54:467-477; Kasten TL, White SA, Norton TT, Bond CT, Adelman JP, Fernald RD 1996 Characterization of two new preproGnRH mRNAs in the tree shrew: first direct evidence for mesencephalic GnRH gene expression in a placental mammal. Gen Comp Endocrinol 104:7-19; Jimenez-Linan M, Rubin BS, King JC 1997 Examination of guinea pig luteinizing hormone-releasing hormone gene reveals a unique decapeptide and existence of two transcripts in the brain. Endocrinology 138:4123-4130.*

In contrast, Applicants have proposed and obtained substantial data to support the hypothesis that non-mammalian isoforms ofGnRH and their specific receptors are expressed in extra-hypothalamic tissues and that the non-mammalian GnRH molecules are the true ligands for these receptors. Applicants have also proposed that these GnRH molecules have specific roles in regulating cell growth and cell death and are pivotal in regulating cell growth of GnRH responsive tumors by a direct receptor mediated action on these tumor cells. *Siler-Khodr TM, Grayson M 1999 Comparison of GnRH and its synthetic and naturally occurring analogs for binding to the human placental receptor. J Soc Gynecol Invest 6:225A(Abstr.); Siler-Khodr TM, Grayson M 2000 Inhibiton of human trophoblast function by superagonists of chicken II GnRH. J Soc Gynecol Invest 7:280A(Abstr.).*

It is believed that the non-mammalian GnRH isoforms and analogs of the present invention may act either as a superagonist at the tumor tissue leading to tissue receptor down-regulation, or as a pure antagonist of the endogenous isoform of GnRH in the tumor tissue, acting via the tumor tissue receptor. The down-regulation of the GnRH receptor or the antagonism of the endogenous isoform of GnRH will provide for a reduction in cell proliferation and/or induce apoptosis. The specific action of the non-mammalian GnRH analog will compete at the tumor cell GnRH receptor(s) with the endogenous isoform of GnRH effecting an antagonism or a superagonistic down-regulation of the receptor, leading to cell death and regression of the tumor and inhibition of metastasis. Thus, this agent may be used to reduce tumor growth. To date, no such non-mammalian GnRH analog has been designed which has stability and tumor tissue specificity.

To date, little if any data, has been reported in relation to non-mammalian GnRH activity on cell proliferation or tumor tissues. Chicken I GnRH and Lamprey have been studied and limited activity was found. Applicants have studied these isoforms of GnRH and have found no or limited binding activity in chorionic tissues or ovarian tissues. On the other hand, Applicants have demonstrated greatly enhanced binding and bioactivity of chicken II GnRH and salmon GnRH analogs as compared to mammalian GnRH or its analogs in both breast cancer cells and placental tissue. Thus, Applicants have obtained data to support the hypothesis that certain non-mammalian GnRH analogs have enhanced receptor and bioactivity for tumor tissues and this finding taken together with the understanding of the unique metabolism of GnRH isoforms in cell specific sites have formed the basis of Applicants invention, i.e., the utilization of stable, cell-active analogs of non-mammalian GnRH isoforms to regulate tumor cell growth and the treatment of cancer. In addition, Applicants postulate that due to similar amino acid structures, Herring GnRH, Dogfish GnRH, and Catfish GnRH as well as other GnRH isoforms and analogs with similar amino acid structure should exhibit the same or similar binding and bioactivity. *GnRHPalyi I, Vincze B, Kalnay A, Turi G, Mezo I Teplan I Seprodi J, Pato J, Mora M 1996 Effect of gonadotropin-releasing hormone analogs and their conjugates on gonadotropin-releasing hormone receptor-positive human cancer cell lines. Cancer Detect Prev 20:146-152; Vincze B, Palyi I, Gaal D, Pato J, Mora M, Mezo I, Teplan I, Seprodi J1996In vivo studies of the new gonadotropin-releasing hormone antagonist-copolymer conjugates having* *antitumor activity. Cancer Detect Prev 20:153-159; Mezo I Seprodi J, Vincze B, Palyi I Keri G, Yadasz Z, Toth G, Kovacs M, Koppan M, Horvath JE, Kalnay A, Teplan I 1996 Synthesis of GnRH analogs having direct antitumor and low LH-releasing activity. Biomedical Peptides, Proteins & Nucleic Acids 2:33-40; Mezo I, Lovas S, Palyi I, Vincze B, Kalnay A, Turi G, Vadasz Z, Seprodi J, Idei M, Toth G, Gulyas E, Otvos F, MakM, Horvath JE, Teplan I, Murphy RF 1997 Synthesis of gonadotropin-releasing hormone III analogs. Structure-antitumor activity relationships. J Med Chem 40:3353-3358.*

### SUMMARY OF THE INVENTION

The present invention, in a general and overall sense, relates to novel pharmaceutical preparations that include non-mammalian gonadotropin releasing hormone (GnRH) and its analogs specifically designed to bind human chorionic, ovarian, fallopian tube, and uterine tissue GnRH receptors as well as human sperm, testicular, scrotal, seminiferous tubule, Leydig cell, Sertoli cell, epididymis, vas deferentia, prostate, seminal vesicle, ejaculatory duct, urethral GnRH receptors and extra-pituitary GnRH receptors or those extra-pituitary GnRH receptors expressed on tumor tissues. These analogs are designed to be resistant to degradation by post-proline peptidases and endopeptidases. Post-proline peptidases have been found to specifically and very actively degrade GnRH in chorionic, ovarian, tubal, and uterine tissues and maternal blood, and extra-pituitary cells and tumor tissue.

The non-mammalian GnRH or its analogs of the present invention may act either as a superagonist at the placental, ovarian, tubal, uterine, male reproductive tissues, extra-pituitary cells or tumor tissue GnRH receptor leading to acute stimulation then to its down regulation, or as a pure antagonist at the chorionic, ovarian, tubal, uterine, sperm, testicular, scrotal, seminiferous tubule, Leydig cell, Sertoli cell, epididymis, vas deferentia, prostate, seminal vesicle, ejaculatory duct, urethral, extra-pituitary, or tumor tissue GnRH receptor. The down-regulation or antagonism of endogenous chorionic GnRH will provide for a reduction in human chorionic gonadotropin (hCG) production and other direct actions. This will also provide a reduction in ovarian and placental steroidogenesis. In addition, a direct ovarian luteolytic action may be expected to occur. If trophoblastic and/or ovarian function is jeopardized at certain doses, premature luteolytic action will occur. If trophoblastic and/or ovarian function is jeopardized, premature luteolysis of the corpus luteum will occur and menses will ensue. The down-regulation or antagonism of endogenous GnRH activity at the testis will provide for a reduction in testosterone production and will affect sperm function. As a superagonist, the GnRH analog is also expected to act at the sperm, testicles, scrotum, seminiferous tubule, Leydig cell, Sertoli cell, epididymis, vas deferentia, prostate, seminal vesicle, ejaculatory duct, and urethra to affect these tissues function and thus again affect sperm function. The down-regulation or antagonism of endogenous GnRH activity at the extra-pituitary cell or at the tumor tissue will provide for a reduction in cell proliferation and will affect cell or tumor function. As a superagonist, the GnRH analog is also expected to act at the proliferating cell or tumor to reduce growth. Thus, such as agent may be used as an anti-growth, anti-tumor agent.

Thus, such an agent may be used as a post-coital, luteolytic and anti-sperm agent, leading to the induction of menses and sperm inactivation. Until now, no such non-mammalian GnRH or its analog has been found to be active during pregnancy or at the ovary or on the testis or sperm. In addition, maturation of the egg and sperm and the process of ovulation, as well as the process of fertilization and maturation of the fertilized egg and sperm, will be affected. Sperm capacitation in the male and female tracts and fertilizing capability will be affected. The activity of the fallopian tube will be affected altering transport and maturation of the morula during transit. In addition, uterine hormone and cell functions will be affected both directly and indirectly by non-mammalian GnRH or its analogs. PGE production is decreased which will lead to decreased vaso-function and vasodilation. The uterine environment will be made hostile to implantation of the blastocyst or the maintenance of pregnancy. The regression of uterine endometrial tissue will result.

The inventor has designed non-mammalian GnRH analogs that are active as luteolytic, menses-inducing agents, and anti-sperm agents, post-coital contraceptives, and/or anti-proliferation or anti-tumor agents. The chorionic, ovarian, and uterine and sperm, testicular, scrotal, seminiferous tubule, Leydig cell, Sertoli cell, epididymis, vas deferentia, prostate, seminal vesicle, ejaculatory duct, urethral, and extra-pituitary and tumor receptor binding activity of these particularly designed non-mammalian GnRH analogs and or the non-mammalian GnRH isoforms has also been characterized in the development of the present analogs. The analogs of the invention may be further defined as long-acting or resistant to enzymatic degradation by blood, ovarian, uterine, and placental and sperm, testicular, scrotal, seminiferous tubule, Leydig cell, Sertoli cell, epididymis, vas deferentia, prostate, seminal vesicle, ejaculatory duct, urethral and extra-pituitary cells and tumor cell enzymatic activity by specific endopeptidase and post-proline peptidase, such as C-ase-1. The chronic administration of the non-mammalian GnRH or its agonist and antagonists with the greatest receptor affinity and tissue stability are expected to effectively inhibit hCG and progesterone release from human placenta and ovary, and PGE production from fallopian tubes and uterine tissues and testosterone from the testis and tissue functions of these cells and testicular, sperm prostate, seminal vesicle, epididymis and extra-pitutiary proliferation cells and tumor tissue growth in those cells having GnRH receptors. The non-mammalian GnRH or its analogs of the invention may be used to inhibit placental production of hCG and progesterone, and have a direct effect on steroidogenesis at the ovary and prostaglandins in the fallopian tubes and uterus, or to directly effect male reproductive tissue function or cell proliferation or tumor growth and function. The effects of the analogs may thus be used to induce luteolysis and menses-induction and anti-implantation, anti-pregnancy activity, regulation male fertility or cell proliferation or tumor growth.

In one aspect, the invention provides methods of designing analogs of non-mammalian GnRH having increased activity in the chorionic tissues. Methods to inhibit hCG production by placental tissues, that in turn provide a reduction of ovarian and placental steroidogenesis, i.e., luteolysis and menses-induction, are provided in another aspect of the present invention. The use of these analogs directly on the ovary is another particular embodiment of the invention. The use of these analogs to directly affect fallopian tube function is still another embodiment of the invention. The use of these analogs to alter uterine prostaglandin production is yet another embodiment of the invention. Another aspect of the invention is decreasing testosterone, sperm viability, or capacitation. Another aspect is the ability to regulate cell function or tumor function. The analogs of this invention may be used in pharmaceutical preparations as a menses-regulating agent, a contraceptive, or as an abortifacient.

Non-mammalian GnRH analogs that are superagonists or antagonists at the trophoblastic/placental, ovarian, tubal and/or uterine level constitute yet other embodiments of the invention. Such a non-mammalian analog would provide for the inhibition of steroidogenesis during pregnancy, acting both as an anti-chorionic and anti-luteal agent by inhibiting steroidogenesis or at the tubal or uterine level to inhibit PGE production leading to menses induction. The non-mammalian GnRH analogs of the invention thus comprise peptides that are capable of specifically binding the chorionic, ovarian, fallopian tubes and/or uterine, male reproductive tissues, extra-pituitary cells and tumor tissues with GnRH receptors with high affinity, are resistant to degradation by endopeptidase and post-proline peptidase activity and effect either a down-regulation of the GnRH receptor or act as a true antagonist, inhibiting hCG production and ovarian and placental steroidogenesis or directly inhibiting ovarian steroidogenesis and/or inhibiting tubal and/or uterine prostaglandin production, and testicular, sperm prostate, seminal vesicle, epididymis function, or regulating cell proliferation or tumor growth and function. In other embodiments, the invention comprises a salmon sequence (SEQ ID NO: 4) or chicken II GnRH sequence (SEQ ID NO: 2), which both show greater affinity for the placental, ovarian, uterine, testicular, sperm, prostate, seminal vesicle, and epididymis, extra-pituitary cells or tumor tissue receptor than mammalian GnRH, and are modified at the C-terminal. An ethylamide or aza-Gly¹⁰-NH₂ substitution may be used, making the sequence more stable in chorionic, ovarian, tubal, uterine, testicular, sperm, prostate, seminal vesicle, and epididymis tissues and maternal blood, and in extra-pituitary cells and tumor tissues.

In other embodiments the GnRH analog sequence is substituted at the 6-position with a D-Arg, or other D-amino acid. In yet other embodiments, both of these modifications are made to the GnRH analog peptide sequence. The chicken II or salmon backbone and the substitutions of the molecule are expected to enhance the binding of the molecule, while at the same time the substitutions are designed to inhibit any of the peptidases that are present in blood. These analogs are expected to have increased binding to the placental, ovarian, fallopian tube, uterine, sperm, testicular, scrotal, seminiferous tubule, Leydig cell, Sertoli cell, epididymis, vas deferentia, prostate, seminal vesicle, ejaculatory duct, and urethral, extra-pituitary cell and tumor tissue receptor and increased metabolic stability. The placental receptor binding, placental metabolic degradation and the biological activity for hCG, progesterone and prostaglandin production were studied for each of these specially designed non-mammalian GnRH analogs, and compared to closely related pituitary mammalian GnRH analogs (Buserilin, Tryptolein, Leuprolide, etc.). These studies demonstrated greater stability of the non-mammalian GnRH or its analogs, binding affinity and bioactivity compared to the mammalian GnRH analogs examined. The ovarian receptor binding, ovarian metabolic degradation, and the biological activity for progesterone production were studied for each of the specially designed non-mammalian GnRH analogs or non-mammalian GnRH, and compared to closely related mammalian GnRH analogs. These studies demonstrated greater stability, binding affinity, and bioactivity of the non-mammalian GnRH or its analogs compared to the mammalian GnRH analogs examined. The uterine receptor binding and biological activity for the prostaglandin E production were studied for these specially designed non-mammalian GnRH analogs or non-mammalian GnRH and compared to closely related mammalian GnRH analogs. These studies demonstrated greater binding affinity and bioactivity on the non-mammalian GnRH or its analogs compared to the mammalian GnRH analogs examined. The tumor receptor binding, tumor metabolic degradation, and the biological activity on cell proliferation were studied for each of the specially designed non-mammalian GnRH analogs and non-mammalian GnRH, and compared to closely related mammalian GnRH analogs. These studies demonstrated greater stability, binding affinity, and bioactivity of the non-mammalian GnRH or its analogs compared to the mammalian GnRH analogs examined

In other embodiments, the invention provides non-mammalian GnRH or its analogs with enhanced activity within the uterine, as well as a method for regulating hCG production and thus progesterone production during pregnancy, as well as other cell functions. The activity of non-mammalian GnRH or these analogs may be useful in the management of threatened abortion or the induction of abortions. Activity of these analogs may also be useful in the management of abnormal pregnancies, ectopic pregnancies, molar pregnancies, or trophoblastic disease. These non-mammalian GnRH analogs also have a direct action on endometrial tissue. This activity may prove beneficial in treatments for endometriosis, abnormal uterine bleeding, and leiomyomas. These non-mammalian GnRH analogs also have a direct action at the ovary. Such action may prove useful in the manufacture of treatments for ovarian conditions, such as polycystic ovarian disease, ovarian cysts, atresia, used in in vitro fertilization programs or for the induction of luteolysis. Luteolysis may be affected by a dual mechanism i.e., through inhibition of hCG and thus reduction of ovarian steroidogenesis and/or direct inhibition of ovarian steroidogenesis. This will be useful to induce menses and as a contraceptive.

In other embodiments, the invention provides non-mammalian GnRH or its analogs with enhanced activity within the sperm, testicular, scrotal, seminiferous tubule, Leydig cell, Sertoli cell, epididymis, vas deferentia, prostate, seminal vesicle, ejaculatory duct, and urethral tissues. The activity of non-mammalian GnRH or these analogs may be useful in the management of disease and a list of male diseases, impotence, undescented testis, male infertility, azo- or oligospermia and the like.

In other embodiments, the invention provides non-mammalian GnRH or its analogs with enhanced activity within the extra-pituitary cells and tumor tissues. The activity of non-mammalian GnRH or these analogs may be useful in the management of disease or cell proliferation and tumor growth and function and the like.

It is envisioned that non-mammalian GnRH and its analogs will be administered intra-nasally, orally, intramuscularly, intrauterine, subcutaneously, transdermally or vaginally. However, virtually any mode of administration may be used in the practice of the invention. Treatment with these long-acting non-mammalian GnRH isoforms or their analogs may require one to three days of active non-mammalian GnRH analog when used as a post coital contraceptive, but could be continuous. As a monthly contraceptive, the placebo is envisioned to start on the first day of menses and continue for approximately 13 days, then the analog would be given days 13 through 28, or less to suppress luteal and/or endometrial and anti-sperm function and to induce menses. This could be repeated monthly. Use as a post-coital agent could be following coitus. Treatment for male reproductive function or regulation of cell proliferation or tumor growth or function could be chronic or intermitant.

Numerous IVF protocols now routinely use mammalian GnRH analogs for ovulation timing and have been shown to be nontoxic, even after weeks of administration. Long-term therapies with mammalian GnRH analogs have been associated with a hypoestrogenic state, but in the envisioned modes of administration of the present non-mammalian GnRH analogs, exposure would not exceed three days to two weeks. The effect on the mammalian GnRH receptor is expected to be minimal with non-mammalian GnRH or its analogs and with this short duration of treatment, the menstrual cycle may not be altered. Thus, the limited time of exposure in the early to late luteal phase and the specific receptor activity of these analogs make it less likely to interfere with reproductive cyclicity and/or normal physiology. The design of the present non-mammalian GnRH or its analogs considers the specific metabolism of GnRH at extra-pituitary tissues, such as the ovary, fallopian tubes, uterus, placenta, testicle, sperm, prostate, and seminal vesicle, and during pregnancy in maternal blood and in extra-pituitary cells and tumor tissues.

Another embodiment of the invention provides non-mammalian GnRH or its analogs that are resistant to degradation by post-proline peptidases and endopeptidases. This non-mammalian GnRH or its analog will bind the chorionic, ovarian, tubal, and uterine, sperm, prostate, and seminal vesicle, and in extra-pituitary cells and tumor tissues GnRH receptor or non-mammalian GnRH receptor of other tissues with high affinity so to first stimulate then down-regulate the receptor to displace the endogenous GnRH-like activity and block its action.

In another aspect, the invention provides more potent non-mammalian GnRH analogs that will specifically bind to the placental, ovarian, tubal, uterine, sperm, testicular, scrotal, seminiferous tubule, Leydig cell, Sertoli cell, epididymis, vas deferentia, prostate, seminal vesicle, ejaculatory duct, and urethral and extra-pituitary cells and tumor tissue GnRH receptor. In addition, non-mammalian GnRH analogs will be provided that are stable in maternal circulation and in the blood of non-pregnant individuals. It is also anticipated that these non-mammalian GnRH analogs will be biologically active in chorionic tissues, at the ovary, fallopian tube, uterus, sperm, testicles, scrotum, seminiferous tubule, Leydig cell, Sertoli cell, epididymis, vas deferentia, prostate, seminal vesicle, ejaculatory duct, and urethra, and extra-pituitary cells and tumor tissue in the regulation of hormonogenesis that will affect the maintenance of pregnancy and/or the receptivity of the uterus for implantation, or male reproductive function or cell proliferation or tumor growth and function. Due to the specificity of these non-mammalian GnRH analogs and their relatively long half-life, the present invention provides non-mammalian GnRH analogs.

Still in another embodiment it is expected that the human may contain another GnRH defined as salmon GnRH which contains the sequence or a degenerate variant of Salmo salar.

Other proline-containing peptides compete for post-proline peptidase activity, such as angiotensin II, and to a lesser extent, thyrotropin releasing hormone and reduced oxytocin. The existing mammalian GnRH analogs are also proline-containing molecules. Since human pituitary and blood contain an enzymatic activity that degrades GnRH at primarily the 5-6 position, not at the 9-10 position, the present non-mammalian GnRH analogs have been designed to inhibit the former enzymatic activities, and have substitutions in the 5-6 position of the molecule. Some of the present non-mammalian GnRH analogs also have a substitution at the 10 position with an ethylamide which is only a weak inhibitor of the post-proline peptidase. The present non-mammalian GnRH analogs are therefore, resistant to degradation at the pituitary or in the blood of non-pregnant individuals, but not the ovary, fallopian tube, uterus, placenta, testicle, sperm, prostate, seminal vesicle, or in maternal blood. Substitution of the Gly¹⁰-NH₂ with ethylamide is only slightly effective at the placenta, fallopian tube, uterus, ovary, sperm, testicle, scrotum, seminiferous tubule, Leydig cell, Sertoli cell, epididymis, vas deferentia, prostate, seminal vesicle, ejaculatory duct, and urethra, but the even more potent aza-Gly¹⁰-NH₂, inhibits degradation by post-proline peptidase. *Siler-Khodr TM, Kang IS, Jones MA, Harper MJK, Khodr GS, Rhode J 1989 Characterization and purification of a placental protein that inactivates GnRH, TRH and Angiotensin 11. Placenta 10: 283-296; Siler-Khodr TM, Grayson M, Pena A, Khodr T 1997 Definition of enzyme specificity of chorionic peptidase-1 for GnRH, TRH, oxytocin and angiotensin 11. J Soc Gynecol Invest 4:129A(Abstr.). Benuck M, Marka N 1976 Differences in the degradation of hypothalamic releasing factors by rat and human serum. Life Sci 19:1271-1276. Zohar Y, Goren A, Fridkin M, Elhanati E, Koch Y 1990 Degradation of gonadotropin-releasing hormones in the gilthead seabrearn, Sparus aurata. 11. Cleavage of native salmon GnRH, mammalian LHRH, and their analogs in the pituitary, kidney, and liver. Gen Comp Endocrinol 79:306-319.*

The stability of the present non-mammalian GnRH analogs in the presence of C-ase-1 and ovarian tissues was also examined. The degradation of four of these analogs was examined using a competitive inhibition assay for GnRH. While replacement of Gly¹⁰-NH₂ with ethylamide made each of these non-mammalian GnRH analogs more resistant to degradation, some of the analogs still effected a substantial competition with GnRH demonstrating that they could be degraded. Of four ethylamides studied, des-Gly¹⁰-GnRH-ethylamide, the des-Gly¹⁰, D-Leu⁶-GnRH-ethylamide, or Buserilin, each were potent competitive inhibitors of GnRH degradation by C-ase-1. The less active an analog is as a competitor for GnRH degradation by C-ase-1, the more stable that analog will be in the. ovarian, endometrial, chorionic, testicular, sperm, prostate, and seminal vesicle tissues, and in maternal blood, and extra-pituitary cells and tumor tissue. Thus, the existing mammalian GnRH analogs commonly used in medicine can be degraded in the ovarian, endometrial, chorionic, testicular, sperm, prostate, and seminal vesicle tissues, and in maternal blood, and extra-pituitary cells and tumor tissues.

The findings of inhibition of placental, ovarian, and uterine function can be explained by recognizing that the decapeptide sequence for mammalian GnRH is not the only active GnRH sequence in ovarian, fallopian tube, uterine, and chorionic GnRH, male reproductive tissues, and extra-pituitary cells and tumor tissue. Substantial data exists that in these tissues there is a receptor and there is a GnRH of which the chemical nature is not identical to mammalian GnRH. Postulating that a different ovarian, fallopian tube, uterine, sperm, testicular, scrotal, seminiferous tubule, Leydig cell, Sertoli cell, epididymis, vas deferentia, prostate, seminal vesicle, ejaculatory duct, urethral, chorionic or extra-pituitary cell and tumor tissues GnRH from the mammalian GnRH exists, and that there is an ovarian, fallopian tube, uterine, sperm, testicular, scrotal, seminiferous tubule, Leydig cell, Sertoli cell, epididymis, vas deferentia, prostate, seminal vesicle, ejaculatory duct, urethral, placental, or extra-pituitary cell and tumor tissue receptor that prefers this ovarian, tubal, uterine, sperm, testicular, scrotal, seminiferous tubule, Leydig cell, Sertoli cell, epididymis, vas deferentia, prostate, seminal vesicle, ejaculatory duct, urethral, chorionic GnRH or extra-pituitary cells and tumor tissue, explains the biphasic response of placental hormones to mammalian GnRH. Mammalian GnRH acts as a partial agonist of non-mammalian GnRH. When receptors are available, it acts as an agonist of ovarian, tubal, uterine, sperm, testicular, scrotal, seminiferous tubule, Leydig cell, Sertoli cell, epididymis, vas deferentia, prostate, seminal vesicle, ejaculatory duct, urethral, chorionic GnRH or extra-pituitary cell and tumor tissue. When ovarian, tubal, uterine, sperm, testicular, scrotal, seminiferous tubule, Leydig cell, Sertoli cell, epididymis, vas deferentia, prostate, seminal vesicle, ejaculatory duct, urethral, placental or extra-pituitary cells and tumor tissue receptors are low or occupied, mammalian GnRH competes with the more potent non-mammalian GnRH resulting in an antagonistic action.

GnRH-like substances have been found by the present inventor to be decreased at mid-pregnancy in women who later have pre-term labor, and increased in those with post-term deliveries. In more recent studies, a GnRH binding substance has been demonstrated in their circulation and in these cases hCG was abnormally reduced and pregnancy loss was observed. Thus, the current studies of GnRH-like substance production during pregnancy indicate that chorionic GnRH is of significance to the maintenance of normal pregnancy.

Mammalian GnRH analogs, ZOLADEX™ (Goserelin acetate) and Organon 30276, were administered to pregnant baboons via mini-pump on days 14 through 21 post ovulation. The hormonal release and pregnancy outcome was compared to saline treated controls. CG and progesterone decreased, and in most animals pregnancy outcomes were jeopardized. However, using these analogs, abortions were not consistently effected, except for the 100 mg - 7 day regiment of the Organon antagonist. In a dose-response saline-controlled study using very high doses of mammalian GnRH analog, a small stimulation of hCG in very early pregnancy was observed by the present inventor. However, an inhibition of hCG and progesterone was observed by 12 weeks of pregnancy when chorionic GnRH is maximal. Further studies with these newly designed non-mammalian GnRH analogs or longacting non-mammalian GnRH having enhanced receptor activity and ovarian, endometrial, sperm, testicular, scrotal, seminiferous tubule, Leydig cell, Sertoli cell, epididymis, vas deferentia, prostate, seminal vesicle, ejaculatory duct, urethral, and/or chorionic and extra-pituitary cells and tumor tissue stability promise to provide a much more potent action.

The present inventor has found that certain non-mammalian GnRH and its analogs can act on the specific ovarian, uterine, and chorionic non-mammalian GnRH receptor, and with high affinity binding, affect changes in the ovarian and/or intrauterine environment that effect fertility, reproductive function, and the outcome of pregnancy. This finding is the basis of the invention disclosed herein. Thus, the present investigator has developed particular (non-mammalian) GnRH analogs that can be used for regulation of ovarian, tubal, and uterine function, induction of luteolysis and menstruation, and regulation of uterine PGE production. The ability of specific (non-mammalian) GnRH analogs to interact with the physiologic regulation of hCG, progesterone and prostaglandin during luteal phase of the cycle and early pregnancy, may be used to specifically interrupt ovarian and luteal function and early pregnancy according to the invention as outlined here.

In additional embodiments, the specificity, activity and stability of non-mammalian GnRH and its analogs were investigated at the ovary, the endometrium and the pituitary and their acute action was assessed on chorionic tissues. A direct action on ovarian and endometrial tissue was found. A potential direct contraceptive action of these analogs, as well as their placental hCG stimulation followed by inhibition and steroidogenic suppression activity is indicated. Such analogs could be used to regulate reproductive functions and disorders, used as menses regulators, contraceptives, or as abortifacients.

In addition, the present invention relates to novel preparations of non-mammalian GnRH and its analogs that can be useful in male fertility regulation essentially acting as a male contraceptive agent. This male contraceptive agent can act within the male reproductive system to reduce or eliminate sperm production or to disable the motility and travel of the sperm through the male reproductive system. In addition, the present preparations can provide a reduction in testosterone production.

In another embodiment the male contraceptive agent can be introduced into the female along with the semen upon the male's ejaculation during coitus. This non-mammalian GnRH or its analog may lead to sperm inactivation or inability to capacitate and thus induce infertility. Once the non-mammalian GnRH analog of the present invention is introduced into the female, it may act either as a superagonist at the placental, ovarian, tubal, or uterine receptor leading to its down regulation, or as a pure antagonist of chorionic, ovarian, tubal, or uterine GnRH at the GnRH receptor. The down-regulation or antagonism of endogenous GnRH will provide for a reduction in human chorionic gonadotropin (hCG) production. This will also provide a reduction in ovarian and placental steroidogenesis and other activities. In addition, a direct ovarian luteolytic action may be expected to occur. If trophoblastic and/or ovarian function is jeopardized, premature luteolytic action will occur. If trophoblastic and/or ovarian function is jeopardized, premature luteolysis of the corpus luteum will occur and menses will ensue. Thus, such an agent may be used as a post-coital, luteolytic agent, leading to the induction of menses in the female. In addition, maturation of the egg and the process of ovulation, as well as the process of fertilization and maturation of the fertilized egg, will be affected. The ability of the sperm to capacitate or to bind or fertilize the egg may be affected. The activity of the fallopian tube will be affected altering transport and maturation of the morula during transit. In addition, uterine hormone and cell functions will be affected. PGE production will be decreased which will lead to decreased vaso-function and vasodilation. The uterine environment will be made hostile to implantation of the blastocyst or the maintenance of pregnancy. The regression of uterine endometrial tissue will result.

An additional embodiment, the specificity, activity and stability of non-mammalian GnRH and its analogs were investigated in cancer cells and their action on proliferation was assessed. A direct action on tumor cells was found. A potential direct anti-cell proliferation and anti-tumor growth and function activity of these analogs, is indicated. Such analogs could be used to regulate cell proliferation and tumor growth.

In addition, the present invention relates to novel pharmaceutical preparations that include non-mammalian gonadotropin releasing hormone (GnRH) and its analogs and any biomimetic or chemomimetic agents, i.e., functional mimetics of the present non-mammalian GnRH analogs specifically designed to bind to GnRH receptors in the female and male reproductive system including human sperm, testicular, scrotal, seminiferous tubule, Leydig cell, Sertoli cell, epididymis, vas deferentia, prostate, seminal vesicle, ejaculatory duct, and urethral GnRH, and extra-pituitary cells and tumor tissue receptors. Any functional mimetics may be used for any purpose as the non-mammalian GnRH or its analogs of the present invention which can include, among other things, antagonizing the activity of GnRH receptor or as an antigen in a manner described elsewhere herein. Functional mimetics of the non-mammalian GnRH or its analog of the present invention include but are not limited to truncated polypeptides or synthetic organic or inorganic molecules comprising a comparable GnRH receptor binding site. Polynucleotides encoding each of these functional mimetics may be used as expression cassettes to express each mimetic polypeptide. It is preferred that these cassettes comprise 5' and 3' restriction sites to allow for a convenient means to ligate the cassettes together when desired. It is further preferred that these cassettes comprise gene expression signals known in the art or described elsewhere herein. These analogs and mimetics are designed to be resistant to degradation by post-proline peptidases and endopeptidases. Post-proline peptidases have been found to specifically and very actively degrade GnRH in female and male reproductive system tissues, and extra-pituitary cells and tumor tissue.

Other proline-containing peptides compete for post-proline peptidase activity, such as angiotensin II, and to a lesser extent, thyrotropin releasing hormone and reduced oxytocin. The existing mammalian GnRH analogs are also proline-containing molecules. Since human pituitary and blood contain an enzymatic activity that degrades GnRH at the 5-6 position, not at the 9-10 position, the present non-mammalian GnRH analogs have been designed to inhibit the former enzymatic activities, and have substitutions in the 5-6 position of the molecule. Some of the analogs also have a substitution at the 10 position with an ethylamide which is only a weak inhibitor of the post-proline peptidase. The present mammalian analogs are therefore, resistant to degradation at the pituitary or in the blood, seminal fluid, or vaginal fluid of individuals, and extra-pituitary cells and tumor tissue. The even more potent aza-Gly¹⁰-NH₂, inhibits degradation by post-proline peptidase. *Zohar Y, Goren A, Fridkin M, Elhanati E, Koch Y 1990 Degradation of gonadotropin-releasing hormones in the gilthead seabrearn, Sparus aurata. 11. Cleavage of native salmon GnRH, mammalian LHRH, and their analogs in the pituitary, kidney, and liver. Gen Comp Endocrinol 79:306-319. Siler-Khodr TM*, *Kang IS, Jones MA*, *Harper MJK*, *Khodr GS, Rhode J 1989 Characterization and purification of a placental protein that inactivates GnRH*, *TRH and Angiotensin 11*. *Placenta 10:283-296; Siler-Khodr TM*, *Grayson M*, *Pena A, Khodr T 1997 Definition of enzyme specificity of chorionic peptidase-1 for GnRH, TRH, oxytocin and angiotensin 11. JSoc* *Gynecol Invest 4:129A(Abstr.). Benuck M, Marka N 1976 Differences in the degradation of hypothalamic releasingfactors by rat and human serum. Life Sci 19:1271-1276.*

The non-mammalian GnRH and its analogs of the present invention may act either as a superagonist at the female reproductive tissues, sperm, testicular, scrotal, seminiferous tubule, Leydig cell, Sertoli cell, epididymis, vas deferentia, prostate, seminal vesicle, ejaculatory duct, urethral or extra-pituitary cells and tumor tissue receptor leading to its down regulation, or as a pure antagonist of female reproductive tissues, sperm, testicular, scrotal, seminiferous tubule, Leydig cell, Sertoli cell, epididymis, vas deferentia, prostate, seminal vesicle, ejaculatory duct, urethra, or extra-pituitary cells and tumor tissue GnRH at the GnRH receptor.

In other embodiments, the invention comprises a salmon sequence (SEQ ID NO: 4) or chicken II GnRH sequence (SEQ ID NO: 2), which both show greater affinity for the female reproductive tissues, sperm, testicular, scrotal, seminiferous tubule, Leydig cell, Sertoli cell, epididymis, vas deferentia, prostate, seminal vesicle, ejaculatory duct, urethra, or extra-pituitary cells and tumor tissue receptor than mammalian GnRH, that are modified at the C-terminal. An ethylamide or aza-Gly¹⁰-NH₂ substitution may be used, making the sequence more stable in female reproductive tissues, sperm, testicular, scrotal, seminiferous tubule, Leydig cell, Sertoli cell, epididymis, vas deferentia, prostate, seminal vesicle, ejaculatory duct, and urethral, and extra-pituitary cells and tumor tissue tissues. In other embodiments the non-mammalian GnRH or its analog sequence (SEQ ID NO: 4 and SEQ ID NO: 2) is substituted at the 6-position with a D-Arg, or other D-amino acid. In yet other embodiments, both of these modifications are made to the non-mammalian GnRH peptide sequence. The chicken II or salmon backbone and the substitutions of the molecule are expected to enhance the binding of the non-mammalian GnRH analog, while at the same time the substitutions are designed to inhibit any of the peptidases that are present in blood, seminal fluid, or vaginal fluid. These non-mammalian GnRH analogs are expected to have increased binding to female reproductive tissues the sperm, testicular, scrotal, seminiferous tubule, Leydig cell, Sertoli cell, epididymis, vas deferentia, prostate, seminal vesicle, ejaculatory duct, urethral, or extra-pituitary cells and tumor tissue receptor and increased metabolic stability.

Another object of the present invention is to provide non-mammalian GnRH or its analogs which regulate cell functions and are used as tumor tissue specific anti-tumor agents to reduce tumor cell growth and proliferation, induce apoptosis, and reduce tumor cell metastasis. These novel GnRH analogs have increased activity in tumor tissues and can be used to reduce tumor cell growth. Still another object of the present invention is to provide a novel method for synthesizing analogs of non-mammalian GnRH isoforms. A novel method is also provided for inhibiting tumor growth which in turn reduces tumor cell proliferation, tumor size and metastasis i.e. apoptosis and tumor regression. These non-mammalian GnRH analogs are used directly on tumors as an anti-tumor or anti-metastasis drug.

The novel non-mammalian GnRH or its analog is composed of Salmon, Chicken II, or Herring GnRH or other non-mammalian isoforms or their analogs that are modified at the C-terminal or otherwise made to be longacting and show greater affinity for the tumor GnRH receptors than mammalian GnRH. These analogs can have an aza-Gly-NH₂ substitution at the number 10 position to make the sequence more stable in tumor tissues and in blood and to inhibit degradation by post-proline peptidases. The analogs can be also substituted at the 6 position with preferably a D-Arg but could be any other D-amino acid such as, but not limited to, D-Leu, D-Trp, and D-Bu-Ser. The GnRH analog has increased binding affinity to the tumor receptor and metabolic stability and is preferably nontoxic after long-term therapies.

In addition, since the non-mammalian GnRH analog is more potent it can be used to bind to the tumor tissue GnRH receptor with high affinity so as to displace the endogenous GnRH-like activity and block its action. It can incorporate a substitution of Gly(10)-NH₂ with ethylamide to inhibit degradation by post-proline peptidases and has minimal effect on the mammalian GnRH receptofr.

Further, a method is envisioned for regulating production of endogenous non-mammalian GnRH and/or the non-mammalian GnRH receptor. This regulation can occur at the transcription, translation or secretion level. In addition, the regulation of production of the endogenous non-mammalian GnRH or its receptor can occur directly by the introduction of an oligonucleotide or polypeptide with SEQ ID NO: 6 within the cell or indirectly by the polypeptide of SEQ ID NO: 6 binding to a receptor located on or within the cell.

Also a method is envisioned for determining whether a biological sample contains non-mammalian GnRH or its receptors. This method involves contacting the sample with the antibody of to the non-mammalian GnRH or its receptor and determining whether the antibody specifically binds to the sample, said binding being an indication that the sample contains either the non-mammalian GnRH or its receptor, respectively. In addition, a method is envisioned for determining whether a biological sample contains the mRNA or DNA, or the respective complements thereof, that codes for the non-mammalian GnRH or its receptor. This method involves subjecting the sample to in situ localization or any binding or hybridization procedure to determine whether said sample contains said mRNA or DNA. These methods are utilized to monitor cell function and tumor growth.

Antibodies are also envisioned that bind specifically to non-mammalian GnRH or its receptor. These antibodies are necessary for use in regulating cell proliferation and in the treatment of tumors.

It is envisioned that these non-mammalian GnRH analogs will be administered intra-nasally, orally, subcutaneously, transdermally or intramuscularly to human or animals, or intrauterine or intravaginally to the female. However, virtually any mode of administration may be used in the practice of the invention. As a contraceptive the non-mammalian GnRH analog can be taken daily or during the fertile period. To regulate cell functions or anti-tumor actions, the non-mammalian GnRH analog can be taken daily or intermittantly as needed.

### BRIEF DESCRIPTION OF DRAWINGS

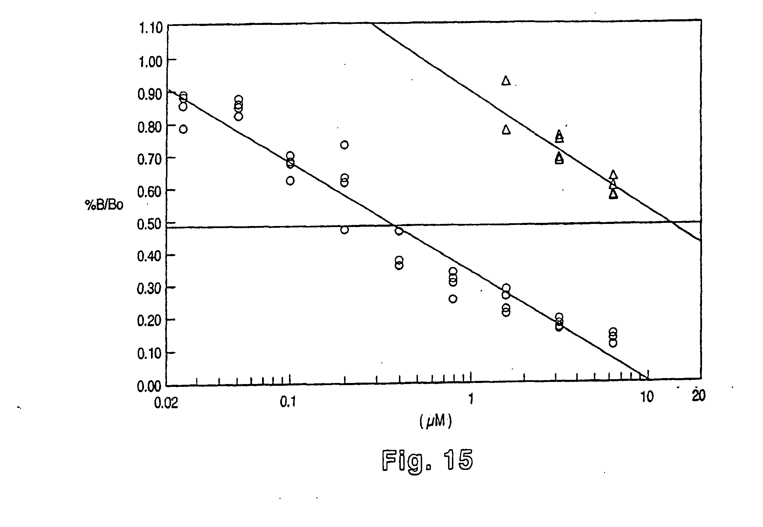

Figure 1. Placental Kds for Chicken II and Mammalian GnRH Analogs
Figure 2. Affinity of Receptor Binding of D-Arg(6) -Chicken II GnRH-aza-Gly(10)-amide for the Human Placental GnRH Receptor.
Figure 3. Effect of des-Gly¹⁰-GnRH-ethylamide on Degradation of GnRH by C-ase-1.
   □ GnRH 0.050 M, F GnRH 0.0250 M, Λ GnRH 0.012 M, ∀ GnRH 0.062 M
Figure 4a. Inhibition of the Degradation of Mammalian GnRH by Placental Enzyme Chorionic Peptidase-1 by Chicken II GnRH.
   ∀ GnRH 0.0250 µM, Λ GnRH 0.01250 ΦM, FGnRH 0.00625 ΦM, □ GnRH 0.00312 ΦM coincubated with varying concentrations of Chicken II GnRH (0.025 ΦM)
Figure 4b. Inhibition of the Degradation of Mammalian GnRH by Placental Enzyme Chorionic Peptidase-1 by D-Arg-Chicken II-ethylamide.
   ∀ GnRH 0.0250 µM, Λ GnRH 0.01250 ΦM, F GnRH 0.00625 ΦM, □ GnRH 0.00312 ΦM coincubated with varying concentrations of D-Arg(6) -Chicken II GnRH-aza-Gly(10)-amide (0.500 ΦM)
Figure 4c. Inhibition of the Degradation of Mammalian GnRH by D-Arg-Chicken II RGnRH-aza-Gly-NH₂
   ∀ GnRH 0.0250 µM, Λ GnRH 0.01250 ΦM, F GnRH 0.00625 ΦM, □ GnRH 0.00312 ΦM coincubated with varying concentrations of D-Arg(6) -Chicken II GnRH-aza-Gly(10)-amide (0.500 ΦM) with varying concentrations of D-Arg-Chicken II GnRH-aza-Gly-NH₂
Figure 5a and 5b. Release of hCG by Human Term Placental Explants Incubated with Varying Concentrations of D-Arg(6) -Chicken II GnRH-aza-Gly(10)-amide.
Figure 6. Dose-Related Effect of D-Arg(6) -Chicken II GnRH-aza-Gly(10)-amide on hCG Release.
Figure 7. Effect of Chicken II GnRH Analog on hCG Release.
Figure 8. Effect of Chicken II GnRH Analog on Placental Progesterone Release.
Figure 9a. Effect of Chicken II GnRH Analog on PGE2 Release Incubation 2 Hours.
Figure 9b. Effect of Chicken II GnRH Analog on PGE2 Release Incubation 24 Hours.
Figure 10. Effect of TRH on the Degradation of GnRH by C-ase-1.
   □GnRH 1.000M, F GnRH 0.500 M, Λ GnRH 0.250 M, ∀ GnRH 0.125 M
Figure 11. Effect of Reduced Oxytocin on the Degradation of GnRH by C-ase-1.
   □ GnRH 0.050 M, F GnRH 0.0250 M, Λ GnRH 0.012 M, ∀ GnRH 0.062 M
Figure 12A and 12B. Action of Angiotensin II on Degradation of GnRH.
   - 12A: □ Angio 0.12 M, F Angio 0.25 M, Λ Angio 0.50 M, ∀ Angio 1.000 M
   - 12B: □ GnRH 1.00 M, F GnRH 0.50 M, Λ GnRH 0.25 M, ∀ GnRH 0.12 M
Figure 13. Action of Chick II-ethylamide On Degradation of GnRH By C-ase-1.
   □ GnRH 0.00313 M,Λ GnRH 0.0125 M, ∀ GnRH 0.0250M
   GnRH was actively degraded by C-ase-1. This activity of C-ase-1 was inhibited by, ⁹OH-Pro-GnRH, Lamprey, Chicken I-GnRH, Antide, Chicken II-GnRH and Salmon GnRH with a relative potency of 1.5, 1.5, 0.6, 0.6, and 0.2 and 0.2, respectively to that for GnRH. Both Chicken II GnRH-¹⁰ ethylamide and ⁶Im-btl-D-His-GnRH¹⁰ ethylamide were essentially inactive, i.e., <0.001 inhibitory activity for GnRH.

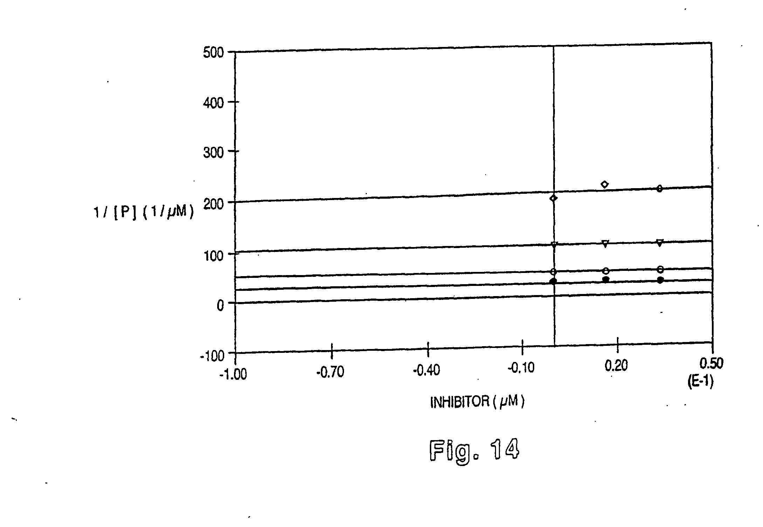

Figure 14. Effect of des-Gly¹⁰-Im-Btl-D-His⁶-GnRH-ethylamide on Degradation of GnRH by C-ase-1.
   □ GnRH 0.0500 M, F GnRH 0.0250 M, Λ GnRH 0.012 M, ∀ GnRH 0.062 M
Figure 15. Competitive Placental Receptor Binding For GnRH Analogs With Labeled Chicken II Analog.
   - Buserilin: Λ GnRH , FD-Arg-CII-EA

   GnRH was bound by the placental GnRH receptor with a K_{d} of 10⁻⁶ M. Chicken II GnRH was similar to GnRH. The K_{d} for ⁶Im-btl-D-His-GnRH⁻¹⁰ ethylamide was half the potency of GnRH, while Buserilin and ⁶D-Trp-GnRH⁻¹⁰ ethylamide were twice as active as GnRH. The greatest potency, having a K^{d} of 3 non-mammalian, i.e. 33-fold more activity than GnRH.

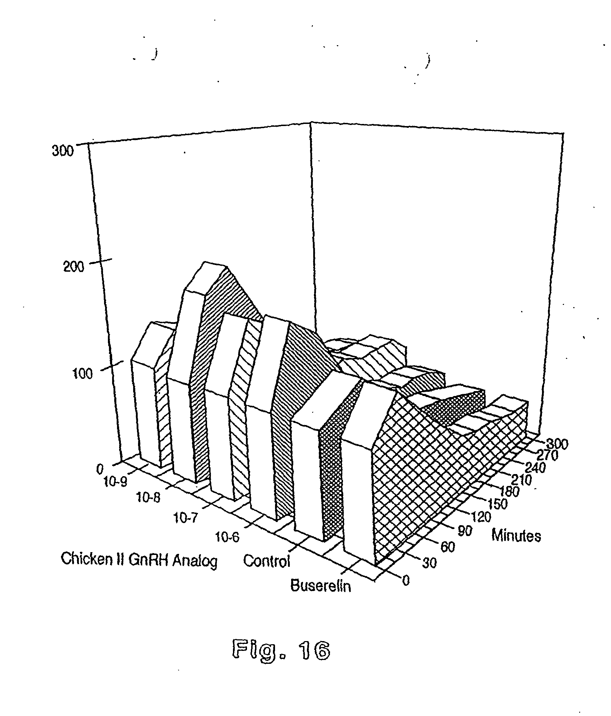

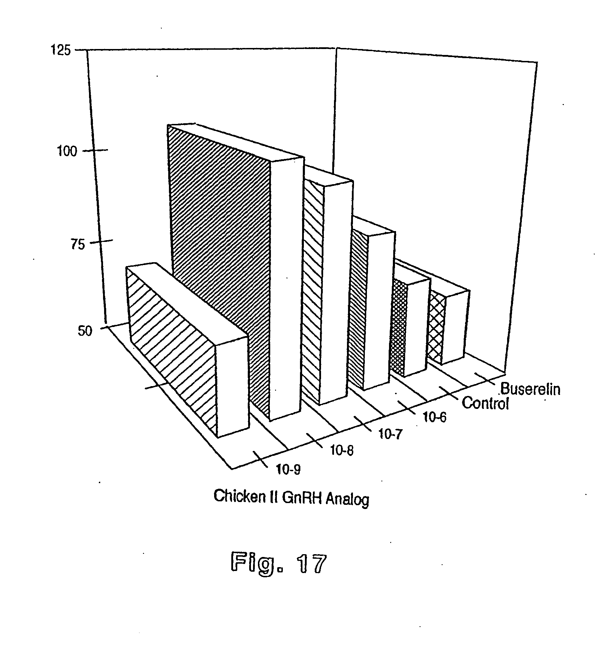

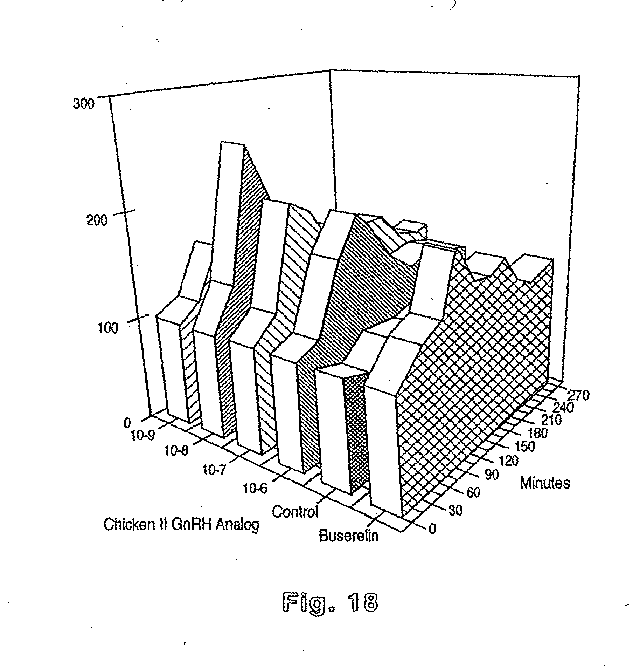

Figure 16. Effect of Chicken II GnRH Analog on hCG in Early Human Placenta.

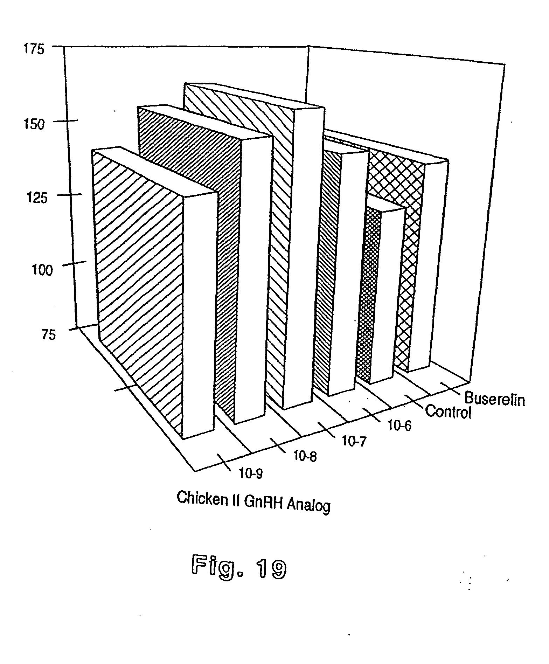

Figure 17. Effect of Chicken II GnRH Analog on hCG in Early Human Placenta - Average Response Over 300 Minutes.
Figure 18. Effect of Chicken II GnRH Analog on hCG on Early Human Placenta.
Figure 19. Effect of Chicken II GnRH Analog on hCG on Early Human Placenta - Average Response over 270 Minutes.

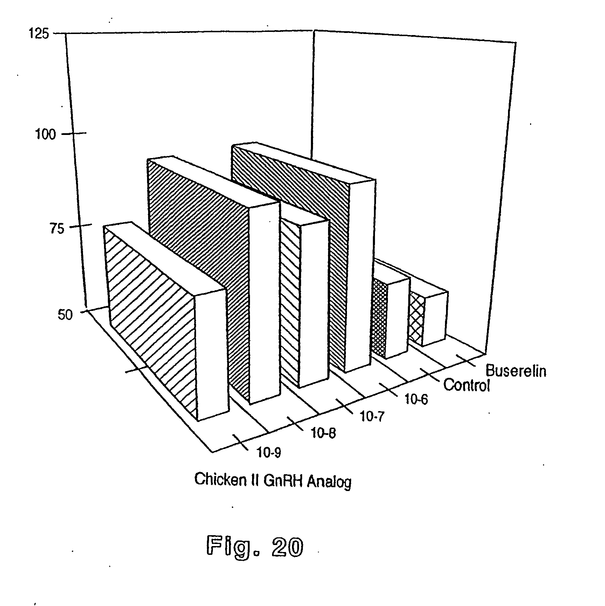

Figure 20. Effect of Chicken II GnRH Analog on HCG in Early Human Placenta

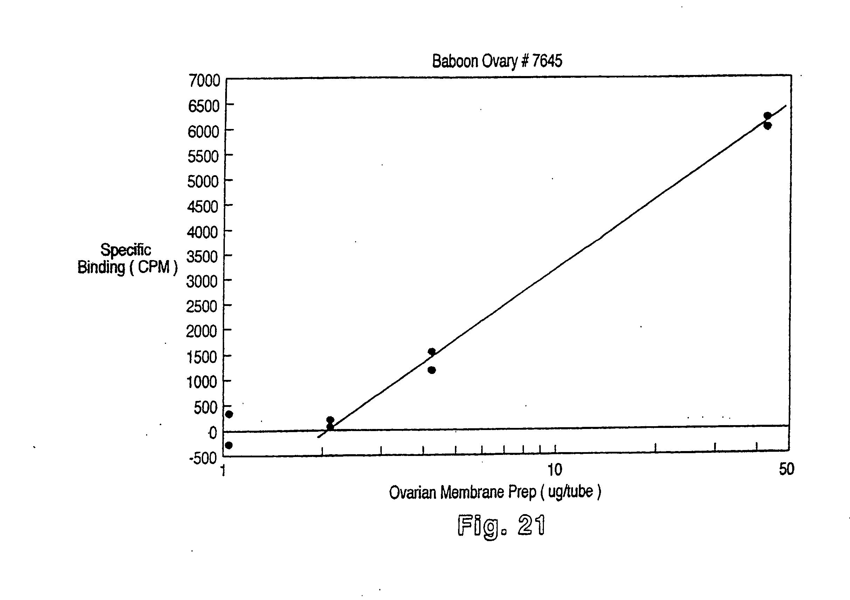

Figure 21. Binding of D-Arg-Chicken II GnRH-aza-Gly-amide by Baboon Ovary.

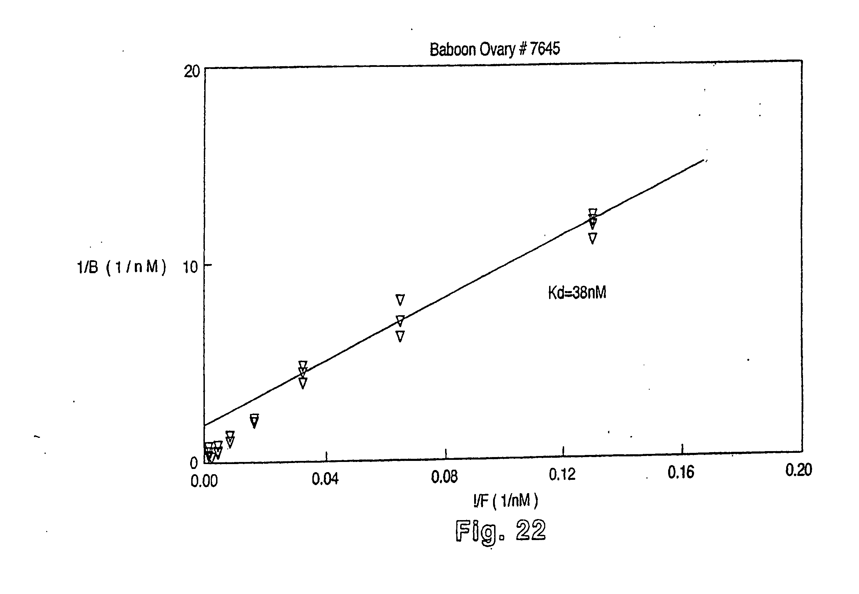

Figure 22. Affinity of Chicken II Analog of Ovarian Receptor.

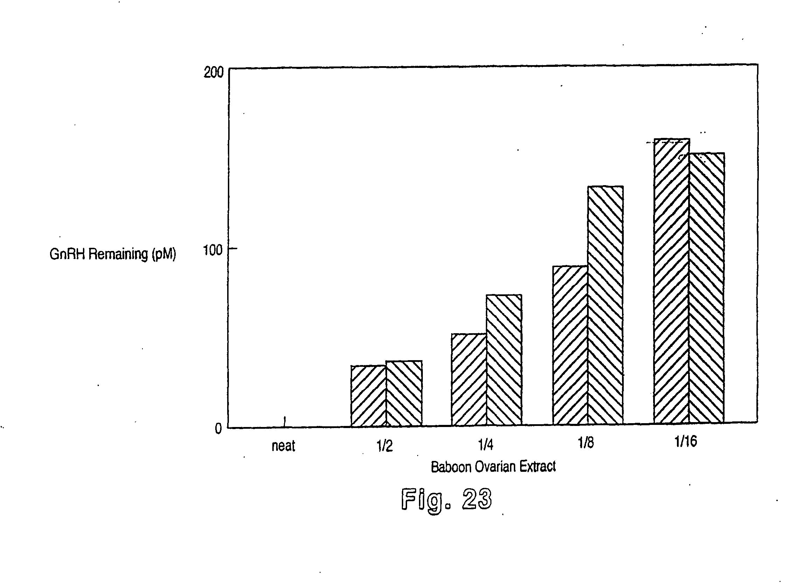

Figure. 23. Degradation of Mammalian GnRH in Baboon Ovary Extract.

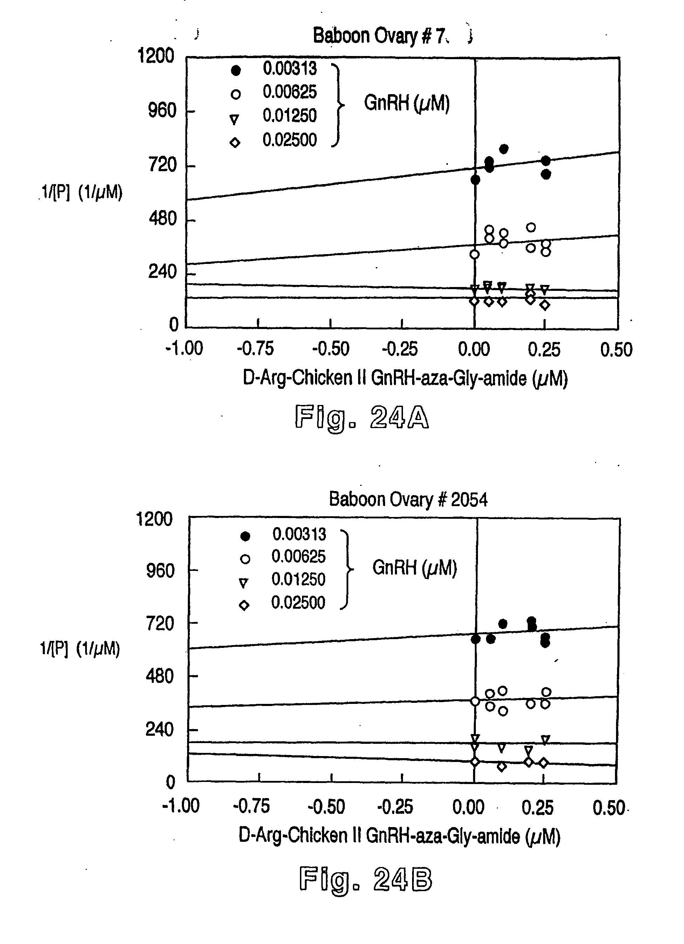

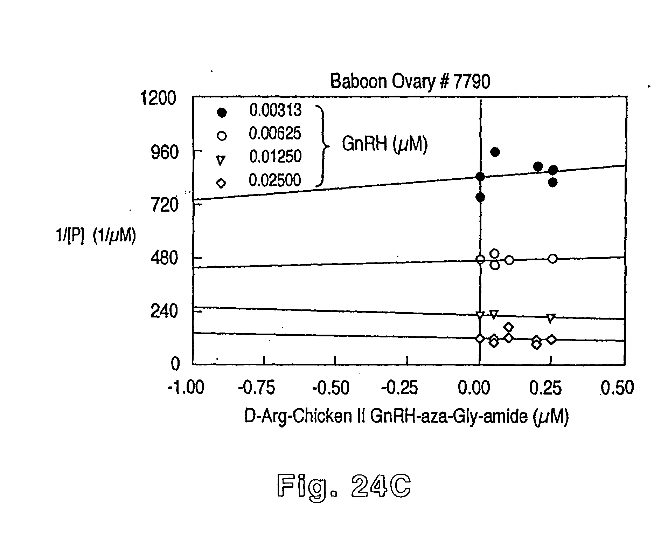

Figure 24. Inhibition of Degradation of Mammalian GnRH Analog in the Baboon Ovary.

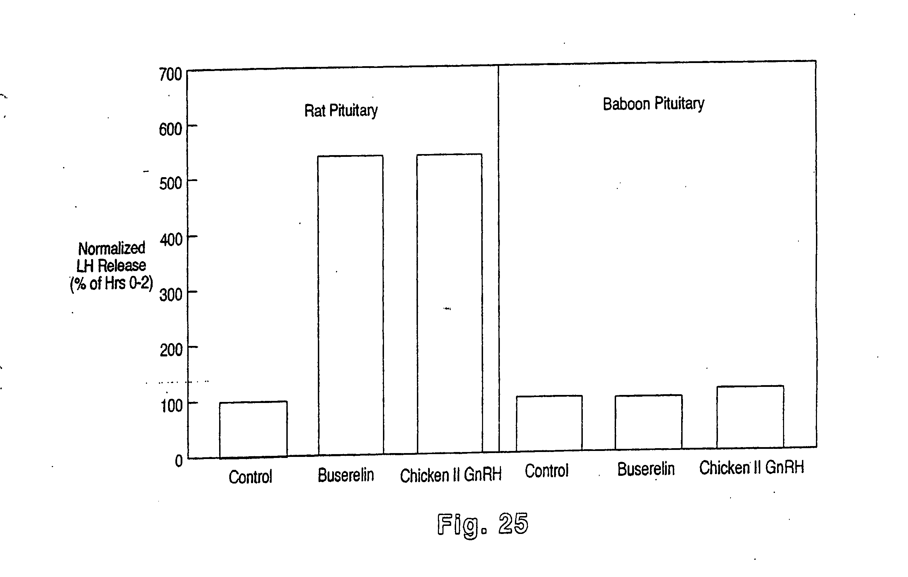

Figure 25. Effect of Mammalian and Chicken GnRH Analogs on Pituitary LH Release.

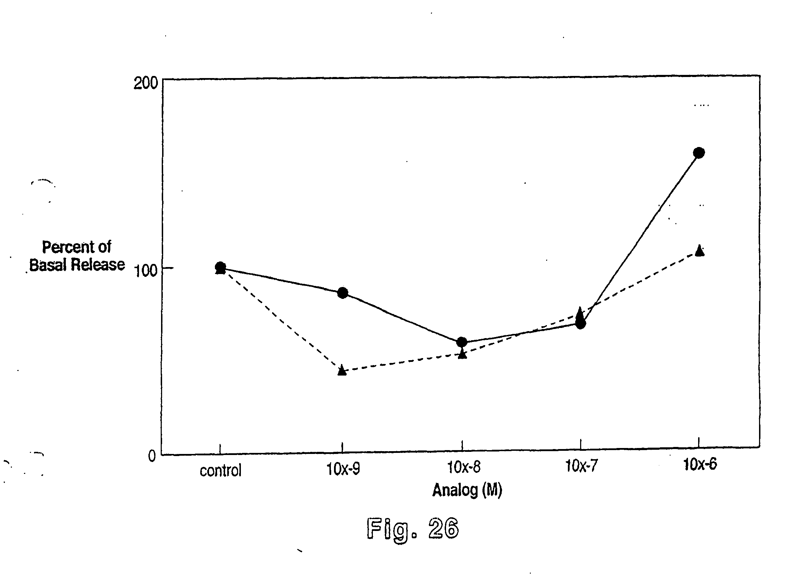

Figure 26. Effect of Chicken II GnRH Analog on Two Different Baboon Pituitaries.

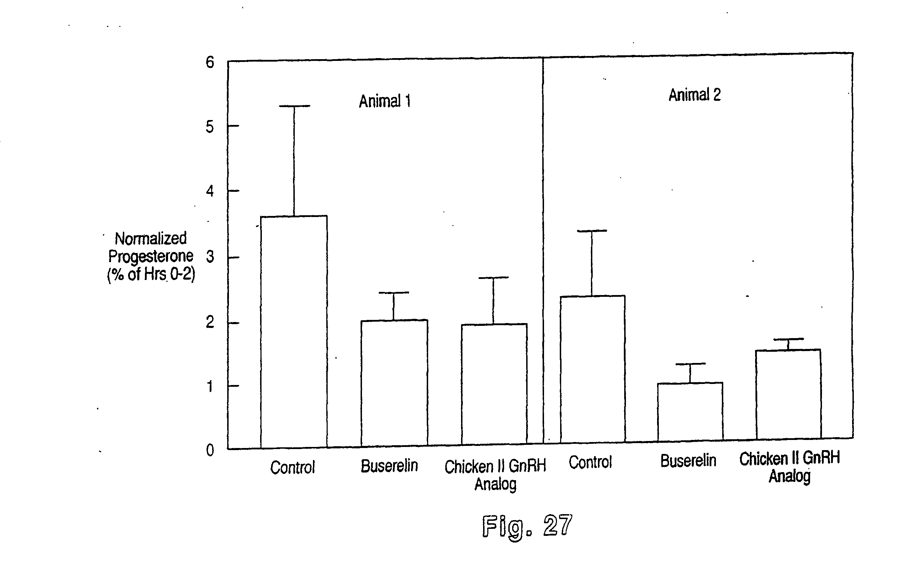

Figure 27. Effect of Mammalian and Chicken GnRH Analogs on Pregnant Rat Ovaries.

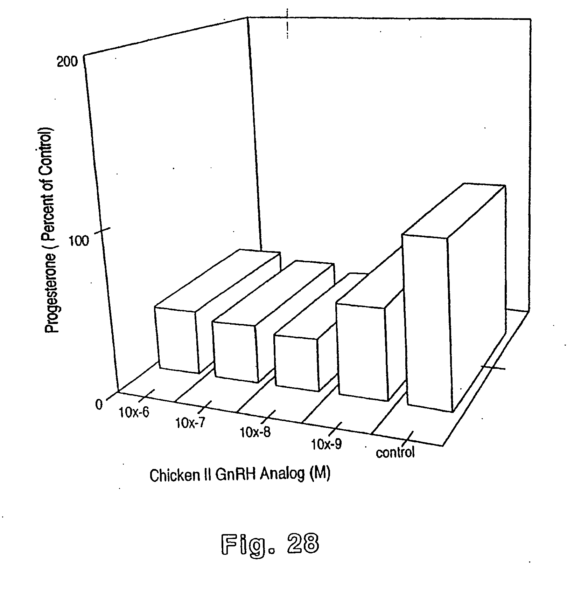

Figure 28. Effect of Chicken II GnRH Analog on Baboon Granulosa Cells.

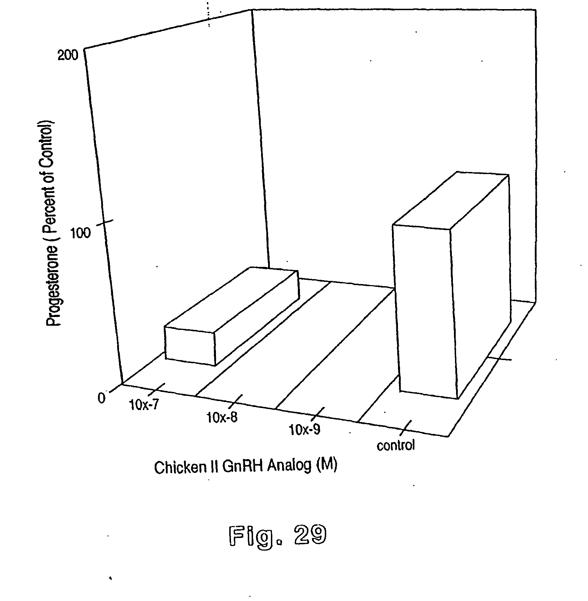

Figure 29. Effect of Chicken II GnRH Analog on Baboon Granulosa Cells.

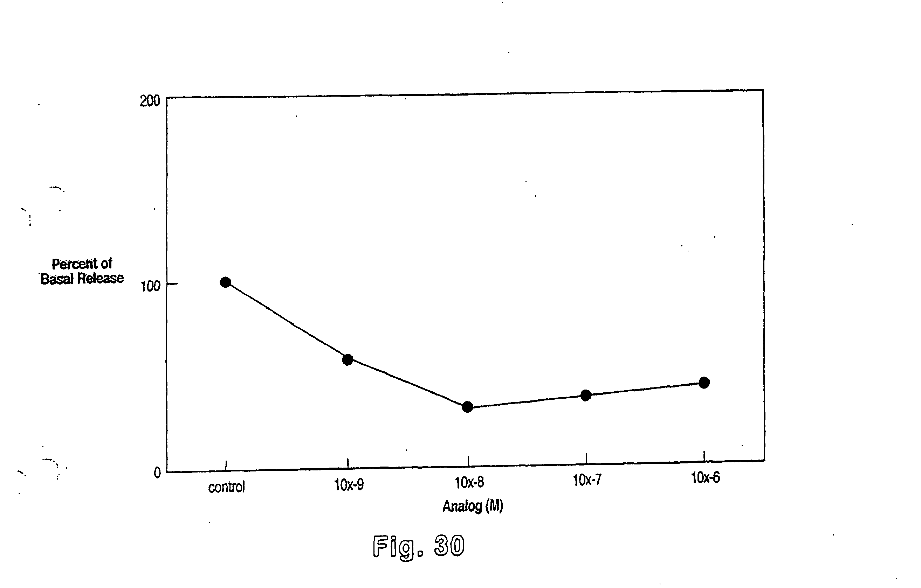

Figure 30. Effect of Chicken II GnRH Analog on Baboon Granulosa Cells.

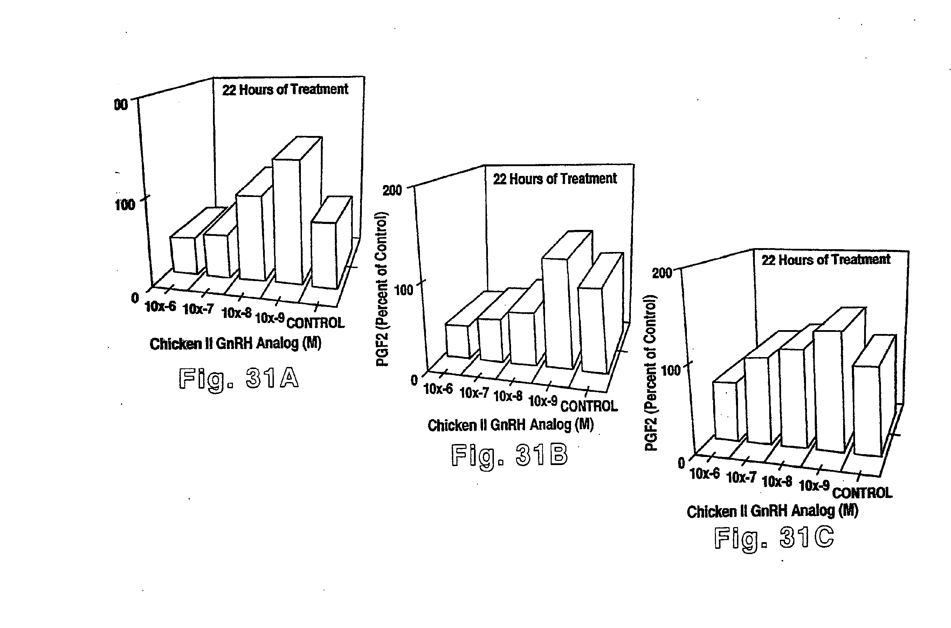

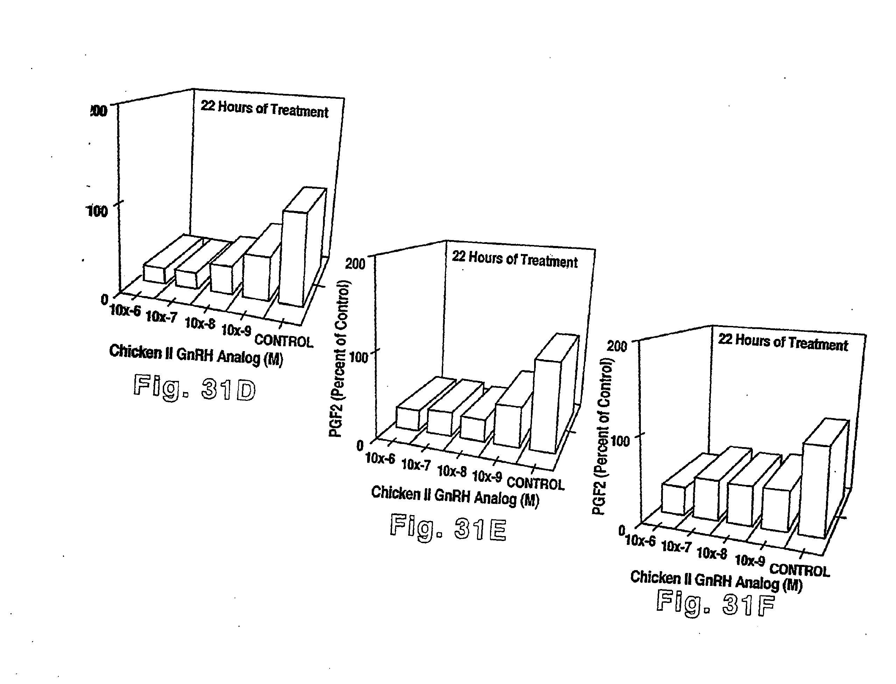

Figure 31. Effect of Chicken II GnRH Analog on PGE₂ in Human Endometrial Cells

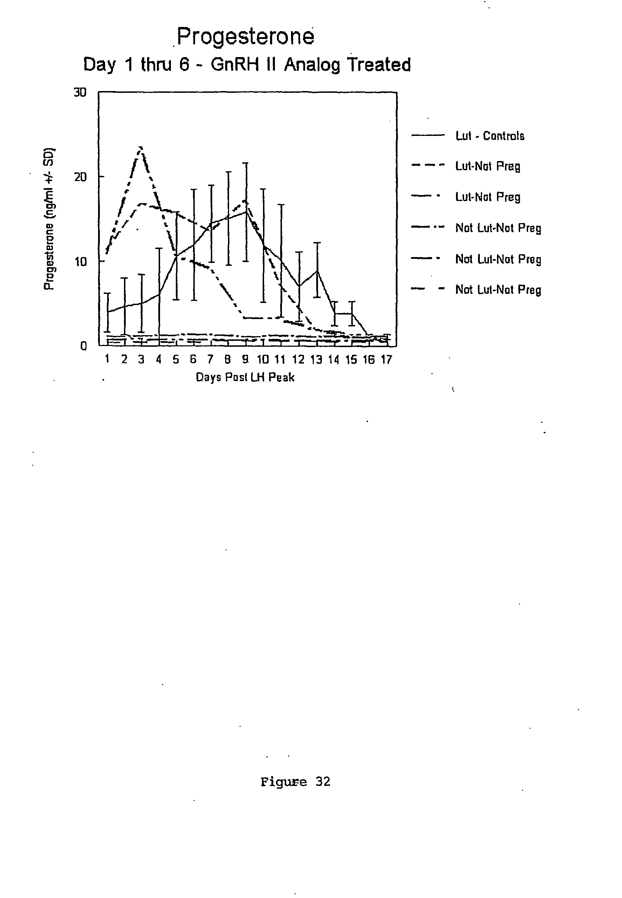

Figure 32. Maternal circulating progesterone for each of the five Day 1-6 GnRH II analog-treated animals is compared to the circulating progesterone for saline treated-controls (mean ±sd).

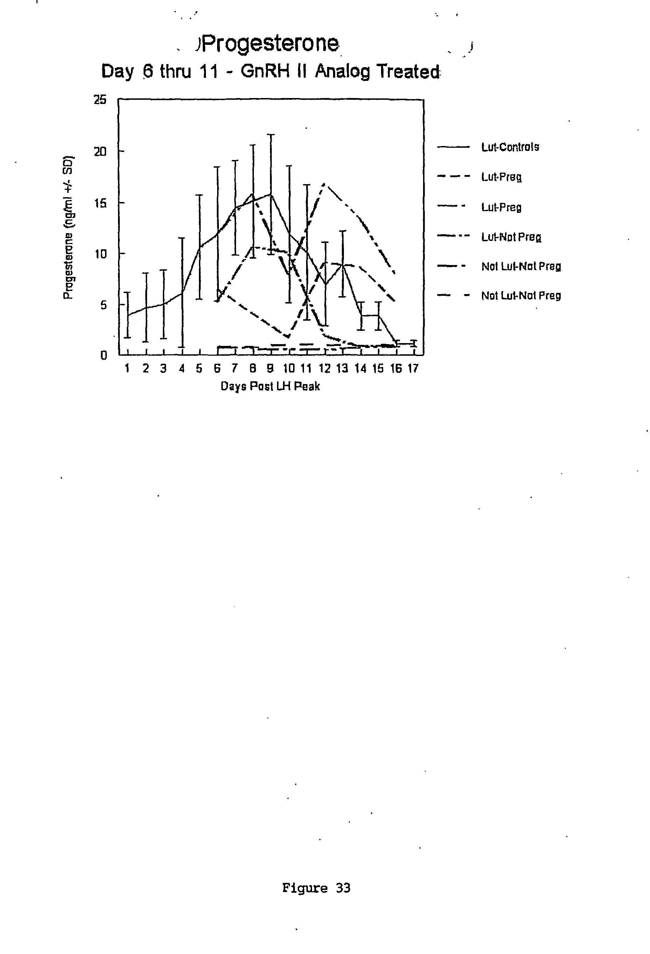

Figure 33. Maternal circulating progesterone for each of the five Day 6-11 GnRH II analog-treated animals is compared to the circulating progesterone for saline treated-controls (mean ±sd).

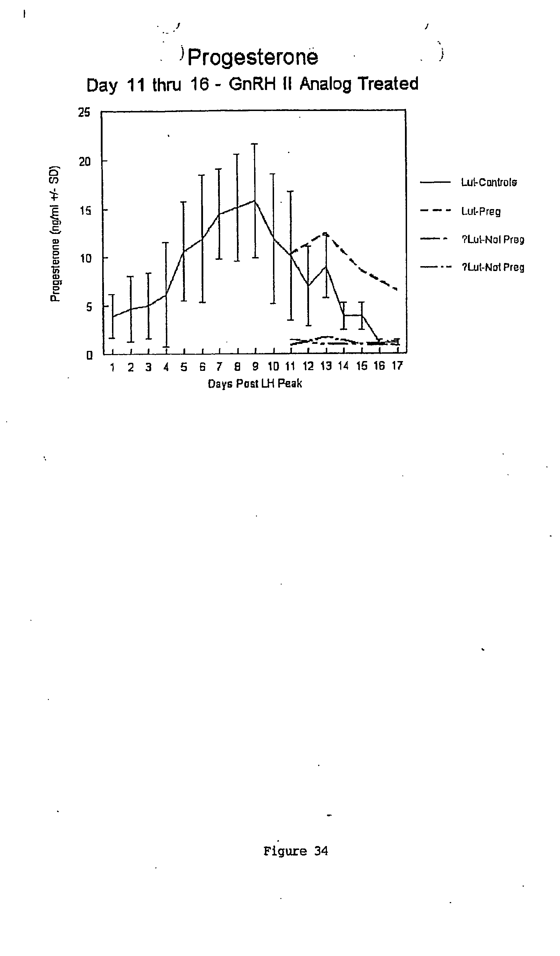

Figure 34. Maternal circulating progesterone for each of the five Day 11-17 GnRH II analog-treated animals is compared to the circulating progesterone for saline treated-controls (mean ±sd).

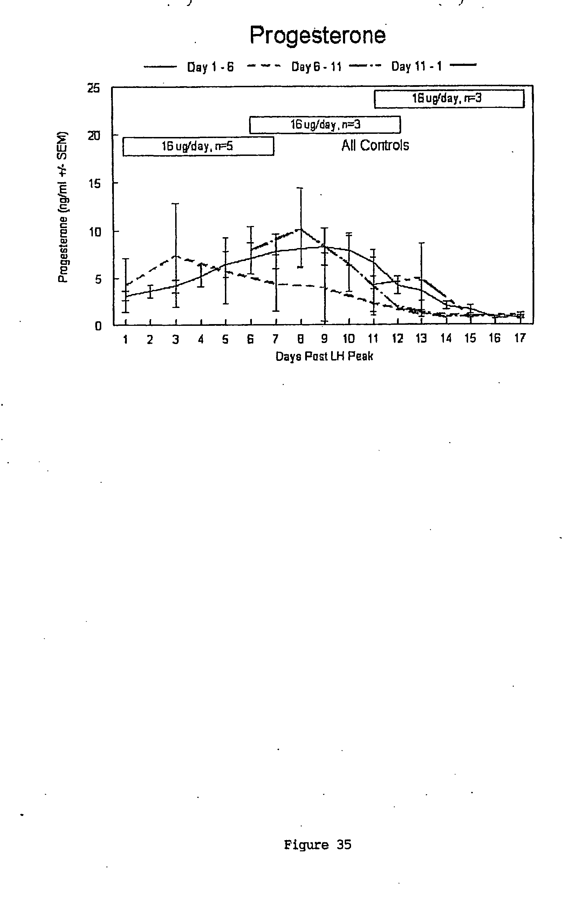

Figure 35. The maternal circulating progesterone animals (mean ±sem, n=12) for all saline-treated animals is compared to that for GnRH II analog-treated animals on Day 1-6, Day 6-11 and Day 11-17.

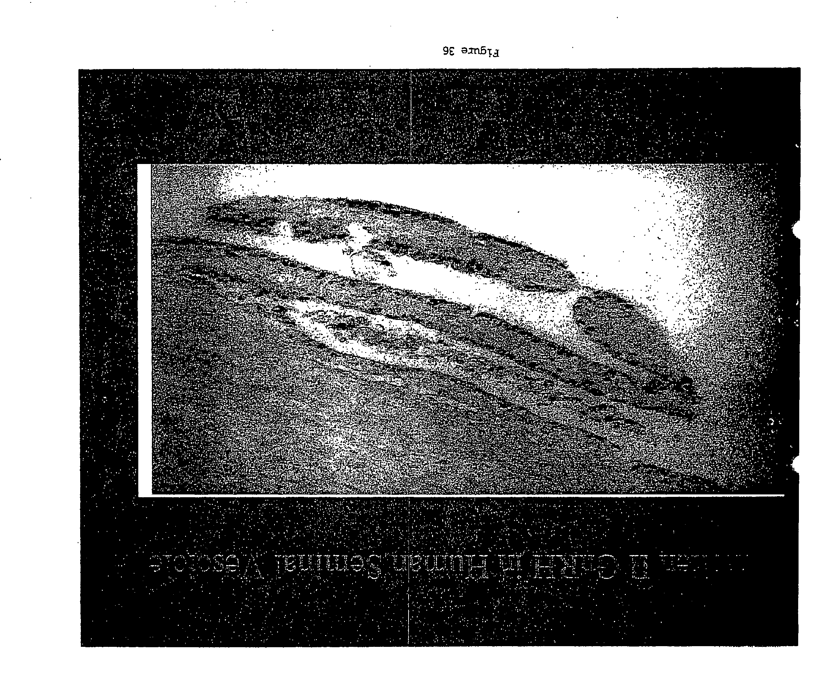

Figure 36. Chicken II GnRH in Human Seminal Vesicle.

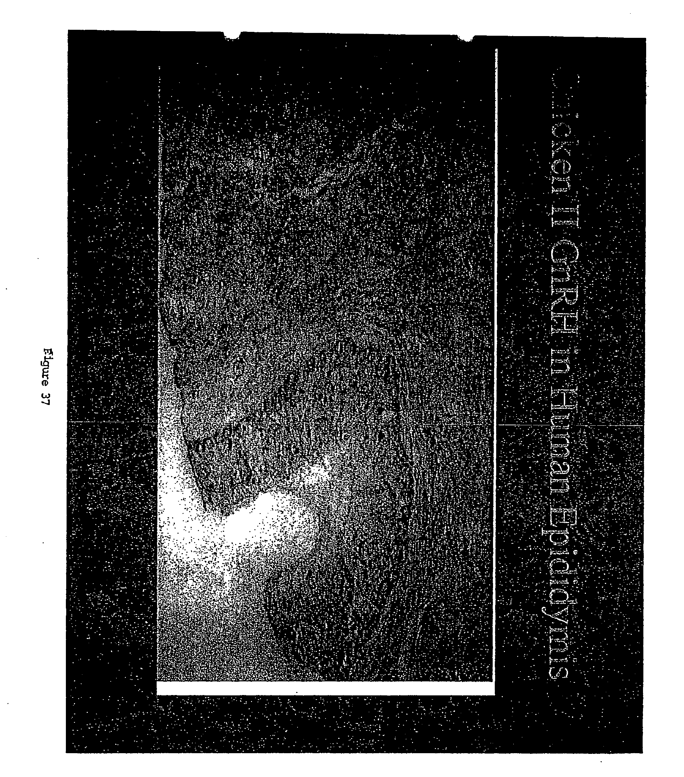

Figure 37. Chicken II GnRH in Human Epididymis.

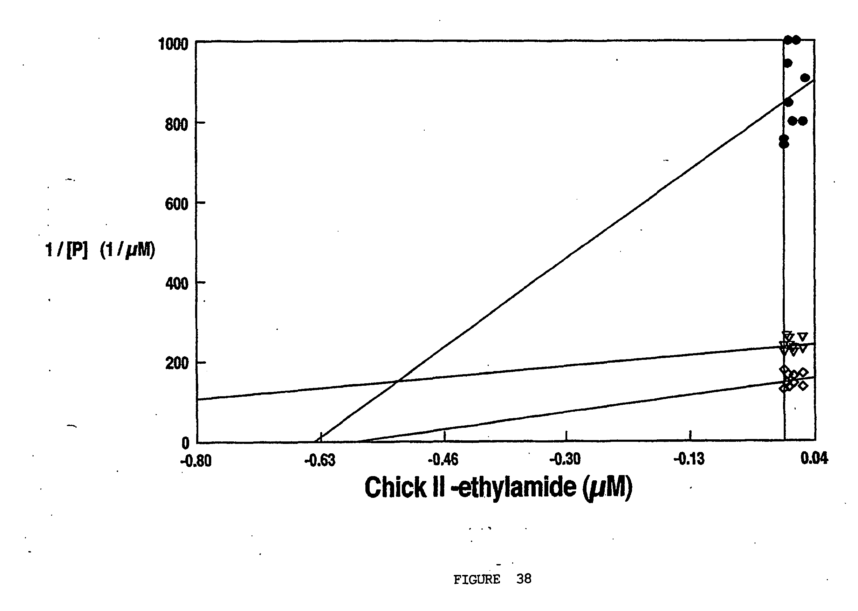

Figure 38. Effect of des-Gly(10)-mammalian GnRH-ethylamide on the degradation of mammalian GnRH by the chorionic post-proline peptidase.
   ∀ GnRH 0.00313 M, Λ GnRH 0.0625 M, □GnRH 0.0125 M.

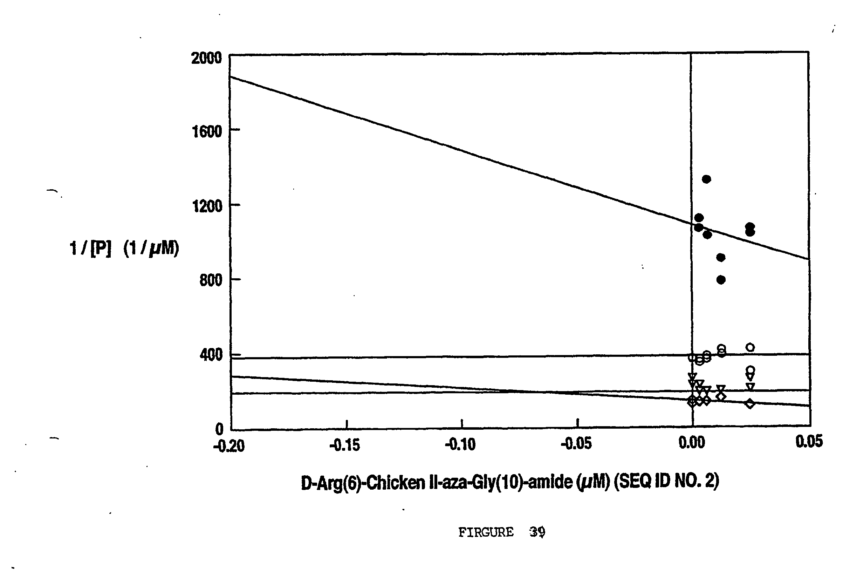

Figure 39. Action ofD-Arg- Chicken II-aza-Gly-NH₂ on the Degradation of Mammalian GnRH by Chorionic Post-Proline Peptidase. ∀ GnRH 0.00313 M, Λ GNRH 0.0625 M, □ GnRH 0.0125 M, 0 GnRH 0.0250 M.

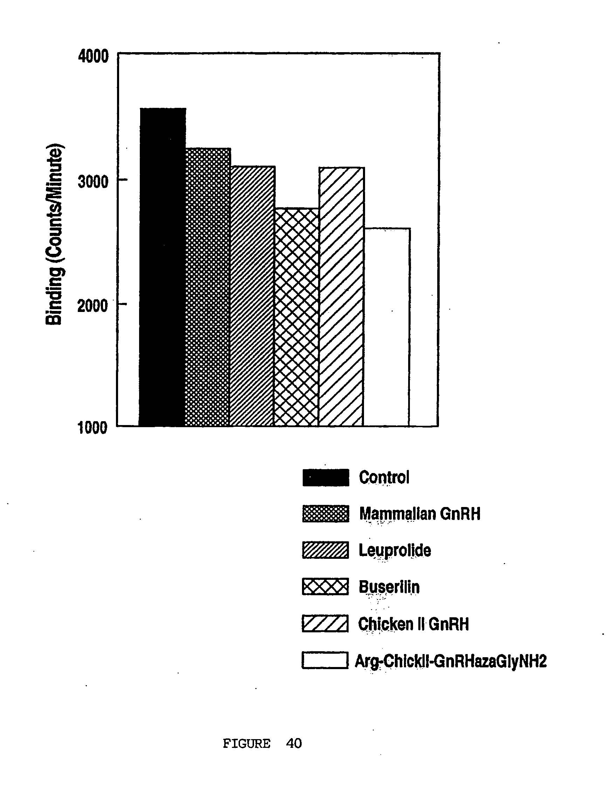

Figure 40. Binding of I¹²⁵-D-Arg(6)-Chicken II GnRH-aza-Gly(10)-amide to MCF 7 breast cancer cells after 24 hours of incubation with no exogenous GnRH or competing isoforms or analogs of GnRH

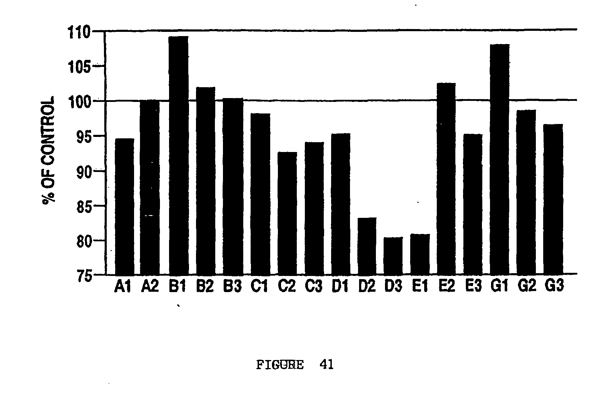

Figure 41. The anti proliferative, tumor regression activity of D-Arg(6)-Chicken II GnRH-aza-Gly(10)-amide compared to controls and other isoforms and analogs of GnRH after 24 hours.

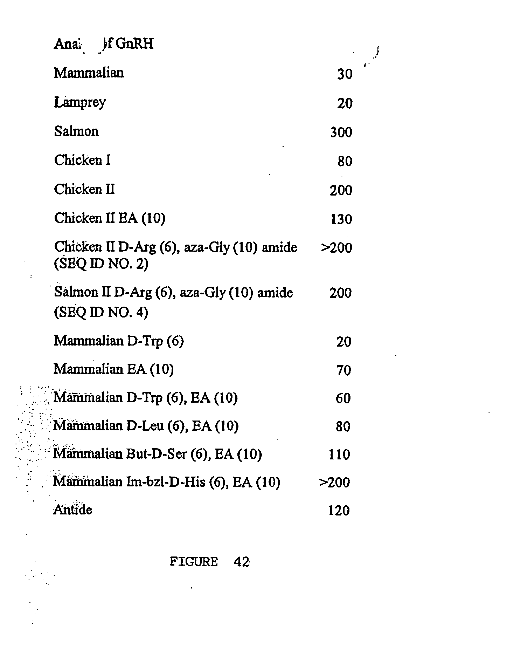

Figure 42. Inhibitor constants for analogs of GnRH.

### DETAILED DESCRIPTION OF THE PREFERRED EMBODIMENTS

Following long-standing patent law convention, the terms a and an mean one or more when used in this application, including the claims.

An isolated nucleic acid is a nucleic acid the structure of which is not identical to that of any naturally occuring nucleic acid or to that of any fragment of a naturally occuring genomic nucleic acid spanning more than three separate genes. The term therefore covers, for example, (a) a DNA which has the sequence of part of a naturally occuring genomic DNA molecule, but is not flanked by both of the coding sequences that flank that part of the molecule in the genome of the organism in which it naturally occurs; (b) a nucleic acid incorporated into a vector or into the genomic DNA of a prokaryote or eukaryote in a manner such that the resulting molecule is not identical to any naturally occuring vector or genomic DNA; (c) a separate molecule such as a cDNA, a genomic fragment, a fragment produced by polymerase chain reaction (PCR), or a restriction fragment; and (d) a recombinant nucleotide sequence that is part of a hybrid gene, i.e., a gene encoding a fusion protein. Specifically, excluded from this definition are nucleic acids present in mixtures of (i) DNA molecules, (ii) transfected cells, and (iii) cell clones, e.g., as these occur in a DNA library such as a cDNA or genomic DNA library.

The terms "complementary" or "complementarity" refer to the natural binding of polynucleotides by base pairing. For example, the sequence 5'-AGT-3' binds to the complementary sequence 3'-TCA-5'. Complementarity between two single-stranded molecules may be "partial" such that only some of the nucleic acids bind or it may be "complete" such that total complementarity exists between the single stranded molecules. The degree of complementarity between the nucleic acid strands has significant effects on the efficiency and strength of the hybridization between the nucleic acid strands.

The term "expression modulating fragment," EMF, means a series of nucleotides which modulates the expression of an operably linked ORF or another EMF. As used herein, a sequence is said to "modulate the expression of an operably linked sequence" when the expression of the sequence is altered by the presence of the EMF. EMFs include, but are not limited to, promoters, and promoter modulating sequences (inducible elements). One class of EMFs are nucleic acid fragments which induce the expression of an operably linked ORF in response to a specific regulatory factor or physiological event.

The terms "nucleotide sequence" or "nucleic acid" or "polynucleotide" or "oligonucleotide" are used interchangeably and refer to a heteropolymer of nucleotides or the sequence of these nucleotides. These phrases also refer to DNA or RNA of genomic or synthetic origin which may be single-stranded or double-stranded and may represent the sense or the antisense strand, to peptide nucleic acid (PNA) or to any DNA-like or RNA-like material. Generally, nucleic acid segments provided by this invention may be assembled from fragments of the genome and short oligonucleotide linkers, or from a series of oligonucleotides, or from individual nucleotides, to provide a synthetic nucleic acid which is capable of being expressed in a recombinant transcriptional unit comprising regulatory elements derived from a microbial or viral operon, or a eukaryotic gene. Probes may, for example, be used to determine whether specific mRNA molecules are present in a cell or tissue or to isolate similar nucleic acid sequences from chromosomal DNA as described by Walsh et al. (Walsh, P. S. et al., 1992, PCR Methods Appl 1:241-250). They may be labeled by nick translation, Klenow fill-in reaction, PCR, or other methods well known in the art. Probes of the present invention, their preparation and/or labeling are elaborated in Sambrook, J. et al., 1989, Molecular Cloning: A Laboratory Manual, Cold Spring Harbor Laboratory, NY; or Ausubel, F. M. et al., 1989, Current Protocols in Molecular Biology, John Wiley & Sons, New York N.Y., both of which are incorporated herein by reference in their entirety.

The term "open reading frame," ORF, means a series of nucleotide triplets coding for amino acids without any termination codons and is a sequence translatable into protein.

The terms "operably linked" or "operably associated" refer to functionally related nucleic acid sequences. For example, a promoter is operably associated or operably linked with a coding sequence if the promoter controls the transcription of the coding sequence. While operably linked nucleic acid sequences can be contiguous and in the same reading frame, certain genetic elements e.g. repressor genes are not contiguously linked to the coding sequence but still control transcription/translation of the coding sequence.

The term "translated protein coding portion" means a sequence which encodes for the full length protein which may include any leader sequence or any processing sequence.

The term "mature protein coding sequence" means a sequence which encodes a peptide or protein without a signal or leader sequence. The peptide may have been produced by processing in the cell which removes any leader/signal sequence. The peptide may be produced synthetically or the protein may have been produced using a polynucleotide only encoding for the mature protein coding sequence.

The term "derivative" refers to polypeptides chemically modified by such techniques as ubiquitination, labeling (e.g., with radionuclides or various enzymes), covalent polymer attachment such as pegylation (derivatization with polyethylene glycol) and insertion or substitution by chemical synthesis of amino acids such as ornithine, which do not normally occur in human proteins.

The term "variant"(or "analog") refers to any polypeptide differing from naturally occurring polypeptides by amino acid insertions, deletions, and substitutions, created using, e g., recombinant DNA techniques. Guidance in determining which amino acid residues may be replaced, added or deleted without abolishing activities of interest, may be found by comparing the sequence of the particular polypeptide with that of homologous peptides and minimizing the number of amino acid sequence changes made in regions of high homology (conserved regions) or by replacing amino acids with consensus sequence. Alternatively, recombinant variants encoding these same or similar polypeptides may be synthesized or selected by making use of the "redundancy" in the genetic code. Various codon substitutions, such as the silent changes which produce various restriction sites, may be introduced to optimize cloning into a plasmid or viral vector or expression in a particular prokaryotic or eukaryotic system. Mutations in the polynucleotide sequence may be reflected in the polypeptide or domains of other peptides added to the polypeptide to modify the properties of any part of the polypeptide, to change characteristics such as ligand-binding affinities, interchain affinities, or degradation/turnover rate.

Preferably, amino acid "substitutions" are the result of replacing one amino acid with another amino acid having similar structural and/or chemical properties, i.e., conservative amino acid replacements. "Conservative" amino acid substitutions may be made on the basis of similarity in polarity, charge, solubility, hydrophobicity, hydrophilicity, and/or the amphipathic nature of the residues involved. For example, nonpolar (hydrophobic) amino acids include alanine, leucine, isoleucine, valine, proline, phenylalanine, tryptophan, and methionine; polar neutral amino acids include glycine, serine, threonine, cysteine, tyrosine, asparagine, and glutamine; positively charged (basic) amino acids include arginine, lysine, and histidine; and negatively charged (acidic) amino acids include aspartic acid and glutamic acid. "Insertions" or "deletions" are preferably in the range of about 1 to 10 amino acids, more preferably 1 to 5 amino acids. The variation allowed may be experimentally determined by systematically making insertions, deletions, or substitutions of amino acids in a polypeptide molecule using recombinant DNA techniques and assaying the resulting recombinant variants for activity.

Alternatively, where alteration of function is desired, insertions, deletions or non-conservative alterations can be engineered to produce altered polypeptides. Such alterations can, for example, alter one or more of the biological functions or biochemical characteristics of the polypeptides of the invention. For example, such alterations may change the characteristics such as ligand-binding affinities, interchain affinities, or degradation/turnover rate. Further, such alterations can be selected so as to generate peptides that are better suited for expression, scale up and the like in the host cells chosen for expression. For example, cysteine residues can be deleted or substituted with another amino acid residue in order to eliminate disulfide bridges.

The terms "purified" or "substantially purified" as used herein denotes that the indicated nucleic acid or polypeptide is present in the substantial absence of other biological macromolecules, e.g., polynucleotides, proteins, and the like. In one embodiment, the polynucleotide or polypeptide is purified such that it constitutes at least 95% by weight, more preferably at least 99% by weight, of the indicated biological macromolecules present (but water, buffers, and other small molecules, especially molecules having a molecular weight of less than 1000 daltons, can be present).

The term "recombinant," when used herein to refer to a polypeptide or protein, means that a polypeptide or protein is derived from recombinant (e.g., microbial, insect, or mammalian) expression systems. "Microbial" refers to recombinant polypeptides or proteins made in bacterial or fungal (e.g., yeast) expression systems. As a product, "recombinant microbial" defines a polypeptide or protein essentially free of native endogenous substances and unaccompanied by associated native glycosylation. Polypeptides or proteins expressed in most bacterial cultures, e.g., E. coli, will be free of glycosylation modifications; polypeptides or proteins expressed in yeast will have a glycosylation pattern in general different from those expressed in mammalian cells.

The term "recombinant expression vehicle or vector" refers to a plasmid or phage or virus or vector, for expressing a polypeptide from a DNA (RNA) sequence. An expression vehicle can comprise a transcriptional unit comprising an assembly of (1) a genetic element or elements having a regulatory role in gene expression, for example, promoters or enhancers, (2) a structural or coding sequence which is transcribed into mRNA and translated into protein, and (3) appropriate transcription initiation and termination sequences. Structural units intended for use in yeast or eukaryotic expression systems preferably include a leader sequence enabling extracellular secretion of translated protein by a host cell. Alternatively, where recombinant protein is expressed without a leader or transport sequence, it may include an amino terminal methionine residue. This residue may or may not be subsequently cleaved from the expressed recombinant protein to provide a final product.

The term "recombinant expression system" means host cells which have stably integrated a recombinant transcriptional unit into chromosomal DNA or carry the recombinant transcriptional unit extrachromosomally. Recombinant expression systems as defined herein will express heterologous polypeptides or proteins upon induction of the regulatory elements linked to the DNA segment or synthetic gene to be expressed. This term also means host cells which have stably integrated a recombinant genetic element or elements having a regulatory role in gene expression, for example, promoters or enhancers. Recombinant expression systems as defined herein will express polypeptides or proteins endogenous to the cell upon induction of the regulatory elements linked to the endogenous DNA segment or gene to be expressed. The cells can be prokaryotic or eukaryotic.

The terms "secreted" or "secretion" include a protein that is transported across or through a membrane, including transport as a result of signal sequences in its amino acid sequence when it is expressed in a suitable host cell. "Secreted" proteins include without limitation proteins secreted wholly (e.g., soluble proteins) or partially (e.g., receptors) from the cell in which they are expressed. "Secreted" proteins also include without limitation proteins that are transported across the membrane of the endoplasmic reticulum. "Secreted" proteins are also intended to include proteins containing non-typical signal sequences (e.g. Interleukin-1 Beta, see Krasney, P. A. and Young, P. R. (1992) Cytokine 4(2):134-143) and factors released from damaged cells (e.g. Interleukin-1 Receptor Antagonist, see Arend, W. P. et. al. (1998) Annu. Rev. Immunol. 16:27-55)

Where desired, an expression vector may be designed to contain a "signal or leader sequence" which will direct the polypeptide through the membrane of a cell. Such a sequence may be naturally present on the polypeptides of the present invention or provided from heterologous protein sources by recombinant DNA techniques.

As used herein, "substantially equivalent" can refer both to nucleotide and amino acid sequences, for example a mutant sequence, that varies from a reference sequence by one or more substitutions, deletions, or additions, the net effect of which does not result in an adverse functional dissimilarity between the reference and subject sequences. Typically, such a substantially equivalent sequence varies from one of those listed herein by no more than about 35% (i.e., the number of individual residue substitutions, additions, and/or deletions in a substantially equivalent sequence, as compared to the corresponding reference sequence, divided by the total number of residues in the substantially equivalent sequence is about 0.35 or less). Such a sequence is said to have 65% sequence identity to the listed sequence. In one embodiment, a substantially equivalent, e.g., mutant, sequence of the invention varies from a listed sequence by no more than 30% (70% sequence identity); in a variation of this embodiment, by no more than 25% (75% sequence identity); and in a further variation of this embodiment, by no more than 20% (80% sequence identity) and in a further variation of this embodiment, by no more than 10% (90% sequence identity) and in a further variation of this embodiment, by no more that 5% (95% sequence identity). Substantially equivalent, e.g., mutant, amino acid sequences according to the invention preferably have at least.80% sequence identity with a listed amino acid sequence, more preferably at least 90% sequence identity. Substantially equivalent nucleotide sequences of the invention can have lower percent sequence identities, taking into account, for example, the redundancy or degeneracy of the genetic code. Preferably, nucleotide sequence has at least about 65% identity, more preferably at least about 75% identity, and most preferably at least about 95% identity. For the purposes of the present invention, sequences having substantially equivalent biological activity and substantially equivalent expression characteristics are considered substantially equivalent. For the purposes of determining equivalence, truncation of the mature sequence (e.g., via a mutation which creates a spurious stop codon) should be disregarded. Sequence identity may be determined, e.g., using the Jotun Hein method (Hein, J. (1990) Methods Enzymol. 183:626-645). Identity between sequences can also be determined by other methods known in the art, e.g. by varying hybridization conditions.

The term Aantibody@ includes whole antibodies and fragments thereof, single chain (recombinant) antibodies, Ahumanized@ chimeric antibodies, and immunologically active fragments of antibodies (eg. Fab fragments).

The term"degenerate variant" means nucleotide fragments which differ from a nucleic acid fragment of the present invention (e.g., an ORF) by nucleotide sequence but, due to the degeneracy of the genetic code, encode an identical polypeptide sequence. Preferred nucleic acid fragments of the present invention are the ORFs that encode proteins. The amino acids and their corresponding DNA codons can include, but are not limited to, isoleucine (ATT, ATC, ATA), leucine (CTT, CTC, CTA, CTG, TTA, TTG), valine (GTT, GTC, GTA, GTG), phenylalanine (TTT, TTC), methionine (ATG), cysteine (TGT, TGC), alanine (GCT, GCC, GCA, GCG), glycine (GGT, GGC, GGA, GGG), proline (CCT, CCC, CCA, CCG), threonine (ACT, ACC, ACA, ACG), serine (TCT, TCC, TCA, TCG, AGT, AGC), tyrosine (TAT, TAC), tryptophan (TGG), glutamine (CAA, CAG), asparagine (AAT, AAC), histidine (CAT, CAC), glutamic acid (GAA, GAG), aspartic acid (GAT, GAC), lysine (AAA, AAG), and arginine (CGT, CGC, CGA, CGG, AGA, AGG).

"Male fertility" depends on the proper function of a complex system of organs and hormones. The process begins in the area of the brain called the "hypothalamus-pituitary axis" which is a system of glands, hormones, and chemical messengers called "neurotransmitters" critical for reproduction. The first step in fertility is the production of GnRH in the hypothalamus, which prompts the pituitary gland to manufacture follicle-stimulating hormone (FSH) and luteinizing hormone (LH). FSH maintains sperm production while LH stimulates the production of the male hormone testosterone. Both sperm and testosterone production occur in the two testicles, or "testes", which are contained in the scrotal sac or "scrotum". The sperm are manufactured in several hundred microscopic "seminiferous" tubules which make-up most of the testicles. Surrounding these tubules are "Leydig cells" which manufacture testosterone.

The development of sperm begins in "Sertoli cells" located in the lower parts of the seminiferous tubules. As they mature, they are stored in the upper part of the tubules. Young sperm cells are known as "spermatids". When the sperm complete the development of their head and tail, they are released from the cell into the "epididymis". This C-shaped tube is 1/300 of an inch in diameter and about 20 feet long. It loops back and forth on itself within a space of only about one and a half inches long. The sperm's journey through the epididymis takes about three weeks. The fluid in which the sperm is transported contains fructose sugar, which provides energy as the sperm matures. In the early stages of its passage, the sperm cannot swim in a forward direction and can only vibrate its tail weakly. By the time the sperm reaches the end of the epididymis, however, it is mature. At maturity, each healthy sperm consists of a head that contains the male DNA and a tail that propels the head forward at about four times its own length every second.

When a man experiences sexual excitement, nerves stimulate the muscles in the epididymis to contract, which forces the sperm out through the penis. The sperm first pass from the epididymis into one of two muscular channels, called the "vasa deferentia". (A single channel is called a vas deferens.) Muscle contractions in the vas deferens from sexual activity propel the sperm past the "seminal vesicles" which contribute "seminal fluid" to the sperm. The vas deferens also collects fluid from the nearby "prostate gland". This mixture of various fluids and sperm is the "semen". Semen provides several benefits to the sperm. It provides a very short-lived alkaline environment to protect sperm from the harsh acidity of the female vagina. In addition, it contains a gelatin-like substance that prevents it from draining from the vagina too quickly. Also, it contains fructose to provide instant energy for sperm locomotion.

Each vas deferens then joins together to form the "ejaculatory duct". This duct, which now contains the sperm-containing semen, passes down through the urethra. The "urethra" is the same channel in the penis through which a man urinates, but during orgasm, the prostate closes off the bladder so urine cannot enter the urethra. The semen is forced through the urethra during ejaculation and out of the penis.

Usually about 100 to 300 million sperm are delivered into the ejaculate at any given time, but, even under normal conditions, only about 15% are healthy enough to fertilize an egg. After ejaculation only about 400 sperm survive the orgasm to complete the journey to the egg. Forty or so sperm survive the toxicity of the semen and the hostile environment of the vagina to reach the vicinity of the egg. Sperm that manage to reach the mucous lining in the woman's cervix (the lower part of her uterus) must survive about four more days to reach the woman's fallopian tubes. Here, the egg is positioned for fertilization for only one to two days each month. Normally, the cervical mucus forms an impenetrable barrier to sperm. However, when a woman ovulates or releases her egg, the oocyte, the mucous lining thins to allow sperm penetration. After the few remaining sperm finally penetrate the cervical mucus they become capacitated. "Capacitation" is a one time burst of energy that signals a cascade of events, including speeding up the motion of the sperm and triggering the actions of the "acrosome", a membrane filled with enzymes, which covers the head of the sperm. Dissolving the acrosome is a critical result of the capacitation process. Enzymes in the acrosome are then released that allow the sperm to drill a hole through the tough outer coating of the egg (the corona cells and zona pellucida ). Only one sperm can get through to fertilize the egg.

Disorders of the male reproductive system include (a) priapism - a nonsexual, prolonged, painful erection, (b) balanoposthitis (balanitis)- inflammation of the glans penis, (c) cryptorchidism - undescended testes, one or both, (d) epididymitis- inflammation of the epididymus, (e) cancer, (f) prostatitis - acute or chronic inflammation of the prostate gland, (g) benign prostatic hyperplasia, (h) testicular descent, (i) testicular dysfunction, (j) prostate dysfunction, and (k) preservation of testes during chemotherapy.

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

### EXAMPLE I - Design of Non-Mammalian GnRH Analogs

The present example outlines how analogs of non-mammalian GnRH with increased activity in chorionic, ovarian, tubal and uterine, sperm, testicular, scrotal, seminiferous tubule, Leydig cell, Sertoli cell, epididymis, vas deferentia, prostate, seminal vesicle, ejaculatory duct, and urethral tissues are designed.

Existing mammalian GnRH analogs are designed for activity at the pituitary GnRH receptor and with extended stability in the circulation of non-pregnant individuals. Yet, the existing data indicate that the ovarian, uterine, and chorionic, sperm, testicular, scrotal, seminiferous tubule, Leydig cell, Sertoli cell, epididymis, vas deferentia, prostate, seminal vesicle, ejaculatory duct, and urethral tissues have a high affinity GnRH receptor which differs from that in the pituitary. In addition, the degradation of GnRH is different in the ovary, uterus, and placenta during pregnancy. Therefore, prior known pituitary mammalian GnRH analogs have not been designed for use at extra-pituitary sites or during pregnancy, and potent non-mammalian GnRH analogs have not previously been designed for use at extra-pituitary sites or during pregnancy. The present invention provides potent non-mammalian GnRH analogs for use at extra pituitary sites.

Non-mammalian analogs of GnRH were synthesized by order. They were specifically designed to prevent degradation of the non-mammalian GnRH analog in extra-pituitary tissues, in the maternal circulation as well as within the intrauterine tissues. This allows for the maintenance of sufficient concentrations of non-mammalian GnRH analog to remain active when administered via the individual and to reach the extra-pituitary and intrauterine tissues of pregnancy. Due to the particular specificity of the ovarian, tubal, uterine, sperm, testicular, scrotal, seminiferous tubule, Leydig cell, Sertoli cell, epididymis, vas deferentia, prostate, seminal vesicle, ejaculatory duct, and urethral and placental receptor and specific peptidase in maternal blood and ovarian, tubal, uterine, and, sperm, testicular, scrotal, seminiferous tubule, Leydig cell, Sertoli cell, epididymis, vas deferentia, prostate, seminal vesicle, ejaculatory duct, and urethral tissues, and placental tissue, the particular non-mammalian GnRH analogs of the invention were designed. Analogs of the salmon (SEQ ID NO: 4) and chicken II GnRH (SEQ ID NO: 2) sequences, that both show greater affinity for the ovarian, tubal, uterine, and placental receptor than for the pituitary receptor, were modified to the tenth amino acid to ethylamide or aza-Gly¹⁰-NH₂ analog to make them resistant to degradation in the circulation and by post-proline peptidases. The chicken II GnRH sequence (SEQ ID NO: 2) and the salmon GnRH sequence (SEQ ID NO: 4) were also modified at the 6 position using D-Arg, making them resistant to degradation by the endopeptidase in blood, and were modified at the 10 position making them stable in maternal blood and the ovarian, tubal, uterine, and, sperm, testicular, scrotal, seminiferous tubule, Leydig cell, Sertoli cell, epididymis, vas deferentia, prostate, seminal vesicle, ejaculatory duct, and urethral tissues, and chorionic tissues. These analogs are expected to have increased binding to the ovarian, tubal, uterine, sperm, testicular, scrotal, seminiferous tubule, Leydig cell, Sertoli cell, epididymis, vas deferentia, prostate, seminal vesicle, ejaculatory duct, and urethral and placental receptors and increased metabolic stability.

### EXAMPLE II - Placental Receptor Binding Activity Placental Receptor Studies

The placental receptor binding activity of the different non-mammalian GnRH analogs of the present invention were compared. There is a human placental GnRH receptor which is distinct from that at the pituitary. Prior mammalian GnRH analogs have been designed to increase activity at the pituitary GnRH receptor and stability in the circulation of non-pregnant individuals. These mammalian GnRH analogs do not demonstrate potent binding activity at the placental receptor as they do at the pituitary receptor. The non-mammalian GnRH analogs of the present invention have been designed to interact with preference at the placental receptor and not the pituitary receptor. They have also been designed to limit degradation by the ovarian, tubal, uterine, and chorionic enzymes, present in maternal circulation as well as the ovary, fallopian tube, uterus, and placenta. Placental binding activity of the newly synthesized non-mammalian GnRH analogs have been compared to that for existing pituitary-active analogs of mammalian GnRH (SEQ ID NO: 5).

The newly synthesized non-mammalian GnRH analogs and other commercially available mammalian GnRH analogs were used in placental receptors binding and enzyme stability study described here. On the basis of these studies, the most receptor potent and enzyme-stable analogs were chosen for further biopotency studies. GnRH receptors were purified from the membrane fractions from placentas. The purification procedure for the placental GnRH receptor was performed using a modification of the method described by Bramley et al., which reference is specifically incorporated herein by reference for the purpose. Addition of enzyme inhibitors for the endogenous C-ase-1 were used as well as agents for receptor stabilization. Initially, receptor-binding assays using ¹²⁵I-Buserelin as label were performed. The competitive binding of each of the analogs was studied over a dose range of 10⁻¹¹ to 10⁻⁶ M. Incubation was at room temperature and receptor bound label was precipitated with polyethylene glycol. Specific and non-specific binding was determined. The data was subjected to Scatchard analysis. The non-mammalian GnRH analogs= ability to bind to the placental GnRH receptor was compared to that for synthetic mammalian GnRH (SEQ ID NO: 5), Buserelin (SEQ ID NO: 10) and other mammalian analogs. The more potent analogs were then studied in homologous receptor assays using newly synthesized non-mammalian GnRH analog as the radioiodinated label. This way, the receptor affinity for that analog could be precisely determined. Receptors from three different term placentas were used to study each of these analogs. The most potent analogs were used for the C-ase-1 stability studies. These data enabled the inventor to predict the most potent non-mammalian GnRH analog structure for the placental GnRH receptor, and assisted in the design of even more potent analogs for the chorionic GnRH receptor. *Bramley TA, McPhie CA, Menzies GS 1994 Human placental gonadotropin-releasing hormone (GnRH) binding sites: 111. Changes in GnRH binding levels with stage of gestation. Placenta 15:733-745.*

In these and additional studies, placental GnRH receptors were purified from human term placentas after homogenization in 40 mM Tris (pH 7.4) and filtered through cheesecloth, followed by an initial centrifugation at 1,000 x g for 10 minutes. The resulting supernatant was, again, centrifuged at 35,000 x g for 30 minutes and the membrane pellet was collected and resuspended in Tris buffer with 0.3 M sucrose. The protein concentration was determined. Membranes were stored frozen at -20 C until use. Before use, placental membranes were diluted to 5,000 µg/mL with Tris buffer containing 0.5% BSA and 50 U/mL bacitracin. Placental membranes (100 µL) were used with varying concentrations of mammalian GnRH (SEQ ID NO: 5), Buserelin (SEQ ID NO: 10), chicken II GnRH (SEQ ID NO: 6), D-Arg (6)-chicken II GnRH-des-Gly (10)-ethylamide (SEQ ID NO: 2), or D-Arg (6)-chicken II GnRH-aza-Gly (10)-amide (SEQ ID NO: 2) (100 µL) and either radiolabeled Buserelin (SEQ ID NO: 10) or radiolabeled D-Arg (6)-chicken II GnRH-aza-Gly (10)-amide (SEQ ID NO: 2) (100 µL/tube and iodinated). Following incubation at room temperature for 4 hours, the bound and free hormones were separated using polyethylene glycol precipitation, followed by centrifugation. The binding affinity for each GnRH isoform or analog was calculated using the double reciprocal plot of bound versus free ligand. Each study was done using three different human term placental tissues.

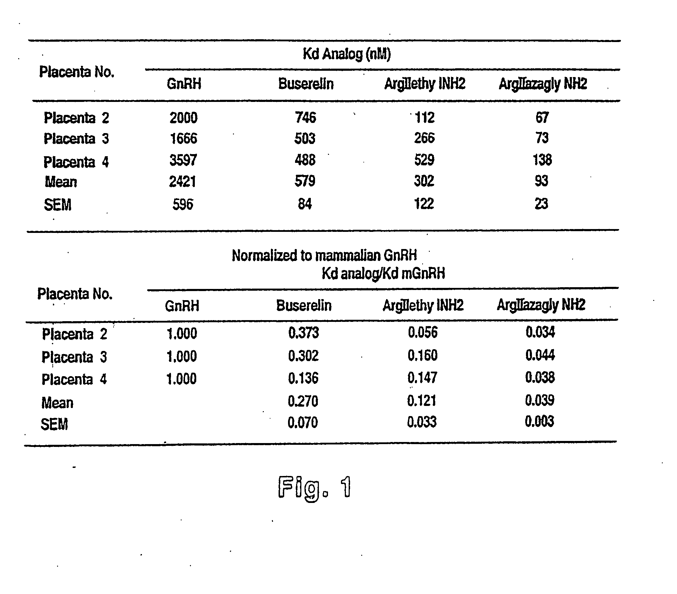

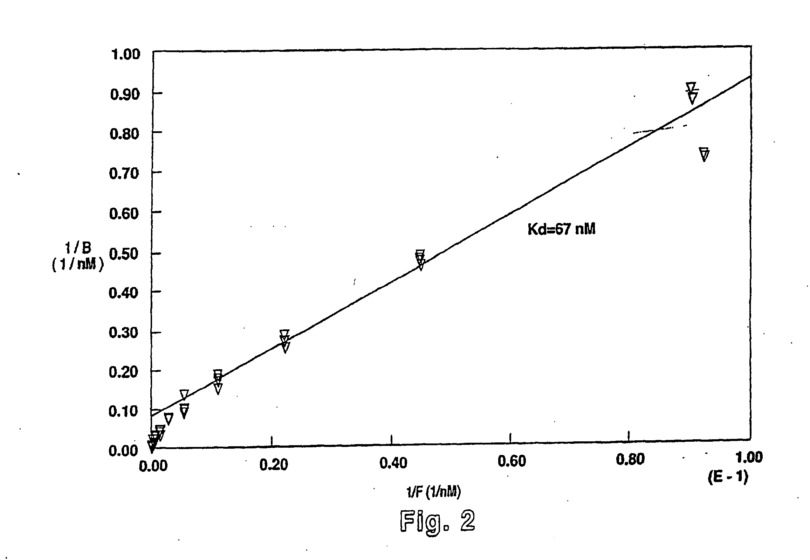

The receptor binding of mammalian and chicken II GnRH isoforms and their analogs were studied using the Buserelin label, and chicken II GnRH (SEQ ID NO: 6) was equipotent to Buserelin (SEQ ID NO: 10) and both were three-fold more potent than mammalian GnRH (SEQ ID NO: 5). The receptor binding for D-Arg- chicken II GnRH-aza-Gly-amide (SEQ ID NO: 2) with the Buserelin label, exhibited a dissociation constant (Kd) of 175∀59 nM (2 fold greater than its natural chicken II GnRH isoform or Buserelin (SEQ ID NO: 10) and 60 fold that of mammalian GnRH (SEQ ID NO: 5)). When D-Arg-chicken II GnRH-aza-Gly-amide analog (SEQ ID NO: 2) was used as a label, the affinity for the placental GnRH receptor was enhanced 2 fold and that for mammalian GnRH (SEQ ID NO: 5) was decreased 1.5 times. Figure 1 compares the average Kd observed for the three different placental membrane preparations for mammalian GnRH (SEQ ID NO: 5), Buserilin (SEQ ID NO: 10), D-Arg(6) chicken II GnRH-aza-Gly (10)-amide (SEQ ID NO: 2) using the D-Arg(6)-chicken II GnRH-aza-Gly-amide (SEQ ID NO: 2) radiolabeled analog. The most potent affinity constant was observed for the D-Arg(6)-chicken II GnRH -aza-Gly(10) amide analog (SEQ ID NO: 2), having a Ks of 68 nM when using the placenta 2 membrane preparation as illustrated in Figure 2. The average binding affinity for this analog was 93∀23 nM (25 fold that observed for mammalian GnRH(SEQ ID NO: 5)).

### Example III - Placental Stability Studies of GnRH Analogs

The present example demonstrates the utility of using the present invention in controlling and modulating the activity of the placenta, such as in a placenta of a pregnant mammal.

Mammalian GnRH (SEQ ID NO: 5) and its analogs bind to placental receptors. The present non-mammalian GnRH analogs had not been examined for placental receptor binding. However, the added stability of these non-mammalian GnRH analogs, would effect a substantial increase in bioactivity alone. Thus, both stability and binding studies were performed.

The enzymatic degradation of the present non-mammalian GnRH analogs were studied using the C-ase-1 enzyme activity assay as well as whole placental homogenate assays. A chorionic peptidase activity that actively degrades GnRH in the placenta, named chorionic peptidase-1(C-ase-1), was used. This enzyme acts as a post-proline peptidase, and is present in the placenta and in maternal circulation. In a non-pregnant individual very little post-proline peptidase activity is present in blood. Thus, currently available mammalian GnRH analogs have not been designed to be resistant to degradation by this activity. Non-mammalian GnRH analogs of the present invention were designed with these specific criteria in mind. The stability of these non-mammalian GnRH analogs to the enzymatic activity of C-ase-1 and in placental homogenate was examined. In addition, the ability of the analogs to competitively inhibit the degradation of mammalian GnRH (SEQ ID NO: 5) by C-ase-1 was studied.

The stability of most potent receptor-active non-mammalian GnRH analogs in the presence of C-ase-1 and placental homogenate was identified. Using the incubation system developed for the C-ase-1 activity, the degradation of each analog was tested. This method has previously been used by the investigator to determine the degradation of GnRH by C-ase-1. Each of these analogs was then studied for its ability to act as a competitive inhibitor of non-mammalian GnRH for C-ase-1 activity. These studies were done using the C-ase-1 enzyme activity assay as described previously. In this assay, incubation of enzyme and mammalian GnRH (SEQ ID NO: 5) with and without the chosen newly synthesized non-mammalian GnRH analog was studied. The reaction was stopped by heating, and the remaining mammalian GnRH (SEQ ID NO: 5) substrate was quantified by radioimmunoassay. The product formed was calculated by subtraction, and its inverse plotted against the inverse of the original substrate concentrations to determine the nature of the competition. The Kᵢ was to be determined by plotting the inverse of the product that formed verses the inhibitor used. *Siler-Khodr TM, Kang IS, Jones MA, Harper MJK, Khodr GS, Rhode J 1989 Characterization and purification of a placental protein that inactivates GnRH, TRH and Angiotensin 11. Placenta 10:283-296.*

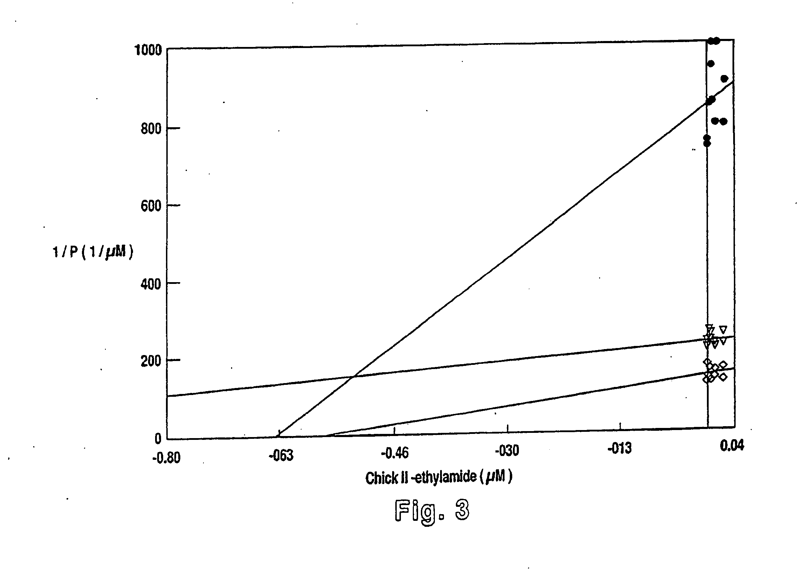

Studies using whole placental homogenate were also performed. The enzymatic degradation of mammalian GnRH (SEQ ID NO: 5) was studied as described above, replacing C-ase-1 with placental homogenate. The competition by the newly synthesized non-mammalian GnRH analogs as compared to mammalian GnRH (SEQ ID NO: 5) was then studied to confirm the C-ase-1 studies above. Similar patterns of inhibition using placental extracts demonstrated the dominance of the C-ase-1 activity in the degradation of GnRH during pregnancy. (Figure 3)

Although the enzyme competition system had already been developed, newly synthesized non-mammalian GnRH analogs have not been utilized in these systems. Previous data generated by the present inventor have demonstrated that the antiserum is specific for mammalian GnRH (SEQ ID NO: 5), thus reducing potential for cross-reaction of non-mammalian GnRH or its analogs in the assay used in these studies.

In these and additional studies, competition for the enzymatic degradation of mammalian GnRH (SEQ ID NO: 5) by a post-proline peptidase was studied by determining the remaining GnRH after incubation of varying concentrations of mammalian GnRH (SEQ ID NO: 5) with a highly active post-proline peptidase, C-ase-1, isolated from term human placentas, in the presence or absence of varying concentrations of other GnRH isoforms or analogs. The remaining GnRH was measured using a radioimmunoassay specific for mammalian GnRH (SEQ ID NO: 5) having less than 0.1% cross-reactivity for any of the analogs or isoforms tested. The concentration of the product of the degraded GnRH was quantified by subtracting the remaining mammalian GnRH from the starting concentrations of mammalian GnRH. Analogs and isoforms of GnRH studied were Buserelin (SEQ ID NO: 10), Leuprolide (SEQ ID NO: 11), chicken II GnRH (SEQ ID NO: 6), and its D-Arg (6), Des-Gly (10) GnRH -ethylamide, and D-Arg (6), aza-Gly (10)-amide (SEQ ID NO: 2) analogs. The Ks for the degradation of mammalian GnRH was calculated from the x axis intercept using Lineweaver-Burke double reciprocal plot of the concentration of the product formed versus the concentration of the substrate used. The inhibitor constant Ki was also calculated from the point of converging lines formed from the plot of the concentration of the product formed using a given concentration of mammalian GnRH in the presence of different concentrations of competing analogs or isoform.

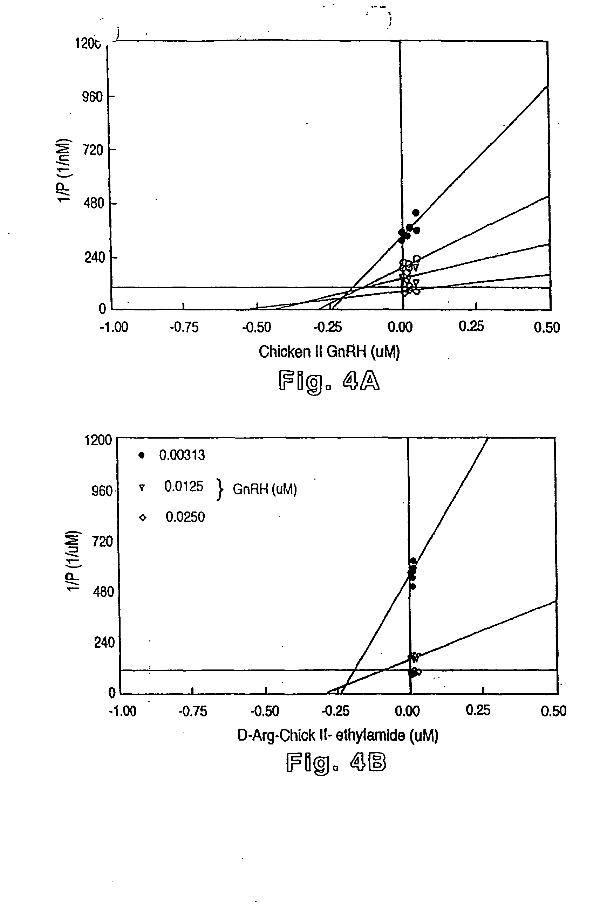

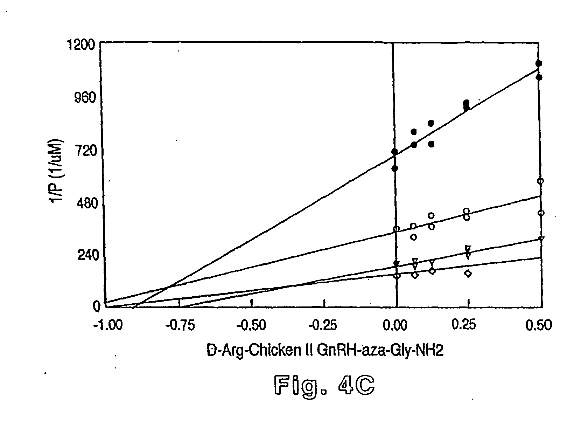

The Ks for mammalian GnRH (SEQ ID NO: 5) degradation by C-ase-1 was ~30 nM. Using the reciprocal plot of the product versus the concentration of the GnRH isoform or analog to determine the Ki, it was determined that Buserelin (SEQ ID NO: 10) was degraded by C-ase-1, although at one fourth the rate of its native mammalian GnRH isoform (Ki of 110 nM). Chicken II GnRH (SEQ ID NO: 6) competed for the degradation of mammalian GnRH (SEQ ID NO: 5) with a Ki of 200 nM (one-sixth that of the mammalian GnRH (SEQ ID NO: 5)). The D-Arg-chicken II GnRH-ethylamide (SEQ ID NO: 2) had a Ki of more than 200 nM and D-Arg (6)-aza-Gly(10) amide analog (SEQ ID NO: 2) of chicken II GnRH was essentially not degraded (Ki of>400 nM). The inhibition of the degradation of mammalian GnRH (SEQ ID NO: 5) by the placental enzyme, chorionic peptidase 1, is shown in more detail in Figures 4a, b, and c.

### EXAMPLE IV - Biological Activity Studies

The hCG inhibiting activity of the chorionic GnRH analogs was studied using an in vitro human placental explant system. The present example demonstrates the utility of using the present non-mammalian analogs to regulate hCG levels in a mammal and in the regulation of pregnancy.

The newly synthesized non-mammalian GnRH analogs are resistant to enzyme degradation and are potent binders of the placental GnRH receptor. Bio-potency was studied using a placental explant system, and by determining the release of hCG, progesterone and prostanoids. hCG is the luteotropin of pregnancy, and known to be critical to the maintenance of the corpus luteum during pregnancy. Thus, it is a primary parameter of interest. The production of progesterone by the placenta and the ovary is affected by hCG, as well as being independently regulated by a GnRH-like substance. Progesterone is primary to the maintenance of uterine quiescence and thus the maintenance of pregnancy, and therefore is of primary interest to these studies. Also, of interest is the effect of these non-mammalian GnRH analogs on prostaglandin production. Prostaglandins are required for abortifacient activity, and thus, the maintenance or increase in their production may be necessary for the proposed action of the non-mammalian GnRH analogs.

The biological activity of the newly synthesized non-mammalian GnRH analogs was studied using a static implant culture system. This system allows for inexpensive extended activity studies. Mammalian GnRH action on the human placenta release of hCG, progesterone and prostaglandins were defined using this system. Replicate cultures were studied, thus allowing for comparison of different doses of each non-mammalian GnRH analog to mammalian GnRH (SEQ ID NO: 5), as well as direct competition assays. In these studies, the action of the most stable and receptor-active chorionic GnRH analogs on hCG, progesterone and prostaglandin E₂ were determined in the spent media using specific sensitive radioimmunoassays. These studies were repeated using different human placentas. *Siler-Khodr TM, Khodr GS, Valenzuela G, Rhode J 1986 Gonadotropin-releasing hormone effects on placental hormones during gestation: II Progesterone, estrone, estradiol and estriol. Biol Reprod 34:255-264; Siler-Khodr TM*, *Khodr GS, Valenzuela G, Rhode J 1986 Gonadotropin-releasing hormone effects on placental hormones during gestation: 1 Alpha-human chorionic gonadotropin, human chorionic gonadotropin and human chorionic somatomammotropin. Biol Reprod 34:245-254; Siler-Khodr TM*, *Khodr GS, Valenzuela G, Harper J*, *Rhode J 1986 GnRH effects on placental hormones during gestation. 111 Prostaglandin E, prostaglandin F, and 13, 14-dihydro-15-keto-prostaglandin F. Biol Reprod 35:312-319.*

Using an in vitro system to define bio-potency is expected to be predictive of in vivo activity. In addition to placental action, these newly synthesized non-mammalian GnRH analogs are also expected to act directly at the corpus luteum to inhibit steroidogenesis. These analogs are also expected to be active at the ovarian level.

In these and additional studies, an explant culture system was used to determine the effect of mammalian GnRH (SEQ ID NO: 5), chicken II GnRH (SEQ ID NO: 6), or the D-Arg(6)-chicken II GnRH-aza-Gly(10)-amide analog (SEQ ID NO: 2) on the release of the hCG, progesterone, and prostaglandin E₂. Human term placentas were dissected free of membranes, minced into fragments of 5 mm, rinsed in medium, and a total weight of ~100 mg (20 explants) was placed on a sterile filter paper resting on an organ culture grid such that they touched the surface of the culture medium, but were not immersed in it. The medium contained penicillin, streptomycin, and fungizone at 100 U/mL, 100 µg/mL, and 2.5 µg/mL respectively with and without varying doses of GnRH isoforms or analogs was added to each Petri dish. Triplicate chambers for each media were made and incubated at 37 C in a humidified chamber with an atmosphere of 5% CO₂ and 95% air. Spent media were collected and replaced after 2 hours, 24 hours, and 48 hour of culture and stored frozen at -20E C until assayed for hormones. HCG, progesterone, and PGE₂ were measured using specific double antibody procedures as described previously. The chicken II GnRH analog (SEQ ID NO: 2) was studied using four different human term placentas, and the native chicken II GnRH isoform was also studied using one human term placenta. *Gibbons JM, Mitnick M, Chieffo V 1975 In vitro biosynthesis, of TSH- and LH-releasing factors by the human placenta. Am J Obstet Gynecol 121:127-131.*

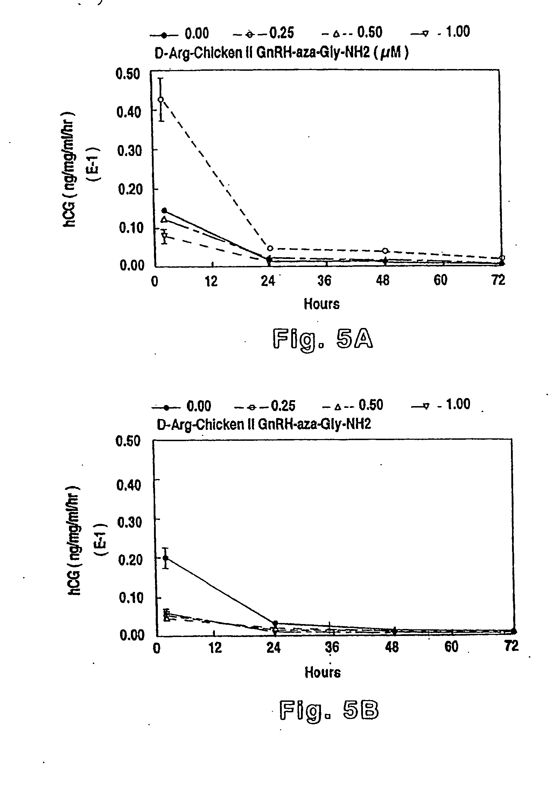

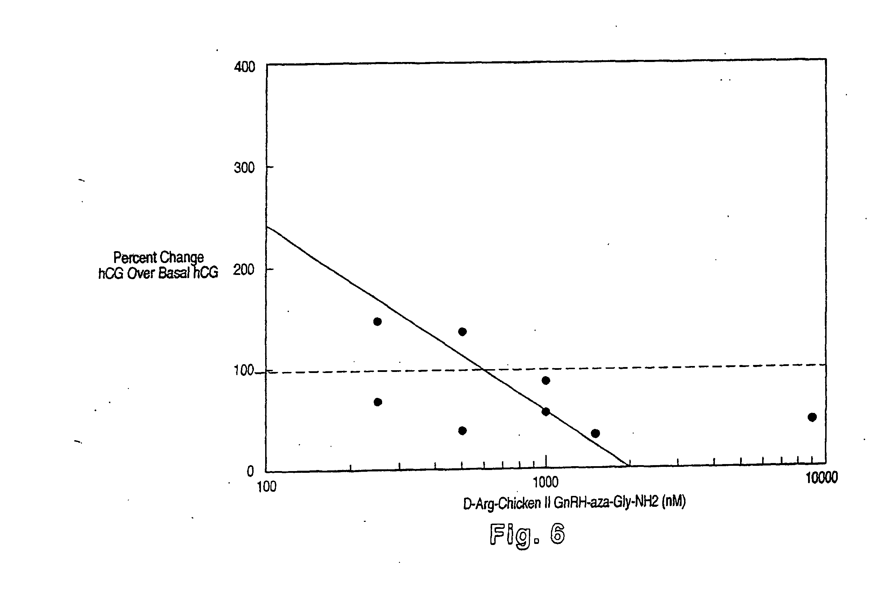

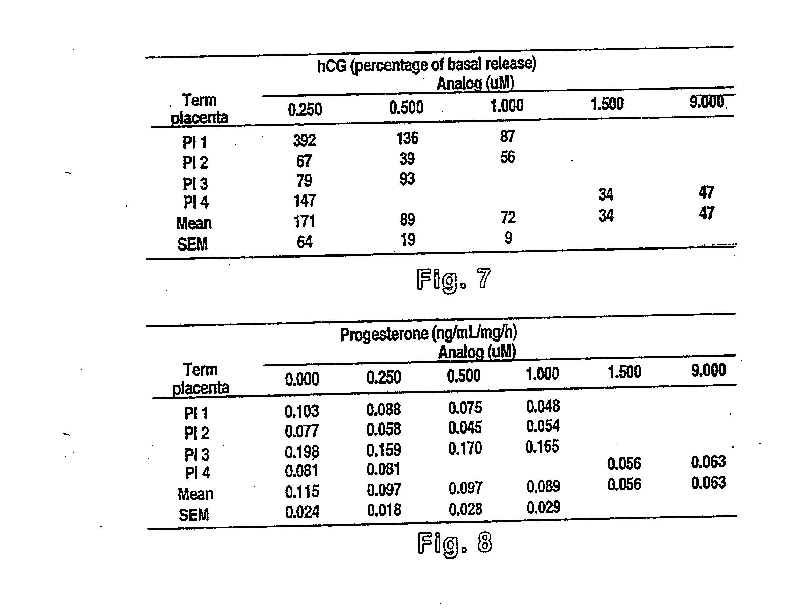

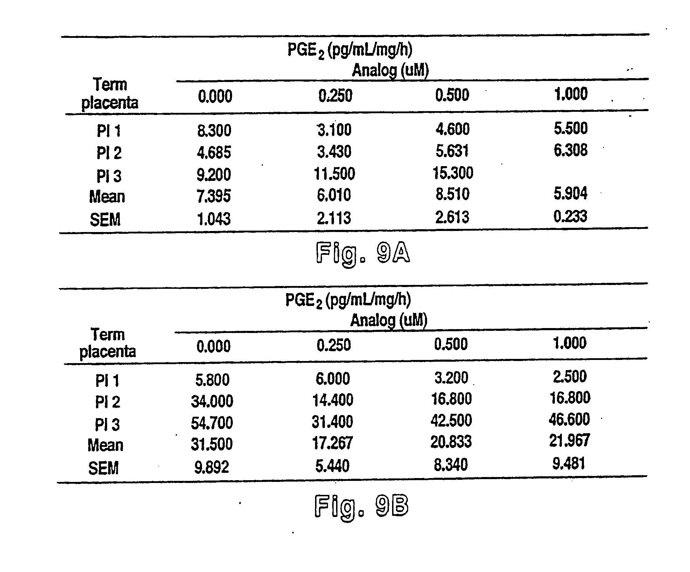

The biopotency of the D-Arg(6) chicken II GnRH-aza-Gly(10) amide analog (SEQ ID NO: 2) was compared with that of mammalian GnRH (SEQ ID NO: 5). The basal release of hCG and progesterone declined after the first day of culture, yet PGE₂ increased throughout the culture period. The addition of mammalian GnRH (0.25 -1.00 µM) (SEQ ID NO: 5) to the media had no significant effect on the release of hCG from four different placentas studied. Progesterone release was not affected by mammalian GnRH (SEQ ID NO: 5) in two of four placentas, but in one placenta it was significantly increased and in the other was decreased. The addition of D-Arg-chicken II GnRH -aza-Gly-amide (SEQ ID NO: 2) (0.25 - 1.00 µM) resulted in as much as a three fold stimulation of hCG during the first two hours of exposure using the lowest concentration of analog tested (250 nM) as illustrated in Figures 5a and 5b. However, the response to D-Arg-chicken II GnRH-aza-Gly-amide (SEQ ID NO: 2) was biphasic i.e. an inhibition of hCG was observed using the higher concentrations (1-9 µM) of the chicken II GnRH analog (SEQ ID NO: 2). After 24 hours and 48 hours of incubation with this analog, a similar pattern of response was observed, even though basal hCG release had fallen 10- to 20- fold during the 2 days in vitro. A significant dose-related inhibition of hCG release (P<0.05) was observed after 2, 24, and 48 hours of treatment with the D-Arg-chicken II GnRH-aza-Gly-amide (SEQ ID NO: 2) as indicated in Figures 6 and 7. The progesterone release was also inhibited when incubated with the higher concentrations of this analog, but not as markedly as hCG in Figure 8. PGE₂ was not significantly changed by exposure to this analog as indicated in Figures 9a and b.

Later studies were done using the D-Arg(6)-chicken II GnRH-aza-Gly(10)-amide analog (SEQ ID NO: 2). Three different placentas were used for these studies. An inhibition of hCG was observed with this non-mammalian GnRH analog regardless of the concentration of exogenous GnRH. The lower dose of non-mammalian GnRH analog was the most effective in this particular study. Progesterone response to this non-mammalian GnRH analog was similar to hCG.

These data demonstrate the complexity of a system having multiple types of GnRH receptors. D-Arg(6)-chicken II GnRH-NH₂ analog (SEQ ID NO: 2) has bioactivity in the regulation of hCG and progesterone in the human term placenta.

These studies demonstrate specific binding of non-mammalian GnRH analogs to the human GnRH placental receptor, which is unique from the pituitary receptor. The most potent analogs were chicken II GnRH derivatives, particularly the D-Arg(6)-chicken II GnRH-aza-Gly¹⁰ NH₂ (SEQ ID NO: 2). This analog may be used in the regulation of chorionic GnRH activity.

### EXAMPLE V - Inhibition Of Chorionic peptidase-I (C-ase-1) Activity by Analogues of GnRH

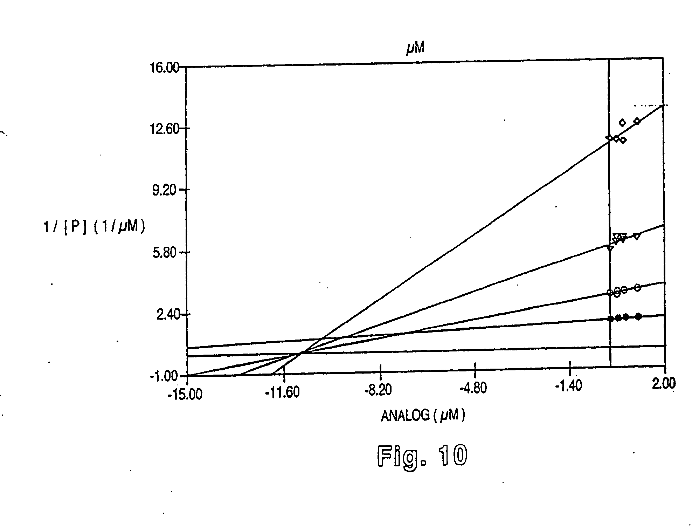

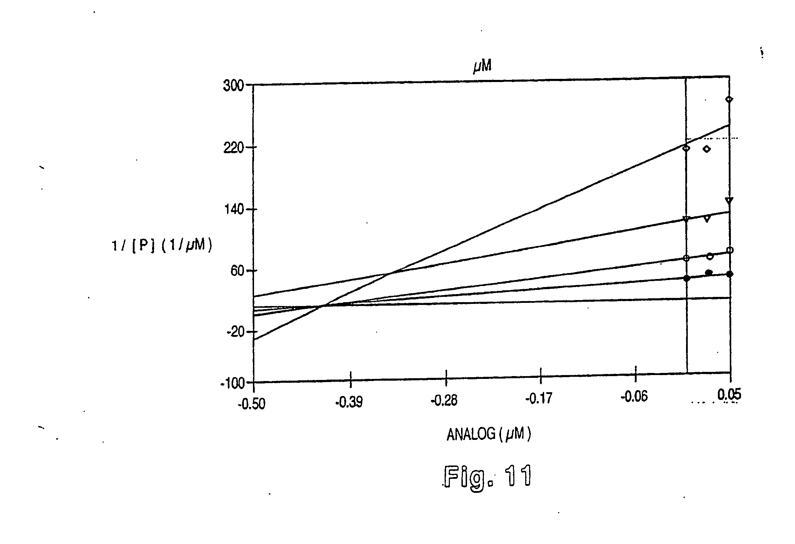

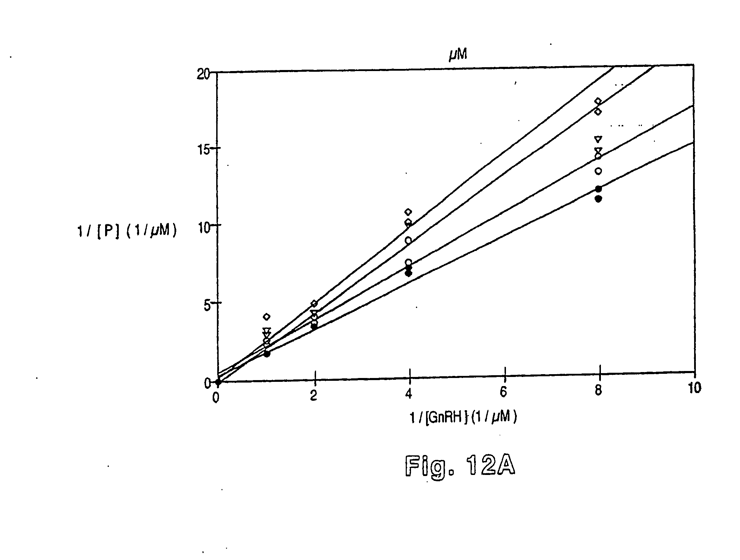

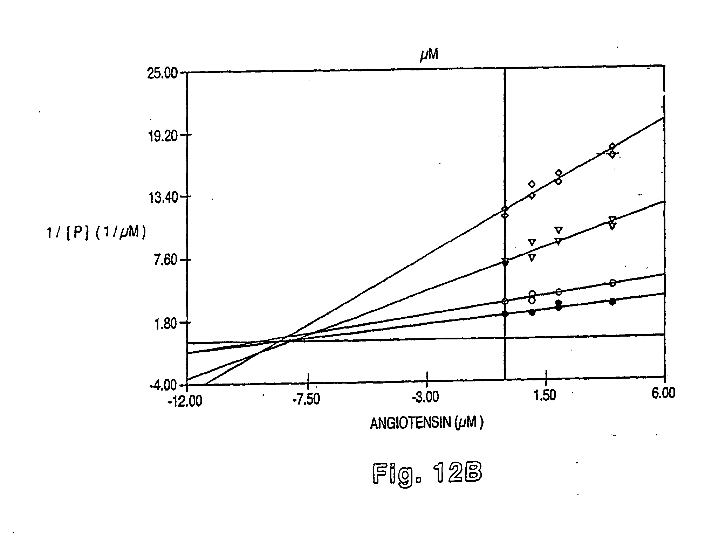

The present example demonstrates the isolation of an enzyme from human placentas, and the action of the enzyme as a post-proline peptidase. It actively degrades peptides, such as gonadotropin releasing hormone (GnRH), thyrotropin releasing hormone (TRH), reduced oxytocin, and Angiotensin II (Ang-II). See Figures 10, 11, 12A, and 12B. These peptides contain a proline residue where the chorionic peptidase-1 (C-ase-1) is to cleave its C-terminal peptide bond.

The present example also defines enzyme inhibitors of C-ase-1 action on GnRH, such that it might regulate GnRH concentrations within the intrauterine tissues.

C-ase-1 enzyme activity studies were done by incubating GnRH with C-ase-1 in the presence of varying concentrations of the non-mammalian GnRH analogs. The reaction was stopped by heating at 85°C for 10 minutes. The remaining GnRH was determined using a specific radioimmunoassay. The formation of product, i.e., the N-terminal nonapeptide of GnRH, was calculated by subtraction and its inverse was plotted versus the inverse of the initial substrate to determine the Kₛ of the reaction. The inhibitory activity of Antide (SEQ ID NO: 12), ⁶Im-btl-D-His-GnRH-¹⁰ ethylamide, ⁹OH-Prl-GnRH, chicken II GnRH-¹⁰ ethylamide, chicken II GnRH (SEQ ID NO: 6), chicken I GnRH (SEQ ID NO: 13), salmon GnRH (SEQ ID NO: 7) and lamprey GnRH (SEQ ID NO: 14) was studied. The relative potency of each analog was compared.

GnRH was actively degraded by C-ase-1. This activity of C-ase-1 was inhibited by ⁹OH-Pro-GnRH, lamprey (SEQ ID NO: 14), chicken I-GnRH (SEQ ID NO: 13), Antide (SEQ ID NO: 12), chicken II-GnRH (SEQ ID NO: 6) and salmon GnRH (SEQ ID NO: 7) with a relative potency of 1.5, 1.5, 0.6, 0.6, 0.2 and 0.2, respectively, compared to that for GnRH.

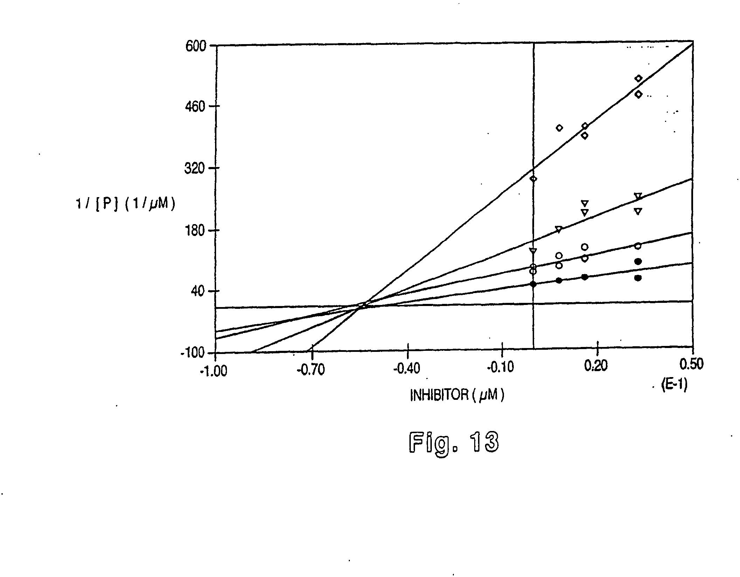

Chorionic peptidase-1, which is a post-proline peptidase with high specificity for the degradation of GnRH, can also degrade other GnRH species. The synthetic mammalian GnRH analogs such as Antide (SEQ ID NO: 12) (see Figure 13) are degraded with reduced activity, while other analogs such as chicken II GnRH-¹⁰ aza-Gly-amide and ⁶Im-btl-D-His-GnRH¹⁰ ethylamide are resistant to degradation by this endogenous chorionic enzyme. See Figure 14. These analogs will be useful in the regulation of chorionic GnRH activity.

### EXAMPLE VI - Comparison Of GnRH And Its Synthetic And Naturally Occurring Analogs For Binding Action in The Human Placental Receptor

The human placental GnRH receptor shows different kinetic constants for GnRH compared to that of the pituitary receptor. The relative decreased potency of GnRH at the placental receptor, together with it rapid degradation in chorionic tissue, leads to question if it is indeed the active sequence for the chorionic receptor.

Studies were designed to compare the human placental receptor activity for numerous synthetic and naturally occurring analogs.

Receptor assays were performed by incubating human term placental GnRH receptors with varying concentrations of GnRH or its analogs in the presence of ¹²⁵I-Buserelin. The reaction was stopped and the bound hormone precipitated with polyethylene glycol. Following centrifugation the receptor binding activity was calculated and compared for GnRH, ⁶Im-btl-D-His-GnRH¹⁰ ethylamide and ⁶D-Trp-GnRH-¹⁰ ethylamide, chicken II-GnRH (SEQ ID NO: 6) and chickenII GnRH-¹⁰ ethylamide. GnRH was bound by the placental GnRH receptor with a K_{d} of 10⁻⁶ M. Chicken II GnRH (SEQ ID NO: 6) was similar to GnRH. The K_{d} for -⁶Im-btl-D-His-GnRH¹⁰ ethylamide was half the potency of GnRH, while Buserelin (SEQ ID NO: 10) and ⁶D-Trp-GnRH-¹⁰ ethylamide were twice as active as GnRH. The greatest potency was for chicken II GnRH ethylamide, having a K_{d} of 30 non-mammalian, i.e. 33-fold more activity than GnRH. See Figure 15.

### EXAMPLE VII - GnRH And Stability Thereof In The Presence of C-ase-1

Fifteen GnRH analogs were examined for their stability in the presence of C-ase-1 and placental homogenate. Using the incubation system developed for the C-ase-1 activity, the degradation of each analog was studied. Previously, this method was used to determine the degradation of GnRH by C-ase-1. Each of these analogs was studied for their ability to act as competitive inhibitors of GnRH for C-ase-1 activity (Table 1). The inverse of the product was plotted against the inverse of the original substrate concentrations to determine Ks of the competition. The Kᵢ was determined by plotting the inverse of the product formed verses the inhibitor used. The placental homogenate studied, demonstrated a similar pattern having Kᵢ three-fold greater than that for C-ase-1.

OH-Pro(9)-GnRH and lamprey GnRH (SEQ ID NO: 14) were determined to be better competitors for GnRH degradation by C-ase-1. They are as or even more potent than GnRH. Antide (SEQ ID NO: 12) and chicken I GnRH (SEQ ID NO: 13) are three-fold less potent than GnRH, but two-fold more potent than the salmon (SEQ ID NO: 7) or chicken II GnRHs (SEQ ID NO: 6) defined here. The addition of the ethylamide to GnRH, with or without the D-Trp(6) or Phe(6) substitution, decreased the competition with GnRH for C-ase-1 degradation, but not as markedly as did the Im-Btl-D-His(6) or chicken II GnRH-ethylamides. Ethylamides of the latter two GnRHs were greater than 200-fold less active in the inhibition of GnRH degradation by C-ase-1. Thus, these ethylamides appear to be very stable in the presence of the C-ase-1 enzyme. The Im-Btl-His(6) analog has reduced receptor potency. The stability of the D-Arg-(6)-chicken II GnRH aza-Gly-amide (SEQ ID NO: 2) was found to be at least 200-fold that of GnRH.

The stability of these analogs in the present of whole placental homogenates was examined. The ethylamide derivative has a slowed degradation rate as compared to GnRH, but can be degraded. Chicken II and its ethylamide analog are more stable than the mammalian GnRH analogs analyzed to date.

### EXAMPLE VIII - Non-Mammalian GnRH and

### Methods for Maintaining Pregnancy

The present example defmes a method by which the present invention may be used to maintain pregnancy in a pregnant mammal. The mammal in some embodiments is a pregnant human. As a proposed dose regimen, it is anticipated that a pregnant female between 100 lbs and 150 lbs would be administered about 10 nanogram to 1.0 gram of chicken II GnRH analog (SEQ ID NO: 2) or salmon GnRH analog (SEQ ID NO: 4). This would be expected to be effective for promoting the maintenance of pregnancy in the mammal when administered.

In some embodiments, the dosing regimen will comprise a pulsatile administration of the chicken II GnRH over a 24-hour period, wherein the daily dosage is administered in relatively equal 1/24^{th} fractions. For example, where the daily dose is about 2.4 micrograms, the patient would be administered about 0.1 micrograms per hour over a 24-hour period. Such a daily pulsatile administration would create a hormonal environment in the patient sufficient to maintain pregnancy. The particular pharmaceutical preparations may be created by one of skill in the pharmaceutical arts. Remington=s Pharmaceutical Sciences Remington: The Science and Practice of Pharmacy, 19^{th} edition, Vol. 102, A.R. Gennaro, ed., Mack Publishing Co. Easton, PA (1995), is specifically incorporated herein by reference for this purpose.

### EXAMPLE IX - Non-mammalian GnRH Analogs and Post Coital Contraception, Menses-Inducement

The present example demonstrates the utility of the present invention for use as a post-coital contraceptive preparation.

By way of example, the non-mammalian GnRH analogs defined here, and conservative variants thereof, may be formulated into a pharmaceutically acceptable preparation, and then administered to a female mammal having been inseminated during the prior 24 to 72 hours (prior 1 to 3 days). Relatively high doses of about 0.1 gram to about 10 grams of the non-mammalian GnRH analog would be given daily for 2 to 5 days, on the average about 3 days. To induce menses, it is anticipated that a dose of between 0.1 grams micrograms to 10.0 grams for 3 days would be adequate to commence menses in the female mammal.

For purposes of practicing the present invention as an oligonucleotide in molecular biology applications, the non-mammalian GnRH analogs of chicken II (SEQ ID NO: 1) and salmon decapeptide GnRH analog cDNA sequences (SEQ ID NO: 3) would be employed. The textbook of Sambrook, et al (1989) *Molecular Cloning, A Laboratory Manual,* 2d Ed.; Cold Springs Harbor Laboratory, Cold Springs Harbor, N.Y., is specifically incorporated herein by reference for this purpose. By way of example, the cDNA sequence for the non-mammalian GnRH of SEQ ID NO: 1 (chicken II GnRH) or SEQ ID NO:3, (salmon GnRH) may be prepared as part of a suitable vector, such as in an adenovirus or retroviral vector, and administered to the animal. Once the sequence is incorporated into the cell, the peptide product will be translated and peptide supplied. Because this method of treatment would not require that the peptide travel in the blood circulation in order to reach the site of action, there would be no requirement that the analog possess enzyme degradation resistance. This mode of treatment has not thus far been proposed, and hence the use of such a method in the regulation of female fertility is a novel clinical regimen.

The non-mammalian GnRH analogs are also contemplated to be useful to directly affect the ovary. By way of example, this technique renders the system useful as a contraceptive. As a contraceptive, the non-mammalian GnRH analog would be given daily from the start of ovulation and continue 8 days to two weeks, stopping with onset of menses. In addition, it is contemplated that the activity of the present non-mammalian GnRH analogs would prove useful in the treatment of ovarian conditions, such as polycystic ovarian disease and ovarian cysts.

### EXAMPLE X - Antibodies Specific for Non-Mammalian GnRH and GnRH Receptor

The present example demonstrates the utility for using the present non-mammalian GnRH analog decapeptides to prepare antibodies that preferentially bind the GnRH peptide sequences, or that bind the ovarian, placental or any other non-pituitary GnRH peptide or protein, or the receptors therefor. It is anticipated that these non-mammalian GnRH analog antibodies may be used in a variety of screening assays. For example, these antibodies may be used to determine levels of GnRH, or the GnRH receptor, present in a sample as an indicator molecule. The levels of such GnRH may be used to monitor and follow a patient=s pregnancy as well as an indicator of the length of gestation. The antibodies to non-mammalian GnRH may be monoclonal or polyclonal antibodies.

Polyclonal antibodies may be created by standard immunization techniques, wherein the immunogen used will be the non-mammalian chicken-II GnRH analog (SEQ ID NO: 2) or the salmon GnRH analog (SEQ ID NO: 4) decapeptide described herein. These peptides may be used either alone or together in a pharmaceutically acceptable adjuvant. The animal, such as a rabbit, would be administered several doses of the decapeptide preparation, and the levels of the animal=s antibody blood levels monitored until an acceptable antibody level (titer) had been reached.

For the preparation of monoclonal antibodies, one would follow standard techniques for the immunization of an animal, again using the decapeptide non-mammalian GnRH peptide. Once sufficiently high acceptable antibodies are reached (titer) in the animal, the spleen of the animal would be harvested, and then fused with an immortalized cell line, such as a cancer cell line, to produce a population of hybridoma cells. This hybridoma population of cells would then be screened for those that produce the highest amount of antibody that specifically bind the non-mammalian GnRH analog decapeptide. Such hybridoma cells would be selected, and then cultured. The antibody to non-mammalian GnRH or GnRH receptor would then be collected from the media of the cell culture using techniques well know to those of skill in the art.

For purposes of the practice of preparing polyclonal and monoclonal antibody, the textbook Sambrook et al (1989) *Molecular Cloning, A Laboratory Manual,* 2^{nd} Ed., Cold Springs Harbor Laboratory, Cold Springs Harbor, N.Y., is specifically incorporated herein by reference. All of the compositions and methods disclosed and claimed herein can be made and executed without undue experimentation in light of the present disclosure.

### EXAMPLE XI - HCG Stimulating and Inhibiting Activity of the Non-Mammalian GnRH Analogs

The acute activity of the chicken II GnRH analogs (SEQ ID NO: 2) on hCG release was studied using a human placental explant perifusion system. Prior studies have demonstrated the long term effect of these newly synthesized non-mammalian GnRH analogs and their biological action using a static culture system. A dose-related biphasic response was noted over time. In the perifusion studies, the dynamic of the response to continuous exposure of the chorionic GnRH agonist can be better defined. The system better emulates the in vivo situation and provides a better understanding of the effect of the analogs on chorionic hormonogenesis and the applicable dose appropriate for future studies.

Chicken II GnRH analog with D-Arg at position 6 and aza-Gly-amide at position 10 (SEQ ID NO: 2) and commercially available mammalian GnRH agonist, Buserelin (SEQ ID NO: 10), were used in four different placental perfusion studies. Placental tissues that are normally discarded were obtained from unidentified patients following first trimester pregnancy termination (early human placental explants). Tissue fragments, dissected of vessels and membranes, were placed in a perfusion system for study of 20 replicate chambers of the same tissue. This allows for simultaneous dose-response studies to be performed. To achieve this specific aim, explants from a given placenta were placed in 20 replicate chambers and perfused with basal medium for three hours at a rate of 6 ml/hr (dead volume of the system at 6ml/hr is ten minutes). After three hours of equilibration, the analog was added to the basal perfusing medium. Quaduplicate chambers were made for chicken II GnRH analog (SEQ ID NO: 2) at 0, 10⁻⁹, 10⁻⁸, 10⁻⁷, 10⁻⁶ M and Buserelin (SEQ ID NO: 10) at 10⁻⁷ M. The effluent medium of each chamber was collected after a three hour equilibration period. Two basal samples, at half hour intervals were collected, media with test substances was initiated and effluent medium collection continued for four and one half hours at thirty minute intervals. The hCG release was analyzed in each of these experiments. Figures 16 and 17 illustrate a typical response. A dose related biphasic response was observed. Maximal response was observed within minutes after initiation of perfusion at 10⁻⁸ M, with possible down regulation beginning after five hours at 10⁻⁶ M as illustrated in Figure 18. The integrated response over the 4.5 hours of perfusion also demonstrated the biphasic response with maximal stimulated response using 10⁻⁸M ofthis analog as seen in Figure 19.

In additional experiments, early gestation human placentas were perfused for six hours with medium supplemented with estradiol, progesterone, bovine serum albumin, and antibiotics (basal medium). Twenty replicate chambers were perifused with basal medium for two hours, then triplicate chambers were perifused with the medium containing either Buserelin (SEQ ID NO: 10), or 10⁻⁹, 10⁻⁸, 10⁻⁷, 10⁻⁶ M of a chicken II GnRH analog (SEQ ID NO: 2), leaving five control chambers. The eluted medium was collected at thirty minute intervals, starting one hour prior to the addition of GnRH analogs and throughout five hours of treatment. hCG released into the effluent media, as well as the production of GnRH, were measured using sensitive and specific radioimmunoassays. The release of hCG at each time point was normalized to its zero treatment release and the release throughout the treatment period and its average release was compared for various treatment regimens. These studies were repeated using tissues from three different first trimester placentas. The results of this experiment are illustrated in Figure 20.

hCG release from control chambers decreased over the five hours of treatment to approximately 60% of its initial release. The addition of varying concentrations of chicken II GnRH (SEQ ID NO: 2) analog to the perifusing media resulted in a biphasic stimulation of hCG from these early placental tissues. The greatest response has been observed using 10⁻⁸ M of this chicken II GnRH analog (SEQ ID NO: 2). The response decreased with increasing concentrations of the chicken II analog. Incubation with Buserelin (SEQ ID NO: 10), the mammalian GnRH analog, at 10⁻⁷ M resulted in a small stimulation of hCG at early time points. The average stimulation of hCG release throughout the five hours of perifusion was 150% that of the control tissues.

These studies have led to the definition of the action of chicken II GnRH (SEQ ID NO: 6) and newly designed analogs on placental, ovarian, endometrial, and pituitary tissues. It has been shown that these non-mammalian GnRH analogs are capable of inhibiting hCG and progesterone production in placental tissues after extended exposure. A direct action on ovarian and/or endometrial tissue was demonstrated. A potential direct contraceptive action of these non-mammalian GnRH analogs, as well as their placental hCG and steroidogenic suppression activity is indicated. Such non-mammalian GnRH analogs could be used as a menses regulator, contraceptive, abortifacient or in any other manner to function in regulating reproductive function and disorder and would be valuable agents in population control.

### EXAMPLE XII - Receptor Binding Activity of Non-Mammalian GnRH Analogs at Ovary and Uterus

The receptor binding activity of newly synthesized chorionic GnRH analogs was studied in ovarian, uterine, and pituitary tissues.

There is an ovarian, tubal, and uterine receptor for GnRH which is distinct from that in the pituitary. Existing mammalian GnRH analogs have been designed for activity at the pituitary receptor. These analogs do not demonstrate high potency for the ovarian, tubal, or uterine receptor. The non-mammalian GnRH analogs of the present invention have high affinity for the ovarian, tubal, uterine, and placental receptor and limited degradation by the chorionic enzyme C-ase-1. A similar receptor and enzyme appears to be acting in the ovary and uterus, but not the pituitary. The present study was designed to define the receptor binding of newly synthesized non-mammalian GnRH analogs in the ovary, uterus, and the pituitary and to compare them to the receptor binding of known mammalian GnRH analogs.

The receptor binding activity of newly synthesized non-mammalian GnRH analogs was studied in ovarian and uterine tissues. Synthesized chicken GnRH analogs with D-Arg at position 6 and aza-Gly at the 10 position (SEQ ID NO: 2) and commercially available mammalian GnRH (SEQ ID NO: 5), chicken II GnRH (SEQ ID NO: 6), and Buserelin (SEQ ID NO: 10) were used in the receptor binding studies using three different baboon ovaries . The ovaries of the three baboons were extracted and the cytosolic and membrane fractions were recovered for the tissues. The membrane fraction from one animal was titred for GnRH receptor binding activity using D-Arg(6)-chicken II GnRH-aza-Gly(10)-amide radiolabeled ligand. Receptors were clearly demonstrable even at 4 µg membrane protein/tube. See Figure 21. These tissues expressed a specific activity of binding for this non-mammalian GnRH analog which was about 50 -100 fold more potent than the placental membrane preparations studied to date. The GnRH receptor affinity for this non-mammalian GnRH analog was found to be 10⁻⁸ M as indicated in Figure 22. The chicken II GnRH analog (SEQ ID NO: 2) had the highest affinity for any GnRH analog reported to date. Mammalian GnRH (SEQ ID NO: 5) was rapidly degraded by baboon ovarian cytosol fractions, yet the chicken II GnRH analog (SEQ ID NO: 2) was resistant to such degradation.

One study using human granulosa cells was performed and receptor binding for the GnRH analog was observed. Due to the limited number of cells it was not possible to precisely define the affinity of this receptor. In another study using baboon endometrium (stroma and epithelium) the chicken II GnRH analog (SEQ ID NO: 2) receptor affinity was found to be approximately 60 nM.

Baboon uterine tissue was also demonstrated to have a GnRH receptor with high binding affinity for the chicken II GnRH analog (SEQ ID NO: 2). These chicken II GnRH analogs (SEQ ID NO: 2) may have particular applicability for regulation of implantation and in uterine tissue conditions, such as endometriosis, abnormal uterine bleeding, and leiomyomas. Thus, high affinity receptors for chicken II GnRH analogs (SEQ ID NO: 2) have been defined in baboon ovary and uterus tissues.

### EXAMPLE XIII - Stability of GnRH Analogs in Ovarian Homogenates

The stability of newly synthesized chicken II GnRH analogs (SEQ ID NO: 2) in ovarian, endometrial, and pituitary homogenates was determined using enzyme activity assays.

Chorionic peptidase which actively degrades GnRH in the placenta will be called chorionic peptidase 1 (C-ase-1). The enzyme acts as a post-proline peptidase and is present in maternal circulation. In non-pregnant individuals very little post-proline peptidase activity is present in the circulation. Thus, GnRH analogs in the prior art have not been designed to be resistant to this activity. These studies were designed to test the stability of the present non-mammalian GnRH analogs to the enzymatic activity in ovarian tissue.

The stability of newly synthesized non-mammalian GnRH analogs in baboon ovarian homogenates was determined using an enzyme activity assay. More specifically, chicken II GnRH analogs with D-Arg at position 6 and aza-Gly amide at position 10 (SEQ ID NO: 2) were studied. In these studies the enzyme was in the ovarian extract. It was found that baboon ovary actively degrades mammalian GnRH (SEQ ID NO: 5) as illustrated in Figure 23. The endogenous peptidase specific activity in the degradation of GnRH was tenfold that ofplacental cytosolic fractions.

To determine the stability of the non-mammalian GnRH analog in the baboon ovary, an incubation system was used similar to that developed to study GnRH and its isoforms and analogs in the presence of chorionic tissues except baboon ovarian homogenates were substituted for C-ase-1. In this assay, following incubation of the enzyme and GnRH, with and without the chosen analog, the reaction was stopped by heating and the remaining GnRH substrate was quantified by radioimmunoassay. The product formed is calculated by subtraction, and its inverse plotted against the inverse of the original substrate concentrations to determine the nature of the competition. The Ki was determined by plotting the inverse of the product formed versus the inhibitor used. Figure 24 illustrates the ability of D-Arg-6-chicken II GnRH-aza-Gly-amide (SEQ ID NO: 2) to act as a competitive inhibitor of GnRH for the baboon ovarian enzymatic activity. Since this analog did not significantly compete with the degradation of mammalian GnRH (SEQ ID NO: 5) in the baboon ovary, it is therefore, for all essential purposes, stable in the baboon ovary. Three different ovaries were tested and similar results were obtained as illustrated in Figure 24. It should be appreciated that the degradation of mammalian GnRH (SEQ ID NO: 5) by ovarian tissues has a Ks of ~30 nM. The Ki of the chicken II GnRH analog (SEQ ID NO: 2) is greater than or equal to 1,500 nM. Thus, the stability of this non-mammalian GnRH analog is more than 50 times greater than that of mammalian GnRH (SEQ ID NO: 5).

### EXAMPLE XIV - Bioactivity of Non-Mammalian GnRH Analogs on Ovarian Estradiol and Progesterone Production, Endometrial Prostaglandin E2 (PGE₂) and on Pituitary Luteinizing Hormone (LH) Release

The bioactivity of the newly synthesized non-mammalian GnRH analogs on ovarian estradiol and progesterone production, endometrial stromal prolactin and endothelial prostaglandin production and pituitary luteinizing hormone release was determined using ovarian, endometrial, and pituitary cell cultures.

It was anticipated that certain non-mammalian GnRH analogs tested in the stability and receptor assays would be active in culture. Their biological activity on hormone production, which is the ultimate parameter of function, was studied for each of the specifically designed non-mammalian GnRH analogs and compared to that of closely related pituitary analogs, such as mammalian GnRH (SEQ ID NO: 5) and Buserelin (SEQ ID NO: 10).

The bioactivity of newly synthesized non-mammalian GnRH analogs and commercially available mammalian GnRH agonist Buserelin (SEQ ID NO: 10) on ovarian steroid production, endometrial stromal and epithelial prostaglandin production and pituitary luteinizing hormone release was determined using baboon and human granulosa cells, human endometrial, both epithetial and stromal cell lines and pituitary cell cultures, respectively, from rats and baboons. Using analog concentration of 10⁻⁷ M, baboon LH was not stimulated, but an increase in rat LH was observed for the respective pituitary cell cultures as indicated in Figure 25. The dose-response action of this analog (10⁻⁶ to 10⁻⁹ M) on two different baboon pituitaries has now been analyzed and the results recorded in Figure 26. Ovarian cell cultures from two different pregnant rats were completed and an inhibition of progesterone production was observed as seen in Figure 27.

The biopotency of chicken II GnRH analogs (SEQ ID NO: 2) in the regulation of baboon ovarian function was studied. The effect of mammalian and chicken II GnRH analogs (SEQ ID NO: 2) on ovarian progesterone release was studied using granulosa cell cultures. The dose-related action of a stable analog of chicken II GnRH (SEQ ID NO: 2) on progesterone production was defined using baboon granulosa cell cultures. After a two hour basal study period, the medium was supplemented with either Buserilin (SEQ ID NO: 10) (10⁻⁷) or a chicken II GnRH analog (SEQ ID NO: 21 (10⁻⁹, 10⁻⁸, 10⁻⁷, 10⁻⁶ M) leaving four control wells. The average progesterone releases normalized to each well=s basal release after 22 and 46 hours was compared among groups as seen in Figures 28 and 29. Incubation of baboon granulosa cells with the chicken II GnRH analog (SEQ ID NO: 2) resulted in a dose-related inhibition of progesterone release attaining maximal inhibition at 10⁻⁸M within 24 hours of exposure, i.e. 31% of untreated controls as seen in Figure 30. This inhibition was sustained after 48 hours of treatment. Buserelin (SEQ ID NO: 10) was without significant effect. In addition, the action ofthe analog on three different stromal and three different epithelial endometrial primary cell lines (after 3 - 5 passages) has been studied and PGE₂ determined as indicated in Figure 31.

### EXAMPLE XV- Non-Mammalian GnRH Analogs and Methods of Use in Treatment of Conditions of the Ovary, Fallopian Tubes, and Uterus

Due to the stability of the non-mammalian GnRH analogs, particularly chicken II GnRH (SEQ ID NO: 2) and salmon analogs (SEQ ID NO: 4), in the blood and reproductive tissues, the presence of binding receptors in reproductive tissues, and their biological activity in reproductive tissues, such non-mammalian GnRH analogs can be used in the treatment of conditions of or regulation of the reproductive system and the tissues therein including, but not limited to the endometrium, ovary, fallopian tubes, and uterus. Such treatment or regulation may be for endometriosis, polycystic ovarian disease, ovarian cysts, tubals, abnormal uterine bleeding, leiomyomas, endometrial polyps, fallopian tube mobility, function or obstruction, ectopic pregnancy, molar pregnancy, trophoblastic disease, abnormal placentation, such as pre-eclampsia, intrauterine growth retardation, preterm labor, preservation of the ovary during chemotherapy, in vitro fertilization, and ovarian atresia.

Conventional methods, known to those of ordinary skill in the art of medicine, can be used to administer the pharmaceutical formulation(s) to the patient. Typically, the pharmaceutical formulation will be administered to the patient by intramuscular injection, subdermal pellet, or nasal spray. The pharmaceutical formulation(s) can also be administered via other conventional routes (e.g., oral, subcutaneous, intrapulmonary, transmucosal, intraperitoneal, intrauterine, vaginal, sublingual, or intrathecal routes) by using standard methods. In addition, the pharmaceutical formulations can be administered to the patient via injection depot routes of administration such as by using 1-, 3-, or 6-month depot injectable or biodegradable materials and methods.

Regardless of the route of administration, the therapeutical agent typically is administered at a daily dosage of 0.001 µg to 30 mg/kg of body weight of the patient. The pharmaceutical formulation can be administered in multiple doses per day, if desired, to achieve the total desired daily dose or as a long acting depot.

The effectiveness of the method of treatment can be assessed by monitoring the patient for known signs or symptoms of the disorder. Common symptoms of endometriosis include onset of increasing painful periods, steady dull to severe lower abdominal pain, pelvic or low back pain that may occur at any time during the menstrual cycle, severe pelvic cramps or abdominal pain that may start 1 to 2 weeks before the menstrual cycle, more frequent or totally irregular periods, premenstrual spotting, pain during or following sexual intercourse, pain with bowel movements, and infertility. A laparoscopy is typically performed to make the determination. For ovarian cysts, the symptoms include abnormal uterine bleeding (lengthened, shortened, absent, or irregular menstrual cycle), constant dull aching pelvic pain, pain with intercourse or pelvic pain during movement, pelvic pain shortly after onset or cessation of menses, nausea/vomiting or breast tenderness similar to that associated with pregnancy. Prolonged symptoms that may be associated with polycystic ovarian disease include abnormally light or lack of menstrual periods, infertility, obesity, swollen abdomen, abdominal mass, and hirsutism. Hormonal level tests are typically ordered including FSH, LH, estrogen, and pregnanediol. A serum hCG test may be done to rule out pregnancy.

The symptoms of abnormal uterine bleeding, uterine fibroids, or leiomyomas, may include menorrhagia, menometrorrhagia, severe pressure or pain, urinary or bowel complaints, recurrent abortions, and infertility. Some patients may however be asymptomatic. Diagnosis is made by pelvic examination and can be confirmed by ultrasonography, CT or MRI. While discussion has been made concerning specifically the female reproductive system, this invention have great applicability in the male reproductive system and conditions of the male reproductive system as the developmental reproductive biology of males and females is known by those skilled in the art to have a common origin. By way of example only, it is anticipated that the present invention in the treatment of conditions of or regulation of male reproductive tissues has particular applicability in testicular descent, testicular function, prostate function and preservation of testis during chemotherapy. In addition, it is believed by the present inventor that the non-mammalian GnRH analogs of the present invention can be used in the regulation of the immune system in pregnant and non-pregnant individuals and in systemic lupus erythematosus.

### EXAMIPLE XVI-Design of Non-Mammalian GnRH Analogs for Use in the Male Reproductive System

The present example outlines how analogs of non-mammalian GnRH with increased activity in male reproductive system tissues including human sperm, testicular, scrotal, seminiferous tubule, Leydig cell, Sertoli cell, epididymis, vas deferentia, prostate, seminal vesicle, ejaculatory duct, and urethral tissues are designed.

Existing mammalian GnRH analogs are designed for activity at the pituitary GnRH receptor and with extended stability in the circulation of individuals. Yet, the existing data indicate that the human sperm, testicular, scrotal, seminiferous tubule, Leydig cell, Sertoli cell, epididymis, vas deferentia, prostate, seminal vesicle, ejaculatory duct, and urethral tissues have a high affinity GnRH receptor which differs from that in the pituitary. In addition, the degradation of GnRH is different in the human sperm, testicules, scrotum, seminiferous tubule, Leydig cell, Sertoli cell, epididymis, vas deferentia, prostate, seminal vesicle, ejaculatory duct, and urethra. Therefore, prior known pituitary mammalian GnRH analogs have not been designed for use at extra-pituitary sites, and potent non-mammalian GnRH analogs have not previously been designed for use at extra-pituitary sites. The present invention provides potent non-mammalian GnRH analogs.

Non-mammalian analogs of GnRH were synthesized by order. They were specifically designed to prevent degradation of the analog in extra-pituitary tissues as well as in the male and female reproductive system tissue. This allows for the maintenance of sufficient concentrations of analog to remain active when administered via the individual and to reach the extra-pituitary and male and female reproductive system tissue. Due to the particular specificity of the human sperm, testicular, scrotal, seminiferous tubule, Leydig cell, Sertoli cell, epididymis, vas deferentia, prostate, seminal vesicle, ejaculatory duct, and urethral receptor and specific peptidase in blood, seminal fluid, vaginal fluid and human sperm, testicular, scrotal, seminiferous tubule, Leydig cell, Sertoli cell, epididymis, vas deferentia, prostate, seminal vesicle, ejaculatory duct, and urethral tissue, the particular non-mammalian GnRH analogs of the invention were designed. Analogs of the salmon (SEQ ID NO: 4) and chicken II GnRH (SEQ ID NO: 2) sequences, that both show greater affinity for the human sperm, testicular, scrotal, seminiferous tubule, Leydig cell, Sertoli cell, epididymis, vas deferentia, prostate, seminal vesicle, ejaculatory duct, and urethral receptor than for the pituitary receptor, were modified to the tenth amino acid to ethylamide or aza-Gly¹⁰-NH₂ analog to make them resistant to degradation in the circulation and by post-proline peptidases. The chicken II GnRH sequence (SEQ ID NO: 6) and the salmon GnRH sequence (SEQ ID NO: 7) were also modified at the 6 position using D-Arg, making them resistant to degradation by the endopeptidase in blood, and were modified at the 10 position making them stable in blood, seminal fluid, vaginal fluid and the human sperm, testicular, scrotal, seminiferous tubule, Leydig cell, Sertoli cell, epididymis, vas deferentia, prostate, seminal vesicle, ejaculatory duct, and urethral tissues. These analogs are expected to have increased binding to the human sperm, testicular, scrotal, seminiferous tubule, Leydig cell, Sertoli cell, epididymis, vas deferentia, prostate, seminal vesicle, ejaculatory duct, and urethral receptor and increased metabolic stability.

### EXAMPLE XVII - Receptor Binding Activity in the Male Reproductive System Receptor Studies

The receptor binding activity of the different non-mammalian GnRH analogs of the present invention were compared. There is a human sperm, testicular, scrotal, seminiferous tubule, Leydig cell, Sertoli cell, epididymis, vas deferentia, prostate, seminal vesicle, ejaculatory duct, and urethral GnRH receptor which is distinct from that at the pituitary. Prior mammalian GnRH analogs have been designed to increase activity at the pituitary GnRH receptor and stability in the circulation of individuals. These analogs do not demonstrate potent binding activity at the human sperm, testicular, scrotal, seminiferous tubule, Leydig cell, Sertoli cell, epididymis, vas deferentia, prostate, seminal vesicle, ejaculatory duct, or urethral receptor as they do at the pituitary receptor. The non-mammalian GnRH analogs of the present invention have been designed to interact with preference at the human sperm, testicular, scrotal, seminiferous tubule, Leydig cell, Sertoli cell, epididymis, vas deferentia, prostate, seminal vesicle, ejaculatory duct, and urethral receptor and not the pituitary receptor. They have also been designed to limit degradation by the human sperm, testicular, scrotal, seminiferous tubule, Leydig cell, Sertoli cell, epididymis, vas deferentia, prostate, seminal vesicle, ejaculatory duct, and urethral enzymes present in blood, seminal fluid, and vaginal fluid. Binding activity of the newly synthesized non-mammalian GnRH analogs have been compared to that for existing pituitary-active analogs of mammalian GnRH.

### Example XVIII - Stability Studies of GnRH Analogs in the Male Reproductive System

The present example demonstrated the utility of using the present invention in controlling and modulating the activity of the human sperm, testicles, scrotum, seminiferous tubule, Leydig cells, Sertoli cells, epididymis, vas deferentia, prostate gland, seminal vesicle, ejaculatory duct, and urethra of the mammal. The present non-mammalian GnRH analogs had not previously been examined for receptor binding. However, the added stability of these non-mammalian GnRH analogs would effect a substantial increase in bioactivity alone. Thus, both stability and binding studies were performed. Currently available mammalian GnRH analogs have not been designed to be resistant to degradation by this peptidase. Non-mammalian GnRH analogs were designed with these specific criteria in mind. The stability of these non-mammalian GnRH analogs to the enzymatic activity of peptidase was examined. In addition, the ability of the analogs to competitively inhibit the degradation of mammalian GnRH by peptidase was studied.

### EXAMPLE XIX - Biological Activity Studies of GnRH Analogs in the Male Reproductive System

The testosterone inhibiting activity of the present non-mammalian GnRH analogs was studied using an in vitro human explant system. Initial findings indicate the inhibition of this activity. These studies are ongoing in an effort to obtain conclusive data that the present non-mammalian GnRH analogs can be used to regulate testosterone levels in a mammal.

### EXAMPLE XX - Comparison Of GnRH And Its Synthetic And Naturally Occurring Analogs For Binding Action in The Human Male Reproductive System Receptor

The human male reproductive system GnRH receptor shows different kinetic constants for GnRH compared to that of the pituitary receptor. Studies were designed to compare the human male reproductive system receptor activity for numerous synthetic and naturally occurring analogs. These studies are still being conducted.

### EXAMPLE XXI - Non-mammalian GnRH Analogs and Male Contraception

The present example demonstrates the utility of the present invention for use as a contraceptive preparation.

By way of example, the non-mammalian GnRH analogs defined here, and conservative variants thereof, may be formulated into a pharmaceutically acceptable preparation, and then administered to a male mammal during the 24 hours prior to coitus. Relatively high doses of about 0.1 gram to about 10 grams of the non-mammalian GnRH analog could be given daily.

For purposes of practicing the present invention as an oligonucleotide in molecular biology applications, the non-mammalian GnRH analogs of chicken II (SEQ ID NO: 1) and salmon decapeptide GnRH analog cDNA sequences (SEQ ID NO: 3) would be employed. The textbook of Sambrook, et al (1989) *Molecular Cloning, A Laboratory Manual,* 2d Ed., Cold Springs Harbor Laboratory, Cold Springs Harbor, N.Y., is specifically incorporated herein by reference for this purpose. By way of example, the cDNA sequence for the non-mammalian GnRH of SEQ ID NO: 1 (chicken II GnRH) or SEQ ID NO:3, (salmon GnRH) may be prepared as part of a suitable vector, such as in an adenovirus or retroviral vector, and administered to the animal. Once the sequence is incorporated into the cell, the peptide product will be translated and peptide supplied. Because this method of treatment would not require that the peptide travel in the blood circulation in order to reach the site of action, there would be no requirement that the analog possess enzyme degradation resistance. This mode of treatment has not thus far been proposed, and hence the use of such a method in the regulation of male fertility is a novel clinical regimen.

### EXAMPLE XXII - Antibodies Specific for Non-Mammalian GnRH in the Male Reproductive System

The present example demonstrates the utility for using the present invention non-mammalian GnRH analog decapeptides to prepare antibodies that preferentially bind the GnRH peptide sequences, or that bind the human sperm, testicular, scrotal, seminiferous tubule, Leydig cell, Sertoli cell, epididymis, vas deferentia, prostate, seminal vesicle, ejaculatory duct, or urethral GnRH receptor or any other non-pituitary GnRH peptide or protein, or the receptors therefor. It is anticipated that these non-mammalian GnRH analog antibodies may be used in a variety of screening assays. For example, these antibodies may be used to determine levels of GnRH, or the GnRH receptor, present in a sample as an indicator molecule. The antibodies to non-mammalian GnRH may be monoclonal or polyclonal antibodies.

Polyclonal antibodies may be created by standard immunization techniques, wherein the immunogen used will be the non-mammalian chicken-II GnRH analog (SEQ ID NO: 2) or the salmon GnRH analog (SEQ ID NO: 4) decapeptide described herein. These peptides may be used either alone or together in a pharmaceutically acceptable adjuvant. The animal, such as a rabbit, would be administered several doses of the decapeptide preparation, and the levels of the animal=s antibody blood levels monitored until an acceptable antibody level (titer) had been reached.

For the preparation of monoclonal antibodies, one would follow standard techniques for the immunization of an animal, again using the decapeptide non-mammalian GnRH peptide. Once sufficiently high acceptable antibodies are reached (titer) in the animal, the spleen of the animal would be harvested, and then fused with an immortalized cell line, such as a cancer cell line, to produce a population of hybridoma cells. This hybridoma population of cells would then be screened for those that produce the highest amount of antibody that specifically bind the non-mammalian GnRH analog decapeptide. Such hybridoma cells would be selected, and then cultured. The antibody to non-mammalian GnRH would then be collected from the media of the cell culture using techniques well know to those of skill in the art.

For purposes of the practice of preparing polyclonal and monoclonal antibody, the textbook Sambrook et al (1989) *Molecular Cloning, A Laboratory Manual,* 2^{nd} Ed., Cold Springs Harbor Laboratory, Cold Springs Harbor, N.Y., is specifically incorporated herein by reference. All of the compositions and methods disclosed and claimed herein can be made and executed without undue experimentation in light of the present disclosure.

### EXAMPLE XXIII- Non-Mammalian GnRH Analogs and Methods of Use in Treatment of Conditions of the Male Reproductive System

Due to the stability of the non-mammalian GnRH analogs, particularly chicken II GnRH (SEQ ID NO: 2) and salmon analogs (SEQ ID NO: 4), in the blood and reproductive tissues, the presence of binding receptors in reproductive tissues, and their biological activity in reproductive tissues, such analogs can be used in the treatment of conditions of or regulation of the reproductive system and the tissues therein including, but not limited to the testicles, scrotum, seminiferous tubule, Leydig cells, Sertoli cells, epididymis, vas deferentia, prostate gland, seminal vesicle, ejaculatory duct, and urethra.

Conventional methods, known to those of ordinary skill in the art of medicine, can be used to administer the pharmaceutical formulation(s) to the patient. Typically, the pharmaceutical formulation will be administered to the patient by intramuscular injection, subdermal pellet, or nasal spray. The pharmaceutical formulation(s) can also be administered via other conventional routes (e.g., oral, subcutaneous, intrapulmonary, transmucosal, intraperitoneal, sublingual, or intrathecal routes) by using standard methods. In addition, the pharmaceutical formulations can be administered to the patient via injection depot routes of administration such as by using 1-, 3-, or 6-month depot injectable or biodegradable materials and methods.

Regardless of the route of administration, the therapeutical agent typically is administered at a daily dosage of 0.001µg to 30 mg/kg of body weight of the patient. The pharmaceutical formulation can be administered in multiple doses per day, if desired, to achieve the total desired daily dose or as a long acting depot. The effectiveness of the method of treatment can be assessed by monitoring the patient for known signs or symptoms of the disorder.

### EXAMPLE XXIV - Localization of Non-Mammalian GnRH in Tissues of the Reproductive System

Tissue of the male and female reproductive system were examined for the presence of non-mammalian GnRH in their cells. The localization of non-mammalian GnRH in the testis, seminal vesicle, epididymis, ovarian, uterus and placental tissues of a mammal has not been previously described. Their localization in reproductive tissues of mammals of non-mammalian GnRH isoforms demonstrates the non-mammalian GnRH is produced and/ or acts in these reproductive tissues.

Human tissues from the ovary, testis, uterus, epididymis, seminal vesicle or placenta were fixed, sectioned, and plated by sections on glass slides. The human tissues on the glass slides were incubated with anti-GnRH II (1/100) for 1 hour at RT. The tissues were then washed with phosphate buffered saline and anti-rabbit gamma globulin conjugated with biotin and incubated for 4 minutes at 55 C. The slides were rinsed in buffer followed by blocking of the endogenous peroxidase activity. Then streptavidin horse radish peroxidase was added and incubated for 4 minutes at 55 C. Stable diaminobenzidine (5 minutes at 55 C) was used to generate the signal. The slides were rinsed, mounted and read. The presence of non-mammalian GnRH was localized via the DAB (diaminobenzidine) using microscopy. In each of these reproductive tissues examined, the testis, seminal vesicle (See Figure 36), epididymis (See Figure 37), ovarian, uterus and placental tissues, non-mammalian GnRH was visualized. Tissues such as atrium and liver were negative.

### EXAMPLE XXV - Contraceptive Activity of Non-Mammalian GnRH Analog in Vivo

Mammalian GnRH (SEQ ID NO: 5) was previously thought to be the only isoform of GnRH expressed in mammals, but chicken II GnRH (SEQ ID NO: 6) has now been identified in numerous mammalian tissues, including the ovary, uterus, placenta and brain. Specific, high affinity receptors, which bind chicken II GnRH (SEQ ID NO: 6) and its analogs, have been identified throughout the reproductive tract. Studies using ovarian tissues or placental explants in vitro have shown that chicken II GnRH (SEQ ID NO: 6) can regulate progesterone and hCG production. Thus, we hypothesized that chicken II GnRH (SEQ ID NO: 6) acts as a paracrine factor to regulate extra-hypothalamic tissue functions, and when delivered chronically chicken II GnRH (SEQ ID NO: 6) would be an effective contraceptive agent.

In these studies we examined the effect of a stable analog of chicken II GnRH on ovarian steroidogenesis, implantation and maintainence of pregnancy in the rhesus monkey. We administered this chicken II GnRH analog (SEQ ID NO: 2) or saline via osmotic mini-pumps from Day 1-6, Day 6-11 or Day 11-17 to regularly cycling monkeys mated with fertile males around the time of ovulation. Circulating estradiol (E₂) and progesterone (P) in the luteal phase was observed as well as cycle length in this and subsequent cycle. Implantation and pregnancy were confirmed by circulating CG and continued progesterone production. This chicken II GnRH analog (SEQ ID NO: 2) had no significant effect on progesterone production or cycle length when administered on day 1―6. See Figure 32. In these animals no pregnancies resulted, but in the saline treated controls five of eight animals (62.5%) became pregnant. In animals treated on day 6―11 or days 11―17, no effect on luteinization or cycle length was noted; and two of five (40.0%), and one of three animals (33.3%), respectively, implanted with normal pregnancies resulting. See Figures 33 and 34. Therefore, treatment with chicken II GnRH analog (SEQ ID NO: 2) after Day 6 did not alter cycle length or pregnancy rate. See Figure 35.

These data demonstrate that this chicken II GnRH analog (SEQ ID NO: 2) can act as a contraceptive agent when administered around the time of ovulation or during early luteal development. These findings suggest that GnRH may play a role in reproductive tract physiology and that chicken II GnRH analogs (SEQ ID NO: 2) may serve as effective, non-steroidal, post-coital contraceptives.

### EXAMPLE XXVI - Sperm Motility Studies of GnRH Analogs in vitro

The present example demonstrates the utility of using the present non-mammalian GnrH analog decapeptides to effect the motility of sperm. Human sperm motility is an important factor for normal sperm function. Sperm motility is graded as a percent of motile sperm. The grade of sperm motility ranges from 0-4 with 4 being the most active in forward movement. It is anticipated that the present chicken II GnRH analogs (SEQ ID NO: 2) will effect sperm motility. This is anticipated because we know that chicken II GnRH (SEQ ID NO: 6) is produced in the male reproductive tract and sperm have a receptor for chicken II GnRH (SEQ ID NO: 6).

Normal human sperm (20,000 million/ml) were obtained with seminal plasma and the motility of the sperm was assessed microscopically. Both the fraction of motile sperm and the forward sperm motion were assessed. These parameters were measured just prior to a 1:5 dilution with phosphate buffered saline containing 0 to 200 nM chicken II GnRH analog (SEQ ID NO: 2). The motility and motion of the sperm were assessed at 2, 30, 60, 90, 120 and 180 minutes after incubation at 37 C. The motility was initially inhibited with 20 to 200 nM chicken II GnRH analog (SEQ ID NO: 2); inhibition took 30 minutes using 2 nM of this analog. By 180 minutes the motility of the control had fallen to that of all the treated sperm. The motile sperm that remained after incubation with chicken II GnRH analog (SEQ ID NO: 2) maintained a high grade for forward motion of the sperm.

### EXAMPLE XXVII - Tumor Receptor Binding of GnRH Isoforms and Analogs

The tumor receptor binding activity of the different non-mammalian GnRH and its analogs of the present invention was compared. Prior mammalian GnRH analogs have been designed to increase activity at the pituitary GnRH receptor and stability in the circulation of nonpregnant individuals. Thesemammalian GnRH isoforms or their analogs do not demonstrate as potent binding activity at the tumor receptor as they do at the pituitary receptor. In contrast and as was mentioned earlier, the non-mammalian GnRH analogs of the present invention have been designed to interact with preference at the tumor receptor and not the pituitary receptor.

GnRH receptors on the cells of MCF-7 breast cancer cells were studied. The cells were plated on 96 wells and grown to confluency in base medium (M3D:Fetal Bovine Serum [9:1]). Prior to the experiment the cells were down shifted to M3D:Fetal Bovine Serum [99:1] and then to serum-free medium. Cells were incubated for 24 hours at room temperature with mammalian GnRH, Buserilin, Leuprolide, Chicken II GnRH, and D-Arg(6) Chicken II-aza-Gly(10)-amide (SEQ ID NO: 2). Cells were then collected and studied for receptor binding and receptor number with D-Arg(6) Chicken II-aza-Gly(10)-amide (SEQ ID NO: 2). Addition of enzyme inhibitors of the endogenous post-proline peptidase and other peptidases were used as well as agents for receptor stabilization. Receptor bound label was separated by centrifugation. The binding of the non-mammalian GnRH analog and its ability to regulate the tumor cells= GnRH receptor was compared. Figure 41 shows the binding of I¹²⁵ -D-Arg(6)-Chicken II GnRH-aza-Gly (10) - amide to MCF-7 breast cancer cells after 24 hours of incubation with no exogenous GnRH or competing isoforms or analogs of GnRH. D-Arg(6) -Chicken II GnRH-aza-Gly(10)-amide (SEQ ID NO: 2) specifically bound the MCF-7 breast cancer cells. This binding was competitively inhibited by D-Arg(6)-Chicken II GnRH-aza-Gly(10)-amide (SEQ ID NO: 2) with the greatest potency. This was followed by Buserilin. Mammalian GnRH was the weakest competitor, while Chicken II GnRH was highly potent even though it is not protected from degradation either at the 6 or the 10 position. This indicated the high innate affinity of this isoform of GnRH for the tumor GnRH receptor. It is believed by Applicants that the same or similar results could be obtained using other non-mammalian GnRH isomers or analogs with similar amino acid structure as Chicken II GnRH such as but not limited to Salmon GnRH (SEQ ID NO: 4), Herring GnRH (SEQ ID NO: 16), Catfish GnRH, or Dogfish GnRH.

### EXAMPLE XXVIII - Tumor Tissue Stability Studies for GnRH Isoforms and Analogs

As has been previously mentioned, mammalian GnRH and its analogs bind with weak affinity to tumor cell receptors in certain tumor tissues whereas the non-mammalian GnRH and its analogs exhibit strong affinity for these receptors. Observing this strong affinity it became necessary to examine the non-mammalian GnRH analogs for stability. The non-mammalian GnRH and its analogs of the present invention have not previously been examined for stability. However, the added stability of these non-mammalian isoforms and analogs would effect a substantial increase in bioactivity. Thus, stability studies involving endopeptidase and post-proline peptidase were performed for the non-mammalian GnRH analogs.

### Endopeptidase Stability Studies:

Since human pituitary and blood contain an enzymatic activity that degrades GnRH at the 5-6 position, rather than the 9 position, present non-mammalian GnRH analogs have been designed to inhibit the former enzymatic activities and have substitutions in the 5-6 position of the molecule. The present analogs with these substitutions are therefore resistant to degradation at the pituitary or in the blood of non-pregnant individuals. However, these substitutions alone do not protect the analogs from degradation at the tumor tissues, which contain post-proline peptidase. Substitution of the Gly(10)-NH₂ with ethylamide, or the more potent aza-Gly(10)-NH₂, inhibits degradation by post-proline peptidases. A number of existing mammalian GnRH analogs also have a substitution of Gly(10)-NH₂.

Post-Proline Peptidase Stability Studies: As mentioned earlier, the post-proline peptidase is important in actively degrading peptides that contain a proline residue. GnRH is such a peptide. Initially the enzymatic activity of the tumor cell was studied. Tumor tissue cells and their spent media were studied for enzyme activity. In particular, examination was made for the degradation of GnRH both with and without specific post-proline and endopeptidase activity inhibitors to determine the specificity of the tumor enzymatic activity. These studies have demonstrated very high post-proline peptidase activity produced by the tumor tissue.

The enzymatic degradation of the non-mammalian GnRH analogs (SEQ ID NO: 2 and SEQ ID NO: 4) were studied in MCF-7 breast cancer cells using an enzymatic activity assay and compared to that for the purified chorionic post-proline peptidase. Chorionic post-proline peptidase is a peptidase with high specificity for the degradation of GnRH at the proline-glycine bond, but can also degrade other GnRH species containing this bond.

In a non-pregnant individual very little post-proline peptidase activity is present in the blood or the pituitary. Thus, currently available mammalian GnRH analogs have not been designed to be resistant to degradation by this activity. However, due to the high post-proline peptidase activity present in tumor tissue, the non-mammalian GnRH analogs (SEQ ID NO: 2, SEQ ID NO: 4, and SEQ ID NO: 16) for cancer therapy described herein were designed to be resistant to this type of degradation. The stability of these non-mammalian GnRH analogs (SEQ ID NO: 2, SEQ ID NO: 4, and SEQ ID NO: 16) in the presence of post-proline homogenates was examined and compared to existing mammalian GnRH analogs. In addition, the ability of the analogs (SEQ ID NO: 2, SEQ ID NO: 4, and SEQ ID NO: 16) to competitively inhibit the degradation of GnRH using chorionic post-proline peptidase was studied.

The stability of most potent receptor-active non-mammalian GnRH analogs (SEQ ID NO: 2, SEQ ID NO: 4, and SEQ ID NO: 16) in the presence of tumor tissue cells, spent media, or tumor tissue cells homogenates was identified. Using the incubation system developed for chorionic post-proline peptidase activity, the degradation of GnRH was tested. Each of these analogs (SEQ ID NO: 2, SEQ ID NO: 4, and SEQ ID NO: 16) were first studied for their ability to act as a competitive inhibitor of GnRH for chorionic post-proline peptidase activity using the enzymatic activity assay as described previously (103). In this assay, incubation of enzyme and mammalian GnRH with and without the chosen newly synthesized non-mammalian GnRH analog (SEQ ID NO: 2, SEQ ID NO: 4, and SEQ ID NO: 16) was studied. The reaction was stopped by heating at 85EC for 10 minutes. The remaining mammalian GnRH substrate was quantified by radioimmunoassay. The product formed, i.e. the N-terminal nonapeptide of GnRH, was calculated by subtraction, and its inverse plotted against the inverse of the original substrate concentrations to determine the Ks of the competition. The Ki was determined by plotting the inverse of the product that formed versus the inhibitor used. The inhibitory activity of Antide, Im-btl-D-His(6)-mammalian-GnRH-ethylamide, D-Trp(6)-GnRH-ethylamide, Buserilin, Leuprolide, OH-Pro(9)-Mammalian GnRH, Mammalian GnRH-ethylamide, Chicken II GnRH, Chicken II-ethylamide, D-Arg(6)-Chicken II-ethylamide (SEQ ID NO: 2), D-Arg(6)-Chicken II-aza-Gly(10)-amide (SEQ ID NO: 2), Chicken I GnRH, Salmon GnRH, D-Arg(6)-Salmon GnRH-aza-Gly(10)-amide (SEQ ID NO: 4), and Lamprey GnRH was studied.

Mammalian GnRH was actively degraded by chorionic post-proline peptidase. While replacement of Gly(10)-NH₂ with ethylamide made each of the mammalian GnRH analogs more resistant to degradation than mammalian GnRH alone, some of these Mammalian GnRH were still degraded by the post-proline peptidase. Of four mammalian GnRH ethylamides studied, des-Gly(10)-GnRH ethylamide, des-Gly(10), D-Trp(6)-GnRH ethylamide, des-Gly(10)-D-Leu(6)-GnRH ethylamide, and Buserilin, each competitively inhibited the degradation of mammalian GnRH; thus they were degraded by the post-proline peptidase. The effect of des-Gly(10) GnRH on the degradation of mammalian GnRH by chorionic post-proline peptidase is shown in Figure 39. The less an analog is capable of competing with the GnRH for the post-proline peptidase, the more resistant it is to degradation by post-proline peptidase and the more stable the analog will be in the tumor tissue and/or in the blood. Thus the existing mammalian GnRH analogs commonly used in medicine can be degraded in tumor tissues.

This activity of chorionic post-proline peptidase was inhibited by OH-Pro(9)-GnRH, Lamprey GnRH, Chicken I GnRH, Antide, Chicken II GnRH, and Salmon GnRH with a relative potency of 1.5, 1.5, 0.6, 0.6,0.2, and 0.2, respectively, compared to that for GnRH. In viewing this data, the OH-Pro(9)-GnRH and Lamprey GnRH were determined to be the best competitors for GnRH degradation by chorionic post-proline peptidase. They are as or even more potent than mammalian GnRH. Antide and Chicken I GnRH are three fold less potent than GnRH, but two fold more potent than the Salmon GnRH or Chicken II GnRH. The addition of the ethylamide to mammalian GnRH, both with and without the D-Trp(6)-, D-Phe(6) substitution, decreased the competition with mammalian GnRH for chorionic post-proline peptidase degradation, but not as markedly as did the Im-btl-D-His(6) or Chicken II GnRH analogs. Both D-Arg(6)-Chicken II GnRH-aza-Gly(10)- amide (SEQ ID NO: 2) and Im-btl-D-His(6)-GnRH-ethylamide were essentially inactive, i.e. <0.005 inhibitory activity for GnRH. Essentially these latter two GnRH=s were greater than 200 fold less active in the inhibition of GnRH degradation by chorionic post-proline peptidase. Thus these analogs appear to be very stable in the presence of post proline peptidase activity, however the Im-btl-His(6) analog has reduced receptor potency. The stability of the D-Arg(6)-Chicken II GnRH-aza-Gly(10)-amide (SEQ ID NO: 2) was found to not only be greater than 200 fold more stable than GnRH but it still has increased receptor potency. The action of D-Arg(6)-Chicken II GnRH-aza-Gly(10)- amide (SEQ ID NO: 2) on the degradation of mammalian GnRH by chorionic post-proline peptidase is shown in Figure 40. It is believed by Applicants that the same or similar results could be obtained using non-mammalian GnRH isomers or analogs with similar amino acid structure as Chicken II GnRH such as but not limited to Herring GnRH, Dogfish GnRH, or Catfish GnRH.

Since chorionic post-proline peptidase is a peptidase with high specificity for the degradation of GnRH at the proline-glycine peptide bond it can also degrade other GnRH species containing the same bond. The synthetic mammalian GnRH analogs such as Antide are degraded with reduced activity while other analogs such as D-Arg(6)-Chicken II GnRH-aza-Gly(10)-amide (SEQ ID NO: 2) are resistant to degradation by this endogenous chorionic enzyme. Such a resistant analog can be useful in the regulation of tumor tissue GnRH activity.

Degradation of mammalian GnRH by the tumor tissue cells was essentially 100% after overnight incubation. Specific inhibitors of post-proline peptidase were used to demonstrate this activity in the tumor cell extracts. The degradation of mammalian GnRH was inhibited by bacitracin, but not EDTA, demonstrating the enzyme similarity to chorionic post proline peptidase. From this study it was found that the aza-Gly(10)amide derivatives of Chicken II GnRH and Salmon GnRH have little if any degradation as compared to mammalian GnRH. Each Chicken II and its analogs were more stable than the mammalian GnRH analogs analyzed. It is believed by Applicants that the same or similar results could be obtained using non-mammalian GnRH isomers or analogs with similar amino acid structure as Chicken II GnRH such as but not limited to Herring GnRH, Dogfish GnRH, or Catfish GnRH.

Although the enzyme competition system had already been developed, newly synthesized non-mammalian GnRH analogs have not been utilized in this system. Previous data generated by Applicants have demonstrated that the antiserum is specific for mammalian GnRH, thus reducing potential for cross-reaction of non-mammalian GnRH isoforms or its analogs in the assay used in these studies.

### EXAMPLE XXIX - Biological Activity Studies

The tumor growth inhibiting activity of the non-mammalian GnRH and its analogs was studied. Such data can be used to determine biological activity including regulation of tumor cell growth, tumor proliferation, and tumor regression. Bio-potency was studied by determining cell death and tumor regression. Thus a primary parameter of interest was indicating the cell viability in the tumor cells being regulated by the exogenous GnRH activities, which were studied.

The biological activity of the newly synthesized non-mammalian GnRH analogs (SEQ ID NO: 2, SEQ ID NO: 4, and SEQ ID NO: 16) was studied using an in vitro human tumor cell culture system. This system allows for replicated extended activity studies. Mammalian GnRH action on the tumor tissue cell has been studied using a similar system. Applicants studied replicate cultures, thus allowing for comparison of different doses of each non-mammalian GnRH analog (SEQ ID NO: 2, SEQ 1D NO: 4, and SEQ ID NO: 16) to mammalian GnRH. In these studies, the action of the most stable and receptor-active non-mammalian GnRH analogs (SEQ ID NO: 2, SEQ ID NO: 4, and SEQ ID NO: 16) on tumor cell viability were determined.

The bio-potency studies were done with a MCF-7 breast cancer cell culture system and the cell viability as a measure of survival was assessed using the Alamar Blue assay. The percent difference in the Alamar Blue optical density (OD) readings at 570 and 600 nm in the treated and untreated controls was determined. These studies were done using mammalian GnRH, chicken II GnRH, Leuprolide, Buserelin, the D-Arg(6)-Chicken II GnRH-aza-Gly(10)-amide analog (SEQ ID NO: 2) as well as the D-Leu(6)-Chicken II GnRH-aza-Gly(10)-amide analog. A dose-response study in quadruplicate cultures was performed. Cell viability was assessed after 24 and 48 hours of incubation with the activity agent. The data analysis of these tumor cell culture sets at 24 hours is shown in Figure 42. More specifically, Figure 5 illustrates the anti-proliferative, tumor regression activity of D-Arg(6)-Chicken II GnRH -aza-Gly(10)-amide (SEQ ID NO: 2) as compared to controls and other isoforms and analogs of GnRH after 24 hours of incubation. In this Figure 5, A1 is medium 199 (no vehicle); A2 is medium 199 (with vehicle); B1-B3 is Leuprolide; C1-C3 is mammalian GnRH; D1-D3 is Chicken II GnRH; E1-E3 is D-Arg(6)-Chicken II GnRH-aza-Gly(10) amide (SEQ ID NO: 2); G1-G3 is Buserelin.

After 24 hours of incubation, an inhibition of cell proliferation was observed with the Chicken II GnRH and its analogs, while even the highest doses of mammalian GnRH analogs, Leuprolide, and Buserelin were totally inactive (See Figure 42). The lowest dose of Chicken II studied (10⁻⁸ M) was more effective than 10⁻⁵ M mammalian GnRH. The D-Arg(6)-Chicken II GnRH-aza-Gly(10)-amide (SEQ ID NO: 2) resulted in positive dose-related activity, which was markedly active at 10⁻⁵ M. After 48 hours of incubation this analog was equally as potent as at 24 hours, while its natural isoform lost potency due to degradation. The mammalian GnRH and its analogs were totally ineffective in the inhibition of the MCF-7 breast cancer cell proliferation after 48 hours of continued exposure. These data demonstrate that D-Arg(6)-Chicken II GnRH-aza-Gly(10)-amide analog (SEQ ID NO: 2) is a very stable and bioactive molecule in the regulation of tumor cell growth in the human MCF-7 breast cancer cells. It is believed by Applicants that the same or similar results could be obtained using non-mammalian GnRH isomers or analogs with similar amino acid structure to Chicken II GnRH such as but not limited to Salmon GnRH, Herring GnRH, Dogfish GnRH, or Catfish GnRH.

Using an in vitro system to define bio-potency is expected to be predictive of in vivo activity. In addition to tumor cell action, since these newly synthesized non-mammalian GnRH analogs are known to act directly at the placenta to inhibit steroidogenesis, these analogs are expected to be active at the ovarian level to inhibit steroidogenesis. This would act as an added benefit in the cancer therapy.

### EXAMPLE XXX - Methods for Regulating Tumor Cell Growth and Proliferation In Vivo

In vivo trials utilizing the non-mammalian GnRH or its analogs (SEQ ID NO: 2, SEQ ID NO: 4, SEQ ID NO: 6 and SEQ ID NO: 7) the present invention may be performed to inhibit tumor cell growth and proliferation to thus induce regression of cancer cells in a mammal. The mammal can include a human with cancer. As a proposed dose regimen, it is anticipated that a human between 100 lbs. and 250 lbs. be administered about 10 nanograms to 1.0 gram of a chicken II GnRH analog (SEQ ID NO: 2), salmon GnRH analog (SEQ ID NO: 4) or long-acting chicken II GnRH (SEQ ID NO: 6) or salmon GnRH (SEQ ID NO: 7), or long-acting other non-mammalian GnRH or its analog with similar amino acid structure. This would be expected to be effective for inhibiting tumor growth or metastasis in the mammal once administered.

It is envisioned that these non-mammalian GnRH (long-acting chicken II GnRH (SEQ ID NO: 6) or salmon GnRH (SEQ ID NO: 7), or long-acting other non-mammalian GnRH) or its analogs (SEQ ID NO: 2, SEQ ID NO: 4, and SEQ ID NO: 16) will be administered intra-nasally, orally, intramuscularly, transdermally or vaginally, or directly to the tumor. However, virtually any mode of administration may be used in the practice of the invention. Treatment with these analogs may require short-term, repeated administrations of the active non-mammalian GnRH (long-acting chicken II GnRH (SEQ ID NO: 6) or salmon GnRH (SEQ ID NO: 7), or long-acting other non-mammalian GnRH) or analog (SEQ ID NO: 2, SEQ ID NO: 4, and SEQ ID NO: 16) or long-term continuous therapy until tumor regression has occurred. Repeated administration could be used as needed.

Numerous in vitro fertilization (IVF) protocols now routinely use mammalian GnRH analogs for ovulation timing and have been shown to be nontoxic, even after weeks of administration. Long-term therapies with mammalian GnRH analogs have been used for treatment of endometrious, prostate cancer and other cancers and have been shown to be nontoxic, even after months of administration. Long-term therapies with mammalian GnRH analogs have been associated with a hypoestrogenic state, which is frequently a desired condition in certain cancer therapies. The effect on the pituitary GnRH receptor is expected to be minimal with these non-mammalian GnRH analogs (SEQ ID NO: 2, SEQ ID NO: 4, and SEQ ID NO:16) and with this short duration of treatment. Thus, the specific receptor activity of these analogs makes it less likely to interfere with normal physiology.

In some trials, the dosing regimen can comprise a pulsatile administration of GnRH II or its analog over a 24-hour period, wherein the daily dosage is administered in relatively equal 1/24th fractions. For example, where the daily dose is about 2.4 micrograms, the patient would be administered about 0.1 micrograms per hour over a 24-hour period. Such a daily pulsatile administration would create a hormonal environment in the patient sufficient to inhibit tumor cell growth and proliferation and/or induce its regression. The particular pharmaceutical preparations may be created by one of skill in the pharmaceutical arts. Remington's Pharmaceutical Sciences Remington: The Science and Practice of Pharmacy, 19" edition, Vol. 102, A. R. Gennaro, ed., Mack Publishing co. Easton, PA. (1995), is specifically incorporated herein by reference for this purpose.

### EXAMPLE XXXI - ANTIBODIES SPECIFIC FOR NON-MAMMALIAN GnRH

Another embodiment of the present invention is to utilize non-mammalian GnRH or its analogs to prepare antibodies that preferentially bind the non-mammalian GnRH peptide sequences, or that bind to reproduction tissues, tumor tissues or any other non-pituitary GnRH peptide or protein. It is anticipated that these non-mammalian GnRH antibodies may be used in a variety of screening assays. For example, these antibodies may be used to determine levels of non-mammalian GnRH are present in a sample as an indicator molecule. The levels of such GnRH may be used to monitor and follow a patient's tumor activity or growth, as well as an indicator of the tumor=s presence, to monitor reproductive function. The antibodies to non-mammalian GnRH may be monoclonal or polyclonal antibodies.

Polyclonal antibodies may be created by standard immunization techniques, wherein the immunogen used will be the non-mammalian chicken II GnRH, salmon GnRH, herring GnRH analog, or the naturally occurring decapeptide of any of these described herein, or any other non-mammalian GnRH analog with similar amino acid structure. These peptides may be used either alone or together in a pharmaceutically acceptable adjuvant. The animal, such as a rabbit, would be administered several doses of the decapeptide preparation, and the levels of the animal's antibody blood levels monitored until an acceptable antibody level (titer) had been reached.

For the preparation of monoclonal antibodies, one would follow standard techniques for the immunization of an animal, again using the decapeptide non-mammalian GnRH peptide or its analog. Once sufficiently high acceptable antibodies are reached (titer) in the animal, the spleen of the animal would be harvested and then fused with an immortalized cell line, such as a cancer cell line, to produce a population of hybridoma cells. This hybridoma population of cells would then be screened for those cells that produce the highest amount of antibody that specifically binds the non-mammalian GnRH analog decapeptide. Such hybridoma cells would be selected, and then cultured. The antibody to non-mammalian GnRH would then be collected from the media of the cell culture using techniques well known to those of skill in the art.

For purposes of the practice of preparing polyclonal and monoclonal antibody, the textbook Sambrook et al (1989) *Molecular Cloning, A Laboratory Manual,* @d Ed., Cold Springs Harbor Laboratory, Cold Springs Harbor, N.Y., is specifically incorporated herein by reference.

It is further believed by Applicants that the non-mammalian GnRH or its analogs (SEQ ID NO: 2, 6 and SEQ ID NO: 4, 7) disclosed can be used in the development of stable, toxin conjugated antibodies or ligands that can specifically bind to the GnRH receptor on the tumor cell and kill the cell.

### EXAMPLES XXXII - REGULATION OF OF NON-MAMMALIAN GnRH OR ITS RECEPTOR EXPRESSION THROUGH CELLULAR MECHANISM OR EXOGENOUS ADMINISTRATION

In addition to the embodiments presented it is believed by Applicants that the disclosed non-mammalian GnRH or its receptor activity, as well as any other gene regulator can be used to regulate the gene expression of non-mammalian GnRH or expression of its receptors in tumor cells. This may be achieved by agents that effect receptor activity, cell signaling, transcription or translation for non-mammalian GnRH or its receptor. Alternatively, exogenous non-mammalian GnRH or its receptor may be administered systemically or to the specific tissue to result in regulation of non-mammalian GnRH or its receptor concentration or activity. The exogenous non-mammalian GnRH or its receptor peptide or analog or their biomimetics may be administered through a pump, or a longacting formulation, locally or systemically and can affect the non-mammalian GnRH activity in the cell or at through its receptor.

### EXAMPLE XXXIII - ANTIBODIES SPECIFIC FOR NON-MAMMALIAN GnRH RECEPTOR

Another embodiment of the present invention is to utilize non-mammalian GnRH receptor peptides to prepare antibodies that preferentially bind the non-mammalian GnRH receptor peptide sequences, or that bind the reproductive tumor tissues or any other non-pituitary GnRH receptor. It is anticipated that these non-mammalian GnRH antibodies may be used in a variety of screening assays. For example, these antibodies may be used to determine levels of non-mammalian GnRH receptors are present in a sample as an indicator molecule. The levels of such non-mammalian GnRH receptors may be used to monitor and follow a patient's reproductive function, tumor activity or growth, as well as an indicator of the tissue presence of non-mammalian GnRH receptors. The antibodies to non-mammalian GnRH receptor may be monoclonal or polyclonal antibodies.

It is further believed by Applicants that the non-mammalian GnRH receptor peptides can be used in the development of stable, toxin conjugated antibodies or ligands that can specifically bind to the GnRH receptor on the tumor cell and kill the cell.

### EXAMPLE XXXIV - DETECTION AND USE OF mRNA OR DNA FOR NON-MAMMALIAN GNRH OR ITS RECEPTOR

In addition to the embodiments presented it is believed by Applicants that the detection of mRNA or DNA for the non-mammalian GnRH or their receptor can be utilized to determine tissue function and/or responsiveness to non-mammalian GnRH its analogs or receptor peptides. Detection of the non-mammalian GnRH or its receptor mRNA or DNA in the reproductive or tumor tissues or any other non-pituitary GnRH receptor may be indicative of particular tissue functions. It is anticipated that methods for the detection of mRNA and DNA of the non-mammalian GnRH or its receptor may be used in a variety of screening assays. For example, methods such as in situ hybridization may be used to determine levels of non-mammalian GnRH or its receptors expressed in a sample as an indicator molecule. The levels of such non-mammalian GnRH or its receptor may be used to monitor and follow a patient's reproductive function, tumor activity or growth, as well as an indicator of the tissue presence of non-mammalian GnRH receptors.

It is further believed by Applicants that probes for the non-mammalian GnRH or receptor mRNA or DNA can be used in the development of stable, toxin conjugated ligands that can specifically bind to non-mammalian GnRH or its receptor on reproductive tissues, on the tumor cell or cells expressing the non-mammalian GnRH or receptor mRNA or DNA and kill the cell.

While the compositions and methods of this invention have been described in terms of preferred embodiments, it will be apparent to those of skill in the art that variations may be applied to the composition, methods and in the steps or in the sequence of steps of the method described herein without departing from the scope of the invention, as defined by the claims. More specifically, it will be apparent that certain agents, which are both chemically and physiologically, related, might be substituted for the agents described herein while the same or similar results would be achieved.

The present invention will also be described by way of reference to the following clauses:
1. A chicken II GnRH analog, having the sequence p-Glu-His-Trp-Ser-His-Xaal-Trp-Tyr-Pro-Xaa2, capable of binding to tumor cell GnRH receptors and active in the presence of a post-proline peptidase or an endopeptidase, said analog comprising a D-amino acid substitution at position 6 and an ethylamide or aza-Gly-amide substitution at position 10.
2. The chicken II GnRH analog of clause 1 wherein the chicken II GnRH analog is further defined as:
   D-Arg(6)-chicken II GnRH-ethylamide; or
   D-Arg(6)-chicken II GnRH-aza-Gly(10)-amide.
3. The chicken II GnRH analog of clause 1 or 2 wherein the post-proline peptidase is chorionic peptidase-1.
4. The chicken II GnRH analog of any one of the preceding clauses wherein the chicken II GnRH analog is further defined as D-Arg(6)-chicken II GnRH-aza-Gly(10)-amide having a sequence as defined in SEQ ID NO: 2 (p-Glu-His-Trp-Ser-His-D-Arg-Trp-Tyr-Pro-aza-Gly-NH₂).
5. The chicken II GnRH analog of clause 1 wherein the chicken II GnRH analog is further defined as an aza-Gly(10)-amide Chicken II GnRH analog.
6. The chicken II GnRH analog of clause 1 wherein the chicken II GnRH analog is further defined as comprising a D-Arg, a D-Leu, D-tBu-serine, or a D-Trp substitution at position 6 and an aza-Gly amide or an ethylamide at position 10.
7. A pharmaceutical preparation comprising a compound according to any one of the preceding clauses, in admixture with a pharmaceutically acceptable carrier, diluent or excipient.
8. A purified polypeptide, the amino acid sequence of which comprises SEQ ID NO: 2.
9. An antibody that binds specifically to the Chicken II GnRH polypeptide of SEQ ID NO: 6 or SEQ ID NO: 6 with at least one conservative amino acid substitution.
10. The antibody of Clause 9 wherein said antibody is used in regulating cell proliferation.
11. The antibody of Clause 9 wherein said antibody is used in the treatment of tumors.
12. A method of determining whether a biological sample contains a chicken II GnRH polypeptide, comprising contacting the sample with the antibody of Clause 9 and determining whether the antibody specifically binds to the sample, said binding being an indication that the sample contains a chicken II GnRH polypeptide (SEQ ID NO: 6).
13. An antibody that binds specifically to the receptor for the Chicken II GnRH polypeptide of SEQ ID NO: 6 or SEQ ID NO: 6 with at least one conservative amino acid substitution.
14. The antibody of Clause 13 wherein said antibody is used in regulating cell proliferation.
15. The antibody of Clause 13 wherein said antibody is used in the treatment of tumors.
16. A method of determining whether a biological sample contains receptors for Chicken II GnRH (SEQ ID NO: 6) comprising contacting the sample with the antibody of Clause 13 whether the antibody specifically binds to the sample, said binding being an indication that the sample contains receptors for Chicken II GnRH polypeptide (SEQ ID NO: 6).
17. A method of determining whether a biological sample contains the mRNA or DNA, or the respective complements thereof, that codes for Chicken II GnRH polypeptide of SEQ ID NO: 6 or SEQ ID NO: 6 with at least one conservative amino acid substitution comprising the steps of subjecting said sample to in situ localization or any binding or hybridization procedure to determine whether said sample contains said mRNA or DNA.
18. The method of Clause 17 further comprising the step of monitoring cell function.
19. The method of Clause 17 further comprising the step of monitoring tumor growth.
20. A method of determining whether a biological sample contains the mRNA or DNA, or the respective complements thereof, that codes for the receptor to the Chicken II GnRH polypeptide of SEQ ID NO: 6 or SEQ ID NO: 6 with at least one conservative amino acid substitution comprising the steps of subjecting said sample to in situ localization any binding or hybridization procedure to determine whether said sample contains said mRNA or DNA.
21. The method of Clause 20 further comprising the step of monitoring cell function.
22. The method of Clause 20 further comprising the step of monitoring tumor growth.
23. A method of regulating translation of mRNA with the sequence of SEQ ID NO:8 or a degenerate variant of SEQ ID NO:8 comprising the steps of:
   providing a single stranded oligonucleotide at least 10 nucleotides in length, the oligonucleotide being complementary to a portion of SEQ ID NO:8 or a degenerate variant of SEQ ID NO:8;
   providing a cell comprising mRNA with the sequence of SEQ ID NO: 8 or a degenerate variant of SEQ ID NO: 8; and
   introducing the oligonucleotide into the cell, wherein the oligonucleotide regulates translation of said mRNA in the cell.
24. A method of regulating transcription of the sequence of SEQ ID NO: 1 or a degenerate variant of SEQ ID NO: 1 comprising the steps of:
   providing a single stranded oligonucleotide at least 10 nucleotides in length, the oligonucleotide being complementary to a portion of SEQ ID NO: 1 or a degenerate variant of SEQ ID NO: 1;
   providing a cell comprising a DNA with the sequence of SEQ ID NO: 1 or a degenerate variant of SEQ ID NO: 1; and
   introducing the oligonucleotide into the cell, wherein the oligonucleotide regulates transcription of said DNA in the cell.
25. A method of regulating translation of mRNA with the complement to the sequence of SEQ ID NO: 8 or a degenerate variant of SEQ ID NO: 8 comprising the steps of:
   providing a single stranded oligonucleotide at least 10 nucleotides in length, the oligonucleotide having a portion of the sequence of SEQ ID NO: 8 or a degenerate variant of SEQ ID NO: 8 ;
   providing a cell comprising mRNA with the complement to the sequence of SEQ ID NO: 8 or a degenerate variant of SEQ ID NO: 8; and
   introducing the oligonucleotide into the cell, wherein the oligonucleotide regulates translation of said mRNA in the cell.
26. A method of regulating transcription of the complement to the sequence of SEQ ID NO: 1 or a degenerate variant of SEQ ID NO: 1 comprising the steps of:
   providing a single stranded oligonucleotide at least 10 nucleotides in length, the oligonucleotide having a portion of the sequence of SEQ ID NO: 1 or a degenerate variant of SEQ ID NO: 1;
   providing a cell comprising a DNA with the complement to the sequence of SEQ ID NO: 1 or a degenerate variant of SEQ ID NO: 1; and
   introducing the oligonucleotide into the cell, wherein the oligonucleotide regulates transcription of said DNA in the cell.
27. A method of regulating translation of mRNA with the sequence of SEQ ID NO: 8, the complement thereof, or a degenerate variant of SEQ ID NO: 8 or its complement comprising the steps of:
   providing a polypeptide having the sequence of SEQ ID NO: 6 or SEQ ID NO: 6 with at least one conservative amino acid substitution;
   providing a cell comprising mRNA with the sequence of SEQ ID NO: 8, the complement thereof, or a degenerate variant of SEQ ID NO: 8 or its complement; and
   introducing the polypeptide into the cell, wherein the polypeptide regulates translation of said mRNA in the cell.
28. A method of regulating transcription of the sequence of SEQ ID NO: 1 , the complement thereof, or the degenerate variant of SEQ ID NO: 1 or its complement, comprising the steps of:
   providing a polypeptide having the sequence of SEQ ID NO: 6 or SEQ ID NO: 6 with at least one conservative amino acid substitution;
   providing a cell comprising a DNA with the sequence of SEQ ID NO: 1, the complement thereof, or the degenerate variant of SEQ ID NO: 1 or its complement ; and
   introducing the polypeptide into the cell, wherein the polypeptide regulates transcription of said DNA in the cell.
29. A method of regulating the translation of mRNA for the receptor of the polypeptide with the sequence of SEQ ID NO: 6, the complement thereof, or SEQ ID NO: 6 with at least one conservative amino acid substitution or the DNA complement thereof comprising the steps of:
   providing a single stranded oligonucleotide at least 10 nucleotides in length, the oligonucleotide being complementary to a portion of SEQ ID NO: 1 or a degenerate variant of SEQ ID NO: 1;
   providing a cell comprising mRNA for the receptor of the polypeptide with the sequence of SEQ ID NO: 6, the complement thereof, or SEQ ID NO: 6 with at least one conservative amino acid substitution or the DNA complement thereof; and
   introducing the oligonucleotide into the cell, wherein the oligonucleotide regulates the translation of said mRNA in the cell.
30. A method of regulating transcription of the DNA for the receptor of the polypeptide of SEQ ID NO: 6, the complement thereof, SEQ ID NO: 6 with at least one conservative amino acid substitution or its complement, comprising the steps of:
   providing a single stranded oligonucleotide at least 10 nucleotides in length, the oligonucleotide having a portion of the sequence of SEQ ID NO: 1 or a degenerate variant of SEQ ID NO: 1;
   providing a cell comprising DNA for the receptor of the polypeptide with the sequence of SEQ ID NO: 6, the complement thereof, SEQ ID NO: 6 with at least one conservative amino acid substitution or its complement; and
   introducing the oligonucleotide into the cell, wherein the oligonucleotide regulates the transcription of said DNA in the cell.
31. A method of regulating transcription of the DNA for the receptor of the polypeptide of SEQ ID NO: 6 or SEQ ID NO: 6 with at least one conservative amino acid substitution comprising the steps of:
   providing a single stranded oligonucleotide at least 10 nucleotides in length, the oligonucleotide being complementary to a portion of the DNA for the receptor of the polypeptide with SEQ ID NO: 6 or SEQ ID NO: 6 with at least one conservative amino acid substitution;
   providing a cell comprising a DNA for the receptor of the polypeptide with the sequence of SEQ ID NO: 6 or SEQ ID NO: 6 with at least one conservative amino acid substitution; and
   introducing the oligonucleotide into the cell, wherein the oligonucleotide regulates transcription of said DNA in the cell.
32. A method of regulating translation ofmRNA for the receptor of the polypeptide of SEQ ID NO: 6 or SEQ ID NO: 6 with at least one conservative amino acid substitution comprising the steps of:
   providing a single stranded oligonucleotide at least 10 nucleotides in length, the oligonucleotide being complementary to a portion of the mRNA that codes for receptor that binds the polypeptide of SEQ ID NO: 6 or SEQ ID NO: 6 with at least one conservative amino acid substitution;
   providing a cell comprising mRNA for the receptor of the polypeptide of SEQ ID NO: 6 or SEQ ID NO: 6 with at least one conservative amino acid substitution; and
   introducing the oligonucleotide into the cell, wherein the oligonucleotide regulates the translation of said mRNA in the cell.
33. A method of regulating translation of mRNA for the receptor of the polypeptide with the sequence of SEQ ID NO: 6, the complement thereof, or SEQ ID NO: 6 with at least one conservative amino acid substitution or the complement thereof comprising the steps of:
   providing a polypeptide with the sequence of SEQ ID NO: 6 or SEQ ID NO: 6 with at least one conservative amino acid substitution;
   providing a cell comprising mRNA for the receptor of the polypeptide with the sequence of SEQ ID NO: 6, the complement thereof, or SEQ ID NO: 6 with at least one conservative amino acid substitution or the complement thereof; and
   introducing the polypeptide into the cell, wherein the polypeptide regulates the translation of said mRNA in the cell.
34. A method of regulating transcription of the DNA for the receptor of the polypeptide of SEQ ID NO: 6, the complement thereof, or SEQ ID NO: 6 with at least one conservative amino acid substitution or the complement thereof, comprising the steps of:
   providing a polypeptide with the sequence of SEQ ID NO: 6 or SEQ ID NO: 6 with at least one conservative amino acid substitution;
   providing a cell comprising DNA for the receptor ofthe polypeptide with the sequence of SEQ ID NO: 6, the complement thereof, or SEQ ID NO: 6 with at least one conservative amino acid substitution or the complement thereof; and
   introducing the polypeptide into the cell, wherein the polypeptide regulates the transcription of the DNA in the cell.
35. A method of regulating the function of the receptor to the polypeptide of SEQ ID NO: 6 or SEQ ID NO: 6 with at least one conservative amino acid substitution comprising the steps of:
   providing a polypeptide having the sequence of SEQ ID NO: 6 or SEQ ID NO: 6 with at least one conservative amino acid substitution;
   providing a cell comprising a receptor to the polypeptide of SEQ ID NO: 6 or SEQ ID NO: 6 with at least one conservative amino acid substitution; and
   introducing the polypeptide into the vicinity of the cell, wherein the polypeptide regulates the function of the receptor.
36. A method of regulating transcription of the complement to the DNA for the receptor of the polypeptide of SEQ ID NO: 6 or SEQ ID NO: 6 with at least one conservative amino acid substitution comprising the steps of:
   providing a single stranded oligonucleotide, 10 nucleotides in length, the oligonucleotide coding for the receptor of the polypeptide with SEQ ID NO: 6 or SEQ ID NO: 6 with at least one conservative amino acid substitution;
   providing a cell comprising a DNA complementary to the receptor of the polypeptide with SEQ ID NO: 6 or SEQ ID NO: 6 with at least one conservative amino acid substitution; and
   introducing the oligonucleotide into the cell, wherein the oligonucleotide regulates transcription of said DNA in the cell.
37. A method of regulating translation of the complement to the mRNA for the receptor of the polypeptide of SEQ ID NO: 6 or SEQ ID NO: 6 with at least one conservative amino acid substitution comprising the steps of:
   providing a single stranded oligonucleotide at least 10 nucleotides in length, the oligonucleotide having a portion of the sequence of the mRNA that codes for the receptor that binds the polypeptide of SEQ ID NO: 6 or SEQ ID NO: 6 with at least one conservative amino acid substitution;
   providing a cell comprising the complement to the mRNA for the receptor of the polypeptide of SEQ ID NO: 6 or SEQ ID NO: 6 with at least one conservative amino acid substitution; and
   introducing the oligonucleotide into the cell, wherein the oligonucleotide regulates the translation of said mRNA in the cell.
38. A method of regulating transcription of the DNA for the receptor that binds the polypeptide of SEQ ID NO: 6 or SEQ ID NO: 6 with at least one conservative amino acid substitution comprising the steps of:
   providing a single stranded oligonucleotide at least 10 nucleotides in length, the oligonucleotide being complementary to a portion of SEQ ID NO: 1 or a degenerate variant of SEQ ID NO:1;
   providing a cell comprising DNA for the receptor of the polypeptide with the sequence of SEQ ID NO: 6 or SEQ ID NO: 6 with at least one conservative amino acid substitution; and
   introducing the oligonucleotide into the cell, wherein the oligonucleotide regulates transcription of DNA in the cell.
39. A method of regulating translation of mRNA for the receptor of the polypeptide with the sequence of SEQ ID NO: 6 or SEQ ID NO: 6 with at least one conservative amino acid substitution comprising the steps of:
   providing a single stranded oligonucleotide at least 10 nucleotides in length, the oligonucleotide being complementary to a portion of SEQ ID NO: 1 or a degenerate variant of SEQ ID NO: 1;
   providing a cell comprising mRNA for the receptor of the polypeptide with the sequence of SEQ ID NO: 6 or SEQ ID NO: 6 with at least one conservative amino acid substitution; and introducing the oligonucleotide into the cell, wherein the oligonucleotide regulates translation of said mRNA in the cell.
40. A method of regulating secretion of the polypeptide of SEQ ID NO: 6 or SEQ ID NO: 6 with at least one conservative amino acid substitution comprising the steps of:
   providing a single stranded oligonucleotide at least 10 nucleotides in length, the oligonucleotide being complementary to a portion of SEQ ID NO:8 or a degenerate variant of SEQ ID NO: 8;
   providing a cell comprising mRNA with the sequence of SEQ ID NO: 8 or a degenerate variant of SEQ ID NO: 8; and
   introducing the oligonucleotide into the cell, wherein the oligonucleotide regulates translation of said mRNA in the cell.
41. A method of regulating secretion of the polypeptide of SEQ ID NO: 6 or SEQ ID NO: 6 with at least one conservative amino acid substitution comprising the steps of:
   providing a single stranded oligonucleotide at least 10 nucleotides in length, the oligonucleotide being complementary to a portion of SEQ ID NO: 1 or a degenerate variant of SEQ ID NO: 1;
   providing a cell comprising a DNA with the sequence of SEQ ID NO: 1 or a degenerate variant of SEQ ID NO: 1; and
   introducing the oligonucleotide into the cell, wherein the oligonucleotide regulates transcription of said DNA in the cell.
42. A method of regulating secretion of the polypeptide of SEQ ID NO: 6 or SEQ ID NO: 6 with at least one conservative amino acid substitution comprising the steps of:
   providing a polypeptide having the sequence of SEQ ID NO: 6 or SEQ ID NO: 6 with at least one conservative amino acid substitution;
   providing a cell comprising mRNA with the sequence of SEQ ID NO: 8, the complement thereof, or a degenerate variant of SEQ ID NO: 8 or its complement; and
   introducing the polypeptide into the cell, wherein the polypeptide regulates translation of said mRNA in the cell.
43. A method ofregulating secretion ofthe polypeptide of SEQ ID NO: 6 or SEQ ID NO: 6 with at least one conservative amino acid substitution comprising the steps of:
   providing a polypeptide having the sequence of SEQ ID NO: 6 or SEQ ID NO: 6 with at least one conservative amino acid substitution;
   providing a cell comprising a DNA with the sequence of SEQ ID NO: 1, the complement thereof, or the degenerate variant of SEQ ID NO: 1 or its complement ; and
   introducing the polypeptide into the cell, wherein the polypeptide regulates transcription of said DNA in the cell.
44. A method of regulating secretion of the receptor to the polypeptide of SEQ ID NO: 6 or SEQ ID NO: 6 with at least one conservative amino acid substitution comprising the steps of:
   providing a single stranded oligonucleotide at least 10 nucleotides in length, the oligonucleotide being complementary to a portion of the DNA for the receptor ofthe polypeptide with SEQ ID NO: 6 or SEQ ID NO: 6 with at least one conservative amino acid substitution;
   providing a cell comprising a DNA for the receptor of the polypeptide with the sequence of SEQ ID NO: 6 or SEQ ID NO: 6 with at least one conservative amino acid substitution; and
   introducing the oligonucleotide into the cell, wherein the oligonucleotide regulates transcription of said DNA in the cell.
45. A method of regulating secretion of the receptor to the polypeptide of SEQ ID NO: 6 or SEQ ID NO: 6 with at least one conservative amino acid substitution comprising the steps of:
   providing a single stranded oligonucleotide at least 10 nucleotides in length, the oligonucleotide being complementary to a portion of the mRNA that codes for receptor that binds the polypeptide of SEQ ID NO: 6 or SEQ ID NO: 6 with at least one conservative amino acid substitution;
   providing a cell comprising mRNA for the receptor of the polypeptide of SEQ ID NO: 6 or SEQ ID NO: 6 with at least one conservative amino acid substitution; and
   introducing the oligonucleotide into the cell, wherein the oligonucleotide regulates the translation of said mRNA in the cell.
46. A method of regulating secretion of the receptor to the polypeptide of SEQ ID NO: 6 or SEQ ID NO: 6 with at least one conservative amino acid substitution comprising the steps of:
   providing a polypeptide with the sequence of SEQ ID NO: 6 or SEQ ID NO: 6 with at least one conservative amino acid substitution;
   providing a cell comprising mRNA for the receptor ofthe polypeptide with the sequence of SEQ ID NO: 6, the complement thereof, or SEQ ID NO: 6 with at least one conservative amino acid substitution or the complement thereof; and
   introducing the polypeptide into the cell, wherein the polypeptide regulates the translation of said mRNA in the cell.
47. A method ofregulating secretion ofthe receptor to the polypeptide of SEQ ID NO: 6 or SEQ ID NO: 6 with at least one conservative amino acid substitution comprising the steps of:
   providing a polypeptide with the sequence of SEQ ID NO: 6 or SEQ ID NO: 6 with at least one conservative amino acid substitution;
   providing a cell comprising DNA for the receptor of the polypeptide with the sequence of SEQ ID NO: 6, the complement thereof, or SEQ ID NO: 6 with at least one conservative amino acid substitution or the complement thereof; and
   introducing the polypeptide into the cell, wherein the polypeptide regulates the transcription of the DNA in the cell.
48. A method of regulating the transcription of the mRNA with the sequence of SEQ ID NO: 8 or SEQ ID NO: 8 with at least one conservative amino acid substitution comprising the steps of:
   providing a cell comprising a receptor to the polypeptide with the sequence of SEQ ID NO: 6 or SEQ ID NO: 6 with at least one conservative amino acid substitution;
   binding said polypeptide with the sequence of SEQ ID NO: 6 or SEQ ID NO: 6 with at least one conservative amino acid substitution to said receptor; and
   triggering a biological mechanism within said cell responsible for regulating said transcription.
49. A method of regulating the translation of the polypeptide with the sequence of SEQ ID NO: 6 or SEQ ID NO: 6 with at least one conservative amino acid substitution comprising the steps of:
   providing a cell comprising a receptor to the polypeptide with the sequence of SEQ ID NO: 6 or SEQ ID NO: 6 with at least one conservative amino acid substitution;
   binding said polypeptide with the sequence of SEQ ID NO: 6 or SEQ ID NO: 6 with at least one conservative amino acid substitution to said receptor; and
   triggering a biological mechanism within said cell responsible for regulating said translation.
50. A method of regulating the secretion of the polypeptide with the sequence of SEQ ID NO: 6 or SEQ ID NO: 6 with at least one conservative amino acid substitution comprising the steps of:
   providing a cell comprising a receptor to the polypeptide with the sequence of SEQ ID NO: 6 or SEQ ID NO: 6 with at least one conservative amino acid substitution;
   binding said polypeptide with the sequence of SEQ ID NO: 6 or SEQ ID NO: 6 with at least one conservative amino acid substitution to said receptor; and
   triggering a biological mechanism within said cell responsible for regulating said secretion.
51. A purified polypeptide, the amino acid sequence of which comprises SEQ ID NO: 6, SEQ ID NO: 6 with at least one conservative amino acid substitution, or a mimetic functional equivalent of SEQ ID NO: 6.
52. A pharmaceutical preparation comprising a compound according to Clause 51 in admixture with a pharmaceutically acceptable carrier, diluent or excipient.
53. A method of treating a patient or animal, comprising identifying a patient or animal in need of treatment and administering a polypeptide with SEQ ID NO: 6, SEQ ID NO: 6 with at least one conservative amino acid substitution, or a mimetic functional equivalent of SEQ ID NO: 6 to the patient or animal.
54. A method of treating a patient or animal, comprising identifying a patient or animal in need of treatment and administering a polypeptide which acts as a receptor to the polypeptide of SEQ ID NO: 6, SEQ ID NO: 6 with at least one conservative amino acid substitution, or a mimetic functional equivalent of SEQ ID NO: 6 to the patient or animal.

## Claims

1. A chicken II GnRH analog, having the sequence p-Glu-His-Trp-Ser-His-Xaal-Trp-Tyr-Pro-Xaa2, capable of binding to tumor cell GnRH receptors and active in the presence of a post-proline peptidase or an endopeptidase, said analog comprising a D-amino acid substitution at position 6 and an ethylamide or aza-Gly-amide substitution at position 10.

2. The chicken II GnRH analog of claim 1 wherein the chicken II GnRH analog is further defined as:
D-Arg(6)-chicken II GnRH-ethylamide; or
D-Arg(6)-chicken II GnRH-aza-Gly(10)-amide.

3. The chicken II GnRH analog of claim 1 or 2 wherein the post-proline peptidase is chorionic peptidase-1.

4. The chicken II GnRH analog of any one of claims 1, 2 or 3, wherein the chicken II GnRH analog is further defined as D-Arg(6)-chicken II GnRH-aza-Gly(10)-amide having a sequence as defined in SEQ ID NO: 2 (p-Glu-His-Trp-Ser-His-D-Arg-Trp-Tyr-Pro-aza-Gly-NH₂).

5. The chicken II GnRH analog of claim 1 wherein the chicken II GnRH analog is further defined as an aza-Gly(10)-amide Chicken II GnRH analog.

6. The chicken II GnRH analog of claim 1 wherein the chicken II GnRH analog is further defined as comprising a D-Arg, a D-Leu, D-tBu-serine, or a D-Trp substitution at position 6 and an aza-Gly amide or an ethylamide at position 10.

7. The analog of any one of the preceding claims for use in therapy.

8. A pharmaceutical preparation comprising the analog of any one of claims 1 to 6, in admixture with a pharmaceutically acceptable carrier, diluent or excipient.

9. A purified polypeptide, the amino acid sequence of which comprises SEQ ID NO: 2.

10. An antibody that binds specifically to the Chicken II GnRH polypeptide of SEQ ID NO: 6 or SEQ ID NO: 6 with at least one conservative amino acid substitution.

11. A method of determining whether a biological sample contains a chicken II GnRH polypeptide, comprising contacting the sample with the antibody of Claim 9 and determining whether the antibody specifically binds to the sample, said binding being an indication that the sample contains a chicken II GnRH polypeptide (SEQ ID NO: 6).

12. An antibody that binds specifically to the receptor for the Chicken II GnRH polypeptide of SEQ ID NO: 6 or SEQ ID NO: 6 with at least one conservative amino acid substitution.

13. The antibody of Claim 10 or 12 for use in therapy.

14. The antibody of Claim 10 or 12 for the treatment of tumors.

15. A method of determining whether a biological sample contains receptors for Chicken II GnRH (SEQ ID NO: 6) comprising contacting the sample with the antibody of Claim 12 whether the antibody specifically binds to the sample, said binding being an indication that the sample contains receptors for Chicken II GnRH polypeptide (SEQ ID NO: 6).

16. A method of determining whether a biological sample contains the mRNA or DNA, or the respective complements thereof, that codes for Chicken II GnRH polypeptide of SEQ ID NO: 6 or SEQ ID NO: 6 with at least one conservative amino acid substitution comprising the steps of subjecting said sample to in situ localization or any binding or hybridization procedure to determine whether said sample contains said mRNA or DNA.

17. A method of determining whether a biological sample contains the mRNA or DNA, or the respective complements thereof, that codes for the receptor to the Chicken II GnRH polypeptide of SEQ ID NO: 6 or SEQ ID NO: 6 with at least one conservative amino acid substitution comprising the steps of subjecting said sample to in situ localization any binding or hybridization procedure to determine whether said sample contains said mRNA or DNA.

18. The method of Claim 16 or 17 further comprising the step of monitoring cell function.

19. The method of Claim 16 or 17 further comprising the step of monitoring tumor growth.

20. A single stranded oligonucleotide at least 10 nucleotides in length, the oligonucleotide being complementary to a portion of SEQ ID NO:8 or a degenerate variant of SEQ ID NO:8 for use in therapy.

21. A single stranded oligonucleotide at least 10 nucleotides in length, the oligonucleotide being complementary to a portion of SEQ ID NO: 1 or a degenerate variant of SEQ ID NO: 1 for use in therapy.

22. A single stranded oligonucleotide at least 10 nucleotides in length, the oligonucleotide having a portion of the sequence of SEQ ID NO: 8 or a degenerate variant of SEQ ID NO: 8 for use in therapy.

23. A single stranded oligonucleotide at least 10 nucleotides in length, the oligonucleotide having a portion of the sequence of SEQ ID NO: 1 or a degenerate variant of SEQ ID NO: 1 for use in therapy.

24. A single stranded oligonucleotide at least 10 nucleotides in length, the oligonucleotide being complementary to a portion of the DNA for the receptor of the polypeptide with SEQ ID NO: 6 or SEQ ID NO: 6 with at least one conservative amino acid substitution for use in therapy.

25. A single stranded oligonucleotide at least 10 nucleotides in length, the oligonucleotide being complementary to a portion of the mRNA that codes for receptor that binds the polypeptide of SEQ ID NO: 6 or SEQ ID NO: 6 with at least one conservative amino acid substitution for use in therapy.

26. A single stranded oligonucleotide, 10 nucleotides in length, the oligonucleotide coding for the receptor of the polypeptide with SEQ ID NO: 6 or SEQ ID NO: 6 with at least one conservative amino acid substitution for use in therapy.

27. A single stranded oligonucleotide at least 10 nucleotides in length, the oligonucleotide having a portion of the sequence of the mRNA that codes for the receptor that binds the polypeptide of SEQ ID NO: 6 or SEQ ID NO: 6 with at least one conservative amino acid substitution for use in therapy.

28. A purified polypeptide, the amino acid sequence of which comprises SEQ ID NO: 6, SEQ ID NO: 6 with at least one conservative amino acid substitution, or a mimetic functional equivalent of SEQ ID NO: 6.

29. The protein of claim 2 8 for use in therapy.

30. A pharmaceutical preparation comprising a compound according to Claim 28 in admixture with a pharmaceutically acceptable carrier, diluent or excipient.

31. A polypeptide which acts as a receptor to the polypeptide of SEQ ID NO: 6, SEQ ID NO: 6 with at least one conservative amino acid substitution, or a mimetic functional equivalent of SEQ ID NO: 6 for use in therapy.
